(19) Europäisches Patentamt
European Patent Office
Office européen des brevets

(11) EP 4 491 632 A1

(12) EUROPEAN PATENT APPLICATION
published in accordance with Art. 153(4) EPC

(43) Date of publication:
**15.01.2025 Bulletin 2025/03**

(21) Application number: **23765906.5**

(22) Date of filing: **03.03.2023**

(51) International Patent Classification (IPC):
***C07K 16/28*** *(2006.01)*

(52) Cooperative Patent Classification (CPC):
**C07K 16/28**

(86) International application number:
**PCT/CN2023/079562**

(87) International publication number:
**WO 2023/169328 (14.09.2023 Gazette 2023/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.03.2022 CN 202210226048**
**09.02.2023 CN 202310099967**

(71) Applicants:
- **Sanyou Biopharmaceuticals Co., Ltd.**
  **Shanghai 201114 (CN)**
- **Hangzhou Zhongmeihuadong Pharmaceutical Co., Ltd.**
  **GongShu District**
  **HangZhou, Zhejiang 310011 (CN)**

(72) Inventors:
- **XU, Peifang**
  **Shanghai 201114 (CN)**
- **LIU, Chanjuan**
  **Shanghai 201114 (CN)**
- **LANG, Guojun**
  **Shanghai 201114 (CN)**
- **LIN, Chen**
  **Hangzhou, Zhejiang 310011 (CN)**
- **SHU, Qingyu**
  **Hangzhou, Zhejiang 310011 (CN)**
- **LIU, Dongzhou**
  **Hangzhou, Zhejiang 310011 (CN)**

(74) Representative: **HGF**
**HGF Limited**
**1 City Walk**
**Leeds LS11 9DX (GB)**

Remarks:
The complete document including Reference Table(s) and the Sequence Listing(s) can be downloaded from the EPO website

# (54) ROR1-TARGETED BINDING MOLECULE AND USE THEREOF

(57) The present application relates to antibodies that specifically recognize ROR1, and preparation methods and uses thereof. The present application also relates to antibody-drug conjugates (ADCs) targeting ROR1 and compositions containing the molecule. In addition, the present invention also relates to the therapeutic and diagnostic uses of these antibodies, antibody fragments and antibody-drug conjugates.

EP 4 491 632 A1

## Description

## FIELD OF THE INVENTION

The present application relates to antibodies that specifically recognize ROR1, and preparation methods and uses thereof. In addition, the present application also relates to antibody-drug conjugates (ADCs) targeting ROR1 and compositions containing the molecule. In addition, the present invention also relates to the therapeutic and diagnostic uses of these antibodies, antibody fragments and antibody-drug conjugates.

## BACKGROUND OF THE INVENTION

Antibody-Drug Conjugates (ADCs) are a class of drugs derived from traditional antibody therapies. They consist of monoclonal antibodies targeting specific antigens conjugated to small-molecule cytotoxic agents through linkers. This combination provides the targeted specificity of antibody drugs and the cytotoxic effects of traditional small molecules, making ADCs particularly suited for the treatment of malignant tumors. Due to their excellent tumor-killing effects, ADCs have quickly become a focal point in anticancer drug development. However, ADCs often have higher toxicity, necessitating high specificity for target antigens. Ideally, the targets for ADC development are antigens that are highly expressed in tumors but low or not expressed in healthy tissues. The target antigens should be surface receptors upregulated in tumor cells that promote tumor growth or survival, and they should possess internalization properties.

Both Receptor Tyrosine Kinase-like Orphan Receptor 1 (ROR1, also known as Receptor-related Neurotrophic Tyrosine Kinase 1, NTRKR1) and Receptor Tyrosine Kinase-like Orphan Receptor 2 (ROR2) are single-pass transmembrane proteins, belonging to the family of receptor tyrosine kinase (RTK), extracellular parr thereof consists of an immunoglobulin-like domain (Ig) and two cysteine-rich domains (FZD domain and KRD domain), and the intracellular part thereof consists of a tyrosine kinase domain, two serine- or threonine-rich domains and a proline-rich domain. ROR1 and ROR2 participate in the non-canonical Wnt signaling pathway by binding to the ligand Wnt5a through the FZD domain(Oishi I, Suzuki H, Onishi N, Takada R, Kani S, Ohkawara B, Koshida I, Suzuki K, Yamada G, Schwabe GC et al (2003). Genes Cells 8:645-654; Fukuda T, Chen L, Endo T, Tang L, Lu D, Castro JE, Widhopf GF II, Rassenti LZ, Cantwell MJ, Prussak CE et al (2008). Proc Natl Acad Sci USA 105:3047-3052; Paganoni S, Bernstein J, Ferreira A (2010). Neuroscience 165:1261-1274). ROR1 can inhibit apoptosis, enhance EGFR signaling, and induce epithelial-mesenchymal transition (EMT) (Fukuda T, Chen L, Endo T, Tang L, Lu D, Castro JE, Widhopf GF II, Rassenti LZ, Cantwell MJ, Prussak CE et al (2008). Proc Natl Acad Sci USA 105:3047-3052; Yamaguchi T, Yanagisawa K, Sugiyama R, Hosono Y, Shimada Y, ArimaC, KatoS, TomidaS, Suzuki M,OsadaHet al (2012). Cancer Cell 21:348-361; Cui B, Zhang S, Chen L, Yu J, Widhopf GF, Fecteau J-F, Rassenti LZ, Kipps TJ (2013). Cancer Res 73:3649-3660).

ROR1 is a conserved embryonic protein, the expression of which gradually decreases with embryonic development and is almost absent or lowly expressed in most adult tissues. However, it has been found in more and more literatures that ROR1 is expressed in a variety of cancer cells, such as B-cell chronic lymphocytic leukemia (CLL) and other hematological malignancies, renal cell cancer, colon cancer and certain other cancer cell lines of breast cancer. Furthermore, ROR1 plays an important role in the progression of many hematological and solid malignancies. Therefore, as a cancer marker, ROR1 becomes an ideal drug target for cancer treatment.

Although several antibody drugs against ROR1 have been disclosed in the prior art, there is still an urgent need to develop anti-ROR1 antibodies with high-quality due to ROR1 as tumor markers for pan-cancers. Such antibodies can be used as the basis for developing antibody-based targeted therapies for cancers expressing ROR1, and can also be used as a diagnostic tool to detect ROR1 expression in ROR1-related diseases. In addition, in view of the good prospects shown by ADCs in the field of tumor treatment, there is still an urgent need for ADCs containing ROR1 with effective therapeutic effects, and the present invention meets these needs.

## SUMMARY OF THE INVENTION

In a first aspect, the present invention provides an antibody targeting ROR1, which has the following advantages:

(1) binding to ROR1 and target cells expressing ROR1 with high affinity;
(2) capable of entering cells through internalization;
(3) suitable for constructing effective therapeutic antibody-drug conjugates.

In one embodiment, the present invention provides an anti-ROR1 antibody specifically binding to ROR1 and antigen-binding fragment thereof, comprising:

1) three heavy chain CDRs (HCDR1, HCDR2, HCDR3) comprised in the heavy chain variable region as shown in SEQ

ID NO: 57 and three light chain CDRs (LCDR1, LCDR2, LCDR3) comprised in the light chain variable region as shown in SEQ ID NO: 56;

2) three heavy chain CDRs (HCDR1, HCDR2, HCDR3) comprised in the heavy chain variable region as shown in SEQ ID NO: 55 and three light chain CDRs (LCDR1, LCDR2, LCDR3) comprised in the light chain variable region as shown in SEQ ID NO: 54;

3) three heavy chain CDRs (HCDR1, HCDR2, HCDR3) comprised in the heavy chain variable region as shown in SEQ ID NO: 59 and three light chain CDRs (LCDR1, LCDR2, LCDR3) comprised in the light chain variable region as shown in SEQ ID NO: 58;

4) three heavy chain CDRs (HCDR1, HCDR2, HCDR3) comprised in the heavy chain variable region as shown in SEQ ID NO: 61 and three light chain CDRs (LCDR1, LCDR2, LCDR3) comprised in the light chain variable region as shown in SEQ ID NO: 60;

5) three heavy chain CDRs (HCDR1, HCDR2, HCDR3) comprised in the heavy chain variable region as shown in SEQ ID NO: 63 and three light chain CDRs (LCDR1, LCDR2, LCDR3) comprised in the light chain variable region as shown in SEQ ID NO: 62;

6) three heavy chain CDRs (HCDR1, HCDR2, HCDR3) comprised in the heavy chain variable region as shown in SEQ ID NO: 65 and three light chain CDRs (LCDR1, LCDR2, LCDR3) comprised in the light chain variable region as shown in SEQ ID NO: 64;

7) three heavy chain CDRs (HCDR1, HCDR2, HCDR3) comprised in the heavy chain variable region as shown in SEQ ID NO: 67 and three light chain CDRs (LCDR1, LCDR2, LCDR3) comprised in the light chain variable region as shown in SEQ ID NO: 66;

8) three heavy chain CDRs (HCDR1, HCDR2, HCDR3) comprised in the heavy chain variable region as shown in SEQ ID NO: 68 and three light chain CDRs (LCDR1, LCDR2, LCDR3) comprised in the light chain variable region as shown in SEQ ID NO: 66;

9) three heavy chain CDRs (HCDR1, HCDR2, HCDR3) comprised in the heavy chain variable region as shown in SEQ ID NO: 70 and three light chain CDRs (LCDR1, LCDR2, LCDR3) comprised in the light chain variable region as shown in SEQ ID NO: 69;

10) three heavy chain CDRs (HCDR1, HCDR2, HCDR3) comprised in the heavy chain variable region as shown in SEQ ID NO: 72 and three light chain CDRs (LCDR1, LCDR2, LCDR3) comprised in the light chain variable region as shown in SEQ ID NO: 71;

11) three heavy chain CDRs (HCDR1, HCDR2, HCDR3) comprised in the heavy chain variable region as shown in SEQ ID NO: 74 and three light chain CDRs (LCDR1, LCDR2, LCDR3) comprised in the light chain variable region as shown in SEQ ID NO: 73;

12) three heavy chain CDRs (HCDR1, HCDR2, HCDR3) comprised in the heavy chain variable region as shown in SEQ ID NO: 75 and three light chain CDRs (LCDR1, LCDR2, LCDR3) comprised in the light chain variable region as shown in SEQ ID NO: 79;

13) three heavy chain CDRs (HCDR1, HCDR2, HCDR3) comprised in the heavy chain variable region as shown in SEQ ID NO: 76 and three light chain CDRs (LCDR1, LCDR2, LCDR3) comprised in the light chain variable region as shown in SEQ ID NO: 81;

14) three heavy chain CDRs (HCDR1, HCDR2, HCDR3) comprised in the heavy chain variable region as shown in SEQ ID NO: 77 and three light chain CDRs (LCDR1, LCDR2, LCDR3) comprised in the light chain variable region as shown in SEQ ID NO: 82;

15) three heavy chain CDRs (HCDR1, HCDR2, HCDR3) comprised in the heavy chain variable region as shown in SEQ ID NO: 78 and three light chain CDRs (LCDR1, LCDR2, LCDR3) comprised in the light chain variable region as shown in SEQ ID NO: 80;

16) three heavy chain CDRs (HCDR1, HCDR2, HCDR3) comprised in the heavy chain variable region as shown in SEQ ID NO: 78 and three light chain CDRs (LCDR1, LCDR2, LCDR3) comprised in the light chain variable region as shown in SEQ ID NO: 81;

17) three heavy chain CDRs (HCDR1, HCDR2, HCDR3) comprised in the heavy chain variable region as shown in SEQ ID NO: 78 and three light chain CDRs (LCDR1, LCDR2, LCDR3) comprised in the light chain variable region as shown in SEQ ID NO: 82; or

18) three heavy chain CDRs (HCDR1, HCDR2, HCDR3) comprised in the heavy chain variable region as shown in SEQ ID NO: 84 and three light chain CDRs (LCDR1, LCDR2, LCDR3) comprised in the light chain variable region as shown in SEQ ID NO: 89.

In one embodiment, the present invention provides an anti-ROR1 antibody specifically binding to ROR1 and antigen-binding fragment thereof, comprising:

1) HCDR1, HCDR2, HCDR3 comprising the sequences as shown in SEQ ID NO: 1, 2 and 3, or sequences containing

one or more and no more than 3 amino acid substitutions (e.g., conservative substitutions), deletions or insertions relative to said sequences; and LCDR1, LCDR2, LCDR3 comprising the sequences as shown in SEQ ID NO: 28, 29 and 30, or sequences containing one or more and no more than 3 amino acid substitutions (e.g., conservative substitutions), deletions or insertions relative to said sequences;

2) HCDR1, HCDR2, HCDR3 comprising the sequences as shown in SEQ ID NO: 4, 5 and 6, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 31, 32 and 33, or sequences containing one or more and no more than 3 amino acid substitutions (e.g., conservative substitutions), deletions or insertions relative to said sequences;

3) HCDR1, HCDR2, HCDR3 comprising the sequences as shown in SEQ ID NO: 7, 8 and 9, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 34, 32 and 33, or sequences containing one or more and no more than 3 amino acid substitutions (e.g., conservative substitutions), deletions or insertions relative to said sequences;

4) HCDR1, HCDR2, HCDR3 comprising the sequences as shown in SEQ ID NO: 10, 11 and 12, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 35, 32 and 36, or sequences containing one or more and no more than 3 amino acid substitutions (e.g., conservative substitutions), deletions or insertions relative to said sequences;

5) HCDR1, HCDR2, HCDR3 comprising the sequences as shown in SEQ ID NO: 4, 13 and 14, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 35, 32 and 37, or sequences containing one or more and no more than 3 amino acid substitutions (e.g., conservative substitutions), deletions or insertions relative to said sequences;

6) HCDR1, HCDR2, HCDR3 comprising the sequences as shown in SEQ ID NO: 15, 16 and 17, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 38, 32 and 33, or sequences containing one or more and no more than 3 amino acid substitutions (e.g., conservative substitutions), deletions or insertions relative to said sequences;

7) HCDR1, HCDR2, HCDR3 comprising the sequences as shown in SEQ ID NO: 18, 8 and 19, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 31, 32 and 33, or sequences containing one or more and no more than 3 amino acid substitutions (e.g., conservative substitutions), deletions or insertions relative to said sequences;

8) HCDR1, HCDR2, HCDR3 comprising the sequences as shown in SEQ ID NO: 20, 21 and 12, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 31, 32 and 33, or sequences containing one or more and no more than 3 amino acid substitutions (e.g., conservative substitutions), deletions or insertions relative to said sequences;

9) HCDR1, HCDR2, HCDR3 comprising the sequences as shown in SEQ ID NO: 22, 23 and 24, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 39, 40 and 41, or sequences containing one or more and no more than 3 amino acid substitutions (e.g., conservative substitutions), deletions or insertions relative to said sequences;

10) HCDR1, HCDR2, HCDR3 comprising the sequences as shown in SEQ ID NO: 4, 25 and 26, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 35, 32 and 33, or sequences containing one or more and no more than 3 amino acid substitutions (e.g., conservative substitutions), deletions or insertions relative to said sequences;

11) HCDR1, HCDR2, HCDR3 comprising the sequences as shown in SEQ ID NO: 18, 25 and 27, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 35, 32 and 33, or sequences containing one or more and no more than 3 amino acid substitutions (e.g., conservative substitutions), deletions or insertions relative to said sequences;

12) HCDR1, HCDR2, HCDR3 comprising the sequences as shown in SEQ ID NO: 4, 5 and 6, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 31, 45 and 48, or sequences containing one or more and no more than 3 amino acid substitutions (e.g., conservative

substitutions), deletions or insertions relative to said sequences;

13) HCDR1, HCDR2, HCDR3 comprising the sequences as shown in SEQ ID NO: 4, 5 and 6, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 43, 46 and 48, or sequences containing one or more and no more than 3 amino acid substitutions (e.g., conservative substitutions), deletions or insertions relative to said sequences;

14) HCDR1, HCDR2, HCDR3 comprising the sequences as shown in SEQ ID NO: 4, 5 and 6, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 44, 47 and 48, or sequences containing one or more and no more than 3 amino acid substitutions (e.g., conservative substitutions), deletions or insertions relative to said sequences;

15) HCDR1, HCDR2, HCDR3 comprising the sequences as shown in SEQ ID NO: 4, 42 and 6, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 31, 45 and 48, or sequences containing one or more and no more than 3 amino acid substitutions (e.g., conservative substitutions), deletions or insertions relative to said sequences;

16) HCDR1, HCDR2, HCDR3 comprising the sequences as shown in SEQ ID NO: 4, 42 and 6, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 43, 46 and 48, or sequences containing one or more and no more than 3 amino acid substitutions (e.g., conservative substitutions), deletions or insertions relative to said sequences;

17) HCDR1, HCDR2, HCDR3 comprising the sequences as shown in SEQ ID NO: 4, 42 and 6, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 44, 47 and 48, or sequences containing one or more and no more than 3 amino acid substitutions (e.g., conservative substitutions), deletions or insertions relative to said sequences; or

18) HCDR1, HCDR2, HCDR3 comprising the sequences as shown in SEQ ID NO: 1, 2 and 3, or sequences containing one or more and no more than 3 amino acid substitutions (e.g., conservative substitutions), deletions or insertions relative to said sequences; and LCDR1, LCDR2, LCDR3 comprising the sequences as shown in SEQ ID NO: 49, 50 and 30, or sequences containing one or more and no more than 3 amino acid substitutions (e.g., conservative substitutions), deletions or insertions relative to said sequences.

In one embodiment, the present invention provides an anti-ROR1 antibody specifically binding to ROR1 and antigen-binding fragment thereof, comprising a heavy chain variable region, wherein:

1) the heavy chain variable region comprises the amino acid sequence as shown in SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57;

2) the heavy chain variable region comprises the amino acid sequence as shown in SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55;

3) the heavy chain variable region comprises the amino acid sequence as shown in SEQ ID NO: 59, or an amino acid sequence having at least 90%, 91%, 92%, 93%, 94%, 95%, 96%, 97%, 98% or 99% identity to the amino acid sequence of SEQ ID NO: 59, or consists of SEQ ID NO: 59;

4) the heavy chain variable region comprises the amino acid sequence as shown in SEQ ID NO: 61, or an amino acid sequence having at least 90%, 91%, 92%, 93%, 94%, 95%, 96%, 97%, 98% or 99% identity to the amino acid sequence of SEQ ID NO: 61, or consists of SEQ ID NO: 61;

5) the heavy chain variable region comprises the amino acid sequence as shown in SEQ ID NO: 63, or an amino acid sequence having at least 90%, 91%, 92%, 93%, 94%, 95%, 96%, 97%, 98% or 99% identity to the amino acid sequence of SEQ ID NO: 63, or consists of SEQ ID NO: 63;

6) the heavy chain variable region comprises the amino acid sequence as shown in SEQ ID NO: 65, or an amino acid sequence having at least 90%, 91%, 92%, 93%, 94%, 95%, 96%, 97%, 98% or 99% identity to the amino acid sequence of SEQ ID NO: 65, or consists of SEQ ID NO: 65;

7) the heavy chain variable region comprises the amino acid sequence as shown in SEQ ID NO: 67, or an amino acid sequence having at least 90%, 91%, 92%, 93%, 94%, 95%, 96%, 97%, 98% or 99% identity to the amino acid sequence of SEQ ID NO: 67, or consists of SEQ ID NO: 67;

8) the heavy chain variable region comprises the amino acid sequence as shown in SEQ ID NO: 68, or an amino acid sequence having at least 90%, 91%, 92%, 93%, 94%, 95%, 96%, 97%, 98% or 99% identity to the amino acid

sequence of SEQ ID NO: 68, or consists of SEQ ID NO: 68;

9) the heavy chain variable region comprises the amino acid sequence as shown in SEQ ID NO: 70, or an amino acid sequence having at least 90%, 91%, 92%, 93%, 94%, 95%, 96%, 97%, 98% or 99% identity to the amino acid sequence of SEQ ID NO: 70, or consists of SEQ ID NO: 70;

10) the heavy chain variable region comprises the amino acid sequence as shown in SEQ ID NO: 72, or an amino acid sequence having at least 90%, 91%, 92%, 93%, 94%, 95%, 96%, 97%, 98% or 99% identity to the amino acid sequence of SEQ ID NO: 72, or consists of SEQ ID NO: 72;

11) the heavy chain variable region comprises the amino acid sequence as shown in SEQ ID NO: 74, or an amino acid sequence having at least 90%, 91%, 92%, 93%, 94%, 95%, 96%, 97%, 98% or 99% identity to the amino acid sequence of SEQ ID NO: 74, or consists of SEQ ID NO: 74;

12) the heavy chain variable region comprises the amino acid sequence as shown in SEQ ID NO: 83, or an amino acid sequence having at least 90%, 91%, 92%, 93%, 94%, 95%, 96%, 97%, 98% or 99% identity to the amino acid sequence of SEQ ID NO: 83, or consists of SEQ ID NO: 83;

13) the heavy chain variable region comprises the amino acid sequence as shown in SEQ ID NO: 84, or an amino acid sequence having at least 90%, 91%, 92%, 93%, 94%, 95%, 96%, 97%, 98% or 99% identity to the amino acid sequence of SEQ ID NO: 84, or consists of SEQ ID NO: 84;

14) the heavy chain variable region comprises the amino acid sequence as shown in SEQ ID NO: 85, or an amino acid sequence having at least 90%, 91%, 92%, 93%, 94%, 95%, 96%, 97%, 98% or 99% identity to the amino acid sequence of SEQ ID NO: 85, or consists of SEQ ID NO: 85;

15) the heavy chain variable region comprises the amino acid sequence as shown in SEQ ID NO: 75, or an amino acid sequence having at least 90%, 91%, 92%, 93%, 94%, 95%, 96%, 97%, 98% or 99% identity to the amino acid sequence of SEQ ID NO: 75, or consists of SEQ ID NO: 75;

16) the heavy chain variable region comprises the amino acid sequence as shown in SEQ ID NO: 76, or an amino acid sequence having at least 90%, 91%, 92%, 93%, 94%, 95%, 96%, 97%, 98% or 99% identity to the amino acid sequence of SEQ ID NO: 76, or consists of SEQ ID NO: 76;

17) the heavy chain variable region comprises the amino acid sequence as shown in SEQ ID NO: 77, or an amino acid sequence having at least 90%, 91%, 92%, 93%, 94%, 95%, 96%, 97%, 98% or 99% identity to the amino acid sequence of SEQ ID NO: 77, or consists of SEQ ID NO: 77; or

18) the heavy chain variable region comprises the amino acid sequence as shown in SEQ ID NO: 78, or an amino acid sequence having at least 90%, 91%, 92%, 93%, 94%, 95%, 96%, 97%, 98% or 99% identity to the amino acid sequence of SEQ ID NO: 78, or consists of SEQ ID NO: 78.

In one embodiment, the present invention provides an anti-ROR1 antibody specifically binding to ROR1 and antigen-binding fragment thereof, comprising a light chain variable region, wherein:

1) the light chain variable region comprises the amino acid sequence as shown in SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;

2) the light chain variable region comprises the amino acid sequence as shown in SEQ ID NO: 54, or an amino acid sequence having at least 90%, 91%, 92%, 93%, 94%, 95%, 96%, 97%, 98% or 99% identity to the amino acid sequence of SEQ ID NO: 54, or consists of SEQ ID NO: 54;

3) the light chain variable region comprises the amino acid sequence as shown in SEQ ID NO: 58, or an amino acid sequence having at least 90%, 91%, 92%, 93%, 94%, 95%, 96%, 97%, 98% or 99% identity to the amino acid sequence of SEQ ID NO: 58, or consists of SEQ ID NO: 58;

4) the light chain variable region comprises the amino acid sequence as shown in SEQ ID NO: 60, or an amino acid sequence having at least 90%, 91%, 92%, 93%, 94%, 95%, 96%, 97%, 98% or 99% identity to the amino acid sequence of SEQ ID NO: 60, or consists of SEQ ID NO: 60;

5) the light chain variable region comprises the amino acid sequence as shown in SEQ ID NO: 62, or an amino acid sequence having at least 90%, 91%, 92%, 93%, 94%, 95%, 96%, 97%, 98% or 99% identity to the amino acid sequence of SEQ ID NO: 62, or consists of SEQ ID NO: 62;

6) the light chain variable region comprises the amino acid sequence as shown in SEQ ID NO: 64, or an amino acid sequence having at least 90%, 91%, 92%, 93%, 94%, 95%, 96%, 97%, 98% or 99% identity to the amino acid sequence of SEQ ID NO: 64, or consists of SEQ ID NO: 64;

7) the light chain variable region comprises the amino acid sequence as shown in SEQ ID NO: 66, or an amino acid sequence having at least 90%, 91%, 92%, 93%, 94%, 95%, 96%, 97%, 98% or 99% identity to the amino acid sequence of SEQ ID NO: 66, or consists of SEQ ID NO: 66;

8) the light chain variable region comprises the amino acid sequence as shown in SEQ ID NO: 69, or an amino acid sequence having at least 90%, 91%, 92%, 93%, 94%, 95%, 96%, 97%, 98% or 99% identity to the amino acid

sequence of SEQ ID NO: 69, or consists of SEQ ID NO: 69;

9) the light chain variable region comprises the amino acid sequence as shown in SEQ ID NO: 71, or an amino acid sequence having at least 90%, 91%, 92%, 93%, 94%, 95%, 96%, 97%, 98% or 99% identity to the amino acid sequence of SEQ ID NO: 71, or consists of SEQ ID NO: 71;

10) the light chain variable region comprises the amino acid sequence as shown in SEQ ID NO: 73, or an amino acid sequence having at least 90%, 91%, 92%, 93%, 94%, 95%, 96%, 97%, 98% or 99% identity to the amino acid sequence of SEQ ID NO: 73, or consists of SEQ ID NO: 73;

11) the light chain variable region comprises the amino acid sequence as shown in SEQ ID NO: 79, or an amino acid sequence having at least 90%, 91%, 92%, 93%, 94%, 95%, 96%, 97%, 98% or 99% identity to the amino acid sequence of SEQ ID NO: 79, or consists of SEQ ID NO: 79;

12) the light chain variable region comprises the amino acid sequence as shown in SEQ ID NO: 80, or an amino acid sequence having at least 90%, 91%, 92%, 93%, 94%, 95%, 96%, 97%, 98% or 99% identity to the amino acid sequence of SEQ ID NO: 80, or consists of SEQ ID NO: 80;

13) the light chain variable region comprises the amino acid sequence as shown in SEQ ID NO: 81, or an amino acid sequence having at least 90%, 91%, 92%, 93%, 94%, 95%, 96%, 97%, 98% or 99% identity to the amino acid sequence of SEQ ID NO: 81, or consists of SEQ ID NO: 81;

14) the light chain variable region comprises the amino acid sequence as shown in SEQ ID NO: 82, or an amino acid sequence having at least 90%, 91%, 92%, 93%, 94%, 95%, 96%, 97%, 98% or 99% identity to the amino acid sequence of SEQ ID NO: 82, or consists of SEQ ID NO: 82;

15) the light chain variable region comprises the amino acid sequence as shown in SEQ ID NO: 86, or an amino acid sequence having at least 90%, 91%, 92%, 93%, 94%, 95%, 96%, 97%, 98% or 99% identity to the amino acid sequence of SEQ ID NO: 86, or consists of SEQ ID NO: 86;

16) the light chain variable region comprises the amino acid sequence as shown in SEQ ID NO: 87, or an amino acid sequence having at least 90%, 91%, 92%, 93%, 94%, 95%, 96%, 97%, 98% or 99% identity to the amino acid sequence of SEQ ID NO: 87, or consists of SEQ ID NO: 87;

17) the light chain variable region comprises the amino acid sequence as shown in SEQ ID NO: 88, or an amino acid sequence having at least 90%, 91%, 92%, 93%, 94%, 95%, 96%, 97%, 98% or 99% identity to the amino acid sequence of SEQ ID NO: 88, or consists of SEQ ID NO: 88; or

18) the light chain variable region comprises the amino acid sequence as shown in SEQ ID NO: 89, or an amino acid sequence having at least 90%, 91%, 92%, 93%, 94%, 95%, 96%, 97%, 98% or 99% identity to the amino acid sequence of SEQ ID NO: 89, or consists of SEQ ID NO: 89.

In another embodiment, the present invention provides an anti-ROR1 antibody specifically binding to ROR1 and antigen-binding fragment thereof, comprising a heavy chain variable region and a light chain variable region, wherein:

1) the heavy chain variable region comprises the amino acid sequence as shown in SEQ ID NO: 57, or an amino acid sequence having at least 90%, 91%, 92%, 93%, 94%, 95%, 96%, 97%, 98% or 99% identity to the amino acid sequence of SEQ ID NO: 57, or consists of SEQ ID NO: 57, and the light chain variable region comprises the amino acid sequence as shown in SEQ ID NO: 56, or an amino acid sequence having at least 90%, 91%, 92%, 93%, 94%, 95%, 96%, 97%, 98% or 99% identity to the amino acid sequence of SEQ ID NO: 56, or consists of SEQ ID NO: 56;

2) the heavy chain variable region comprises the amino acid sequence as shown in SEQ ID NO: 55, or an amino acid sequence having at least 90%, 91%, 92%, 93%, 94%, 95%, 96%, 97%, 98% or 99% identity to the amino acid sequence of SEQ ID NO: 55, or consists of SEQ ID NO: 55, and the light chain variable region comprises the amino acid sequence as shown in SEQ ID NO: 54, or an amino acid sequence having at least 90%, 91%, 92%, 93%, 94%, 95%, 96%, 97%, 98% or 99% identity to the amino acid sequence of SEQ ID NO: 54, or consists of SEQ ID NO: 54;

3) the heavy chain variable region comprises the amino acid sequence as shown in SEQ ID NO: 59, or an amino acid sequence having at least 90%, 91%, 92%, 93%, 94%, 95%, 96%, 97%, 98% or 99% identity to the amino acid sequence of SEQ ID NO: 59, or consists of SEQ ID NO: 59, and the light chain variable region comprises the amino acid sequence as shown in SEQ ID NO: 58, or an amino acid sequence having at least 90%, 91%, 92%, 93%, 94%, 95%, 96%, 97%, 98% or 99% identity to the amino acid sequence of SEQ ID NO: 58, or consists of SEQ ID NO: 58;

4) the heavy chain variable region comprises the amino acid sequence as shown in SEQ ID NO: 61, or an amino acid sequence having at least 90%, 91%, 92%, 93%, 94%, 95%, 96%, 97%, 98% or 99% identity to the amino acid sequence of SEQ ID NO: 61, or consists of SEQ ID NO: 61, and the light chain variable region comprises the amino acid sequence as shown in SEQ ID NO: 60, or an amino acid sequence having at least 90%, 91%, 92%, 93%, 94%, 95%, 96%, 97%, 98% or 99% identity to the amino acid sequence of SEQ ID NO: 60, or consists of SEQ ID NO: 60;

5) the heavy chain variable region comprises the amino acid sequence as shown in SEQ ID NO: 63, or an amino acid sequence having at least 90%, 91%, 92%, 93%, 94%, 95%, 96%, 97%, 98% or 99% identity to the amino acid sequence of SEQ ID NO: 63, or consists of SEQ ID NO: 63, and the light chain variable region comprises the amino

acid sequence as shown in SEQ ID NO: 62, or an amino acid sequence having at least 90%, 91%, 92%, 93%, 94%, 95%, 96%, 97%, 98% or 99% identity to the amino acid sequence of SEQ ID NO: 62, or consists of SEQ ID NO: 62;
6) the heavy chain variable region comprises the amino acid sequence as shown in SEQ ID NO: 65, or an amino acid sequence having at least 90%, 91%, 92%, 93%, 94%, 95%, 96%, 97%, 98% or 99% identity to the amino acid sequence of SEQ ID NO: 65, or consists of SEQ ID NO: 65, and the light chain variable region comprises the amino acid sequence as shown in SEQ ID NO: 64, or an amino acid sequence having at least 90%, 91%, 92%, 93%, 94%, 95%, 96%, 97%, 98% or 99% identity to the amino acid sequence of SEQ ID NO: 64, or consists of SEQ ID NO: 64;
7) the heavy chain variable region comprises the amino acid sequence as shown in SEQ ID NO: 67, or an amino acid sequence having at least 90%, 91%, 92%, 93%, 94%, 95%, 96%, 97%, 98% or 99% identity to the amino acid sequence of SEQ ID NO: 67, or consists of SEQ ID NO: 67, and the light chain variable region comprises the amino acid sequence as shown in SEQ ID NO: 66, or an amino acid sequence having at least 90%, 91%, 92%, 93%, 94%, 95%, 96%, 97%, 98% or 99% identity to the amino acid sequence of SEQ ID NO: 66, or consists of SEQ ID NO: 66;
8) the heavy chain variable region comprises the amino acid sequence as shown in SEQ ID NO: 68, or an amino acid sequence having at least 90%, 91%, 92%, 93%, 94%, 95%, 96%, 97%, 98% or 99% identity to the amino acid sequence of SEQ ID NO: 68, or consists of SEQ ID NO: 68, and the light chain variable region comprises the amino acid sequence as shown in SEQ ID NO: 66, or an amino acid sequence having at least 90%, 91%, 92%, 93%, 94%, 95%, 96%, 97%, 98% or 99% identity to the amino acid sequence of SEQ ID NO: 66, or consists of SEQ ID NO: 66;
9) the heavy chain variable region comprises the amino acid sequence as shown in SEQ ID NO: 70, or an amino acid sequence having at least 90%, 91%, 92%, 93%, 94%, 95%, 96%, 97%, 98% or 99% identity to the amino acid sequence of SEQ ID NO: 70, or consists of SEQ ID NO: 70, and the light chain variable region comprises the amino acid sequence as shown in SEQ ID NO: 69, or an amino acid sequence having at least 90%, 91%, 92%, 93%, 94%, 95%, 96%, 97%, 98% or 99% identity to the amino acid sequence of SEQ ID NO: 69, or consists of SEQ ID NO: 69;
10) the heavy chain variable region comprises the amino acid sequence as shown in SEQ ID NO: 72, or an amino acid sequence having at least 90%, 91%, 92%, 93%, 94%, 95%, 96%, 97%, 98% or 99% identity to the amino acid sequence of SEQ ID NO: 72, or consists of SEQ ID NO: 72, and the light chain variable region comprises the amino acid sequence as shown in SEQ ID NO: 71, or an amino acid sequence having at least 90%, 91%, 92%, 93%, 94%, 95%, 96%, 97%, 98% or 99% identity to the amino acid sequence of SEQ ID NO: 71, or consists of SEQ ID NO: 71;
11) the heavy chain variable region comprises the amino acid sequence as shown in SEQ ID NO: 74, or an amino acid sequence having at least 90%, 91%, 92%, 93%, 94%, 95%, 96%, 97%, 98% or 99% identity to the amino acid sequence of SEQ ID NO: 74, or consists of SEQ ID NO: 74, and the light chain variable region comprises the amino acid sequence as shown in SEQ ID NO: 73, or an amino acid sequence having at least 90%, 91%, 92%, 93%, 94%, 95%, 96%, 97%, 98% or 99% identity to the amino acid sequence of SEQ ID NO: 73, or consists of SEQ ID NO: 73;
12) the heavy chain variable region comprises the amino acid sequence as shown in SEQ ID NO: 75, or an amino acid sequence having at least 90%, 91%, 92%, 93%, 94%, 95%, 96%, 97%, 98% or 99% identity to the amino acid sequence of SEQ ID NO: 75, or consists of SEQ ID NO: 75, and the light chain variable region comprises the amino acid sequence as shown in SEQ ID NO: 79, or an amino acid sequence having at least 90%, 91%, 92%, 93%, 94%, 95%, 96%, 97%, 98% or 99% identity to the amino acid sequence of SEQ ID NO: 79, or consists of SEQ ID NO: 79;
13) the heavy chain variable region comprises the amino acid sequence as shown in SEQ ID NO: 76, or an amino acid sequence having at least 90%, 91%, 92%, 93%, 94%, 95%, 96%, 97%, 98% or 99% identity to the amino acid sequence of SEQ ID NO: 76, or consists of SEQ ID NO: 76, and the light chain variable region comprises the amino acid sequence as shown in SEQ ID NO: 80, or an amino acid sequence having at least 90%, 91%, 92%, 93%, 94%, 95%, 96%, 97%, 98% or 99% identity to the amino acid sequence of SEQ ID NO: 80, or consists of SEQ ID NO: 80;
14) the heavy chain variable region comprises the amino acid sequence as shown in SEQ ID NO: 76, or an amino acid sequence having at least 90%, 91%, 92%, 93%, 94%, 95%, 96%, 97%, 98% or 99% identity to the amino acid sequence of SEQ ID NO: 76, or consists of SEQ ID NO: 76, and the light chain variable region comprises the amino acid sequence as shown in SEQ ID NO: 81, or an amino acid sequence having at least 90%, 91%, 92%, 93%, 94%, 95%, 96%, 97%, 98% or 99% identity to the amino acid sequence of SEQ ID NO: 81, or consists of SEQ ID NO: 81;
15) the heavy chain variable region comprises the amino acid sequence as shown in SEQ ID NO: 77, or an amino acid sequence having at least 90%, 91%, 92%, 93%, 94%, 95%, 96%, 97%, 98% or 99% identity to the amino acid sequence of SEQ ID NO: 77, or consists of SEQ ID NO: 77, and the light chain variable region comprises the amino acid sequence as shown in SEQ ID NO: 80, or an amino acid sequence having at least 90%, 91%, 92%, 93%, 94%, 95%, 96%, 97%, 98% or 99% identity to the amino acid sequence of SEQ ID NO: 80, or consists of SEQ ID NO: 80;
16) the heavy chain variable region comprises the amino acid sequence as shown in SEQ ID NO: 77, or an amino acid sequence having at least 90%, 91%, 92%, 93%, 94%, 95%, 96%, 97%, 98% or 99% identity to the amino acid sequence of SEQ ID NO: 77, or consists of SEQ ID NO: 77, and the light chain variable region comprises the amino acid sequence as shown in SEQ ID NO: 81, or an amino acid sequence having at least 90%, 91%, 92%, 93%, 94%, 95%, 96%, 97%, 98% or 99% identity to the amino acid sequence of SEQ ID NO: 81, or consists of SEQ ID NO: 81;
17) the heavy chain variable region comprises the amino acid sequence as shown in SEQ ID NO: 77, or an amino acid

sequence having at least 90%, 91%, 92%, 93%, 94%, 95%, 96%, 97%, 98% or 99% identity to the amino acid sequence of SEQ ID NO: 77, or consists of SEQ ID NO: 77, and the light chain variable region comprises the amino acid sequence as shown in SEQ ID NO: 82, or an amino acid sequence having at least 90%, 91%, 92%, 93%, 94%, 95%, 96%, 97%, 98% or 99% identity to the amino acid sequence of SEQ ID NO: 82, or consists of SEQ ID NO: 82;
18) the heavy chain variable region comprises the amino acid sequence as shown in SEQ ID NO: 78, or an amino acid sequence having at least 90%, 91%, 92%, 93%, 94%, 95%, 96%, 97%, 98% or 99% identity to the amino acid sequence of SEQ ID NO: 78, or consists of SEQ ID NO: 78, and the light chain variable region comprises the amino acid sequence as shown in SEQ ID NO: 80, or an amino acid sequence having at least 90%, 91%, 92%, 93%, 94%, 95%, 96%, 97%, 98% or 99% identity to the amino acid sequence of SEQ ID NO: 80, or consists of SEQ ID NO: 80;
19) the heavy chain variable region comprises the amino acid sequence as shown in SEQ ID NO: 78, or an amino acid sequence having at least 90%, 91%, 92%, 93%, 94%, 95%, 96%, 97%, 98% or 99% identity to the amino acid sequence of SEQ ID NO: 78, or consists of SEQ ID NO: 78, and the light chain variable region comprises the amino acid sequence as shown in SEQ ID NO: 81, or an amino acid sequence having at least 90%, 91%, 92%, 93%, 94%, 95%, 96%, 97%, 98% or 99% identity to the amino acid sequence of SEQ ID NO: 81, or consists of SEQ ID NO: 81;
20) the heavy chain variable region comprises the amino acid sequence as shown in SEQ ID NO: 78, or an amino acid sequence having at least 90%, 91%, 92%, 93%, 94%, 95%, 96%, 97%, 98% or 99% identity to the amino acid sequence of SEQ ID NO: 78, or consists of SEQ ID NO: 78, and the light chain variable region comprises the amino acid sequence as shown in SEQ ID NO: 82, or an amino acid sequence having at least 90%, 91%, 92%, 93%, 94%, 95%, 96%, 97%, 98% or 99% identity to the amino acid sequence of SEQ ID NO: 82, or consists of SEQ ID NO: 82;
21) the heavy chain variable region comprises the amino acid sequence as shown in SEQ ID NO: 83, or an amino acid sequence having at least 90%, 91%, 92%, 93%, 94%, 95%, 96%, 97%, 98% or 99% identity to the amino acid sequence of SEQ ID NO: 83, or consists of SEQ ID NO: 83, and the light chain variable region comprises the amino acid sequence as shown in SEQ ID NO: 86, or an amino acid sequence having at least 90%, 91%, 92%, 93%, 94%, 95%, 96%, 97%, 98% or 99% identity to the amino acid sequence of SEQ ID NO: 86, or consists of SEQ ID NO: 86;
22) the heavy chain variable region comprises the amino acid sequence as shown in SEQ ID NO: 83, or an amino acid sequence having at least 90%, 91%, 92%, 93%, 94%, 95%, 96%, 97%, 98% or 99% identity to the amino acid sequence of SEQ ID NO: 83, or consists of SEQ ID NO: 83, and the light chain variable region comprises the amino acid sequence as shown in SEQ ID NO: 87, or an amino acid sequence having at least 90%, 91%, 92%, 93%, 94%, 95%, 96%, 97%, 98% or 99% identity to the amino acid sequence of SEQ ID NO: 87, or consists of SEQ ID NO: 87;
23) the heavy chain variable region comprises the amino acid sequence as shown in SEQ ID NO: 84, or an amino acid sequence having at least 90%, 91%, 92%, 93%, 94%, 95%, 96%, 97%, 98% or 99% identity to the amino acid sequence of SEQ ID NO: 84, or consists of SEQ ID NO: 84, and the light chain variable region comprises the amino acid sequence as shown in SEQ ID NO: 87, or an amino acid sequence having at least 90%, 91%, 92%, 93%, 94%, 95%, 96%, 97%, 98% or 99% identity to the amino acid sequence of SEQ ID NO: 87, or consists of SEQ ID NO: 87;
24) the heavy chain variable region comprises the amino acid sequence as shown in SEQ ID NO: 84, or an amino acid sequence having at least 90%, 91%, 92%, 93%, 94%, 95%, 96%, 97%, 98% or 99% identity to the amino acid sequence of SEQ ID NO: 84, or consists of SEQ ID NO: 84, and the light chain variable region comprises the amino acid sequence as shown in SEQ ID NO: 88, or an amino acid sequence having at least 90%, 91%, 92%, 93%, 94%, 95%, 96%, 97%, 98% or 99% identity to the amino acid sequence of SEQ ID NO: 88, or consists of SEQ ID NO: 88;
25) the heavy chain variable region comprises the amino acid sequence as shown in SEQ ID NO: 84, or an amino acid sequence having at least 90%, 91%, 92%, 93%, 94%, 95%, 96%, 97%, 98% or 99% identity to the amino acid sequence of SEQ ID NO: 84, or consists of SEQ ID NO: 84, and the light chain variable region comprises the amino acid sequence as shown in SEQ ID NO: 89, or an amino acid sequence having at least 90%, 91%, 92%, 93%, 94%, 95%, 96%, 97%, 98% or 99% identity to the amino acid sequence of SEQ ID NO: 89, or consists of SEQ ID NO: 89;
26) the heavy chain variable region comprises the amino acid sequence as shown in SEQ ID NO: 85, or an amino acid sequence having at least 90%, 91%, 92%, 93%, 94%, 95%, 96%, 97%, 98% or 99% identity to the amino acid sequence of SEQ ID NO: 85, or consists of SEQ ID NO: 85, and the light chain variable region comprises the amino acid sequence as shown in SEQ ID NO: 87, or an amino acid sequence having at least 90%, 91%, 92%, 93%, 94%, 95%, 96%, 97%, 98% or 99% identity to the amino acid sequence of SEQ ID NO: 87, or consists of SEQ ID NO: 87;
27) the heavy chain variable region comprises the amino acid sequence as shown in SEQ ID NO: 85, or an amino acid sequence having at least 90%, 91%, 92%, 93%, 94%, 95%, 96%, 97%, 98% or 99% identity to the amino acid sequence of SEQ ID NO: 85, or consists of SEQ ID NO: 85, and the light chain variable region comprises the amino acid sequence as shown in SEQ ID NO: 88, or an amino acid sequence having at least 90%, 91%, 92%, 93%, 94%, 95%, 96%, 97%, 98% or 99% identity to the amino acid sequence of SEQ ID NO: 88, or consists of SEQ ID NO: 88; or
28) the heavy chain variable region comprises the amino acid sequence as shown in SEQ ID NO: 85, or an amino acid sequence having at least 90%, 91%, 92%, 93%, 94%, 95%, 96%, 97%, 98% or 99% identity to the amino acid sequence of SEQ ID NO: 85, or consists of SEQ ID NO: 85, and the light chain variable region comprises the amino acid sequence as shown in SEQ ID NO: 89, or an amino acid sequence having at least 90%, 91%, 92%, 93%, 94%,

95%, 96%, 97%, 98% or 99% identity to the amino acid sequence of SEQ ID NO: 89, or consists of SEQ ID NO: 89.

In some embodiments, the above mentioned antibody or antigen-binding fragment thereof further comprises a heavy chain and/or light chain constant region sequence derived from human antibody germline consensus sequence. The light chain constant region is preferably a human kappa or lambda chain constant region. The heavy chain constant region can be $\gamma$, $\mu$, $\alpha$, $\delta$, or $\varepsilon$ chain. In some embodiments, the heavy chain constant region is preferably derived from the constant region sequence of human IgG1, IgG2, IgG3, or IgG4. In one embodiment, the light chain constant region comprises the sequence shown in SEQ ID NO: 53, or consists of the sequence. In another embodiment, the heavy chain constant region comprises the sequence shown in SEQ ID NO: 52.

It will be appreciated that sequence variants of these constant region domains may also be used, for example comprising one or more amino acid modifications, wherein amino acid positions are identified according to the EU Index System of Kabat *et al.* (1991).

In a specific embodiment, the present invention provides an anti-ROR1 antibody specifically binding to ROR1 and antigen-binding fragment thereof, comprising a heavy chain and a light chain, wherein:

1) the heavy chain comprises the amino acid sequence as shown in SEQ ID NO: 93, or an amino acid sequence having at least 90%, 91%, 92%, 93%, 94%, 95%, 96%, 97%, 98% or 99% identity to the amino acid sequence of SEQ ID NO: 93, or consists of SEQ ID NO: 93, and the light chain comprises the amino acid sequence as shown in SEQ ID NO: 92, or an amino acid sequence having at least 90%, 91%, 92%, 93%, 94%, 95%, 96%, 97%, 98% or 99% identity to the amino acid sequence of SEQ ID NO: 92, or consists of SEQ ID NO: 92;
2) the heavy chain comprises the amino acid sequence as shown in SEQ ID NO: 91, or an amino acid sequence having at least 90%, 91%, 92%, 93%, 94%, 95%, 96%, 97%, 98% or 99% identity to the amino acid sequence of SEQ ID NO: 91, or consists of SEQ ID NO: 91, and the light chain comprises the amino acid sequence as shown in SEQ ID NO: 90, or an amino acid sequence having at least 90%, 91%, 92%, 93%, 94%, 95%, 96%, 97%, 98% or 99% identity to the amino acid sequence of SEQ ID NO: 90, or consists of SEQ ID NO: 90;
3) the heavy chain comprises the amino acid sequence as shown in SEQ ID NO: 95, or an amino acid sequence having at least 90%, 91%, 92%, 93%, 94%, 95%, 96%, 97%, 98% or 99% identity to the amino acid sequence of SEQ ID NO: 95, or consists of SEQ ID NO: 95, and the light chain comprises the amino acid sequence as shown in SEQ ID NO: 94, or an amino acid sequence having at least 90%, 91%, 92%, 93%, 94%, 95%, 96%, 97%, 98% or 99% identity to the amino acid sequence of SEQ ID NO: 94, or consists of SEQ ID NO: 94;
4) the heavy chain comprises the amino acid sequence as shown in SEQ ID NO: 97, or an amino acid sequence having at least 90%, 91%, 92%, 93%, 94%, 95%, 96%, 97%, 98% or 99% identity to the amino acid sequence of SEQ ID NO: 97, or consists of SEQ ID NO: 97, and the light chain comprises the amino acid sequence as shown in SEQ ID NO: 96, or an amino acid sequence having at least 90%, 91%, 92%, 93%, 94%, 95%, 96%, 97%, 98% or 99% identity to the amino acid sequence of SEQ ID NO: 96, or consists of SEQ ID NO: 96;
5) the heavy chain comprises the amino acid sequence as shown in SEQ ID NO: 99, or an amino acid sequence having at least 90%, 91%, 92%, 93%, 94%, 95%, 96%, 97%, 98% or 99% identity to the amino acid sequence of SEQ ID NO: 99, or consists of SEQ ID NO: 99, and the light chain comprises the amino acid sequence as shown in SEQ ID NO: 98, or an amino acid sequence having at least 90%, 91%, 92%, 93%, 94%, 95%, 96%, 97%, 98% or 99% identity to the amino acid sequence of SEQ ID NO: 98, or consists of SEQ ID NO: 98;
6) the heavy chain comprises the amino acid sequence as shown in SEQ ID NO: 101, or an amino acid sequence having at least 90%, 91%, 92%, 93%, 94%, 95%, 96%, 97%, 98% or 99% identity to the amino acid sequence of SEQ ID NO: 101, or consists of SEQ ID NO: 101, and the light chain comprises the amino acid sequence as shown in SEQ ID NO: 100, or an amino acid sequence having at least 90%, 91%, 92%, 93%, 94%, 95%, 96%, 97%, 98% or 99% identity to the amino acid sequence of SEQ ID NO: 100, or consists of SEQ ID NO: 100;
7) the heavy chain comprises the amino acid sequence as shown in SEQ ID NO: 103, or an amino acid sequence having at least 90%, 91%, 92%, 93%, 94%, 95%, 96%, 97%, 98% or 99% identity to the amino acid sequence of SEQ ID NO: 103, or consists of SEQ ID NO: 103, and the light chain comprises the amino acid sequence as shown in SEQ ID NO: 102, or an amino acid sequence having at least 90%, 91%, 92%, 93%, 94%, 95%, 96%, 97%, 98% or 99% identity to the amino acid sequence of SEQ ID NO: 102, or consists of SEQ ID NO: 102;
8) the heavy chain comprises the amino acid sequence as shown in SEQ ID NO: 104, or an amino acid sequence having at least 90%, 91%, 92%, 93%, 94%, 95%, 96%, 97%, 98% or 99% identity to the amino acid sequence of SEQ ID NO: 104, or consists of SEQ ID NO: 104, and the light chain comprises the amino acid sequence as shown in SEQ ID NO: 102, or an amino acid sequence having at least 90%, 91%, 92%, 93%, 94%, 95%, 96%, 97%, 98% or 99% identity to the amino acid sequence of SEQ ID NO: 102, or consists of SEQ ID NO: 102;
9) the heavy chain comprises the amino acid sequence as shown in SEQ ID NO: 106, or an amino acid sequence having at least 90%, 91%, 92%, 93%, 94%, 95%, 96%, 97%, 98% or 99% identity to the amino acid sequence of SEQ ID NO: 106, or consists of SEQ ID NO: 106, and the light chain comprises the amino acid sequence as shown in SEQ ID

NO: 105, or an amino acid sequence having at least 90%, 91%, 92%, 93%, 94%, 95%, 96%, 97%, 98% or 99% identity to the amino acid sequence of SEQ ID NO: 105, or consists of SEQ ID NO: 105;

10) the heavy chain comprises the amino acid sequence as shown in SEQ ID NO: 108, or an amino acid sequence having at least 90%, 91%, 92%, 93%, 94%, 95%, 96%, 97%, 98% or 99% identity to the amino acid sequence of SEQ ID NO: 108, or consists of SEQ ID NO: 108, and the light chain comprises the amino acid sequence as shown in SEQ ID NO: 107, or an amino acid sequence having at least 90%, 91%, 92%, 93%, 94%, 95%, 96%, 97%, 98% or 99% identity to the amino acid sequence of SEQ ID NO: 107, or consists of SEQ ID NO: 107;

11) the heavy chain comprises the amino acid sequence as shown in SEQ ID NO: 110, or an amino acid sequence having at least 90%, 91%, 92%, 93%, 94%, 95%, 96%, 97%, 98% or 99% identity to the amino acid sequence of SEQ ID NO: 110, or consists of SEQ ID NO: 110, and the light chain comprises the amino acid sequence as shown in SEQ ID NO: 109, or an amino acid sequence having at least 90%, 91%, 92%, 93%, 94%, 95%, 96%, 97%, 98% or 99% identity to the amino acid sequence of SEQ ID NO: 109, or consists of SEQ ID NO: 109;

12) the heavy chain comprises the amino acid sequence as shown in SEQ ID NO: 111, or an amino acid sequence having at least 90%, 91%, 92%, 93%, 94%, 95%, 96%, 97%, 98% or 99% identity to the amino acid sequence of SEQ ID NO: 111, or consists of SEQ ID NO: 111, and the light chain comprises the amino acid sequence as shown in SEQ ID NO: 115, or an amino acid sequence having at least 90%, 91%, 92%, 93%, 94%, 95%, 96%, 97%, 98% or 99% identity to the amino acid sequence of SEQ ID NO: 115, or consists of SEQ ID NO: 115;

13) the heavy chain comprises the amino acid sequence as shown in SEQ ID NO: 112, or an amino acid sequence having at least 90%, 91%, 92%, 93%, 94%, 95%, 96%, 97%, 98% or 99% identity to the amino acid sequence of SEQ ID NO: 112, or consists of SEQ ID NO: 112, and the light chain comprises the amino acid sequence as shown in SEQ ID NO: 116, or an amino acid sequence having at least 90%, 91%, 92%, 93%, 94%, 95%, 96%, 97%, 98% or 99% identity to the amino acid sequence of SEQ ID NO: 116, or consists of SEQ ID NO: 116;

14) the heavy chain comprises the amino acid sequence as shown in SEQ ID NO: 112, or an amino acid sequence having at least 90%, 91%, 92%, 93%, 94%, 95%, 96%, 97%, 98% or 99% identity to the amino acid sequence of SEQ ID NO: 112, or consists of SEQ ID NO: 112, and the light chain comprises the amino acid sequence as shown in SEQ ID NO: 117, or an amino acid sequence having at least 90%, 91%, 92%, 93%, 94%, 95%, 96%, 97%, 98% or 99% identity to the amino acid sequence of SEQ ID NO: 117, or consists of SEQ ID NO: 117;

15) the heavy chain comprises the amino acid sequence as shown in SEQ ID NO: 113, or an amino acid sequence having at least 90%, 91%, 92%, 93%, 94%, 95%, 96%, 97%, 98% or 99% identity to the amino acid sequence of SEQ ID NO: 113, or consists of SEQ ID NO: 113, and the light chain comprises the amino acid sequence as shown in SEQ ID NO: 116, or an amino acid sequence having at least 90%, 91%, 92%, 93%, 94%, 95%, 96%, 97%, 98% or 99% identity to the amino acid sequence of SEQ ID NO: 116, or consists of SEQ ID NO: 116;

16) the heavy chain comprises the amino acid sequence as shown in SEQ ID NO: 113, or an amino acid sequence having at least 90%, 91%, 92%, 93%, 94%, 95%, 96%, 97%, 98% or 99% identity to the amino acid sequence of SEQ ID NO: 113, or consists of SEQ ID NO: 113, and the light chain comprises the amino acid sequence as shown in SEQ ID NO: 117, or an amino acid sequence having at least 90%, 91%, 92%, 93%, 94%, 95%, 96%, 97%, 98% or 99% identity to the amino acid sequence of SEQ ID NO: 117, or consists of SEQ ID NO: 117;

17) the heavy chain comprises the amino acid sequence as shown in SEQ ID NO: 113, or an amino acid sequence having at least 90%, 91%, 92%, 93%, 94%, 95%, 96%, 97%, 98% or 99% identity to the amino acid sequence of SEQ ID NO: 113, or consists of SEQ ID NO: 113, and the light chain comprises the amino acid sequence as shown in SEQ ID NO: 118, or an amino acid sequence having at least 90%, 91%, 92%, 93%, 94%, 95%, 96%, 97%, 98% or 99% identity to the amino acid sequence of SEQ ID NO: 118, or consists of SEQ ID NO: 118;

18) the heavy chain comprises the amino acid sequence as shown in SEQ ID NO: 114, or an amino acid sequence having at least 90%, 91%, 92%, 93%, 94%, 95%, 96%, 97%, 98% or 99% identity to the amino acid sequence of SEQ ID NO: 114, or consists of SEQ ID NO: 114, and the light chain comprises the amino acid sequence as shown in SEQ ID NO: 116, or an amino acid sequence having at least 90%, 91%, 92%, 93%, 94%, 95%, 96%, 97%, 98% or 99% identity to the amino acid sequence of SEQ ID NO: 116, or consists of SEQ ID NO: 116;

19) the heavy chain comprises the amino acid sequence as shown in SEQ ID NO: 114, or an amino acid sequence having at least 90%, 91%, 92%, 93%, 94%, 95%, 96%, 97%, 98% or 99% identity to the amino acid sequence of SEQ ID NO: 114, or consists of SEQ ID NO: 114, and the light chain comprises the amino acid sequence as shown in SEQ ID NO: 117, or an amino acid sequence having at least 90%, 91%, 92%, 93%, 94%, 95%, 96%, 97%, 98% or 99% identity to the amino acid sequence of SEQ ID NO: 117, or consists of SEQ ID NO: 117;

20) the heavy chain comprises the amino acid sequence as shown in SEQ ID NO: 114, or an amino acid sequence having at least 90%, 91%, 92%, 93%, 94%, 95%, 96%, 97%, 98% or 99% identity to the amino acid sequence of SEQ ID NO: 114, or consists of SEQ ID NO: 114, and the light chain comprises the amino acid sequence as shown in SEQ ID NO: 118, or an amino acid sequence having at least 90%, 91%, 92%, 93%, 94%, 95%, 96%, 97%, 98% or 99% identity to the amino acid sequence of SEQ ID NO: 118, or consists of SEQ ID NO: 118;

21) the heavy chain comprises the amino acid sequence as shown in SEQ ID NO: 119, or an amino acid sequence

having at least 90%, 91%, 92%, 93%, 94%, 95%, 96%, 97%, 98% or 99% identity to the amino acid sequence of SEQ ID NO: 119, or consists of SEQ ID NO: 119, and the light chain comprises the amino acid sequence as shown in SEQ ID NO: 122, or an amino acid sequence having at least 90%, 91%, 92%, 93%, 94%, 95%, 96%, 97%, 98% or 99% identity to the amino acid sequence of SEQ ID NO: 122, or consists of SEQ ID NO: 122;

22) the heavy chain comprises the amino acid sequence as shown in SEQ ID NO: 119, or an amino acid sequence having at least 90%, 91%, 92%, 93%, 94%, 95%, 96%, 97%, 98% or 99% identity to the amino acid sequence of SEQ ID NO: 119, or consists of SEQ ID NO: 119, and the light chain comprises the amino acid sequence as shown in SEQ ID NO: 123, or an amino acid sequence having at least 90%, 91%, 92%, 93%, 94%, 95%, 96%, 97%, 98% or 99% identity to the amino acid sequence of SEQ ID NO: 123, or consists of SEQ ID NO: 123;

23) the heavy chain comprises the amino acid sequence as shown in SEQ ID NO: 120, or an amino acid sequence having at least 90%, 91%, 92%, 93%, 94%, 95%, 96%, 97%, 98% or 99% identity to the amino acid sequence of SEQ ID NO: 120, or consists of SEQ ID NO: 120, and the light chain comprises the amino acid sequence as shown in SEQ ID NO: 123, or an amino acid sequence having at least 90%, 91%, 92%, 93%, 94%, 95%, 96%, 97%, 98% or 99% identity to the amino acid sequence of SEQ ID NO: 123, or consists of SEQ ID NO: 123;

24) the heavy chain comprises the amino acid sequence as shown in SEQ ID NO: 120, or an amino acid sequence having at least 90%, 91%, 92%, 93%, 94%, 95%, 96%, 97%, 98% or 99% identity to the amino acid sequence of SEQ ID NO: 120, or consists of SEQ ID NO: 120, and the light chain comprises the amino acid sequence as shown in SEQ ID NO: 124, or an amino acid sequence having at least 90%, 91%, 92%, 93%, 94%, 95%, 96%, 97%, 98% or 99% identity to the amino acid sequence of SEQ ID NO: 124, or consists of SEQ ID NO: 124;

25) the heavy chain comprises the amino acid sequence as shown in SEQ ID NO: 120, or an amino acid sequence having at least 90%, 91%, 92%, 93%, 94%, 95%, 96%, 97%, 98% or 99% identity to the amino acid sequence of SEQ ID NO: 120, or consists of SEQ ID NO: 120, and the light chain comprises the amino acid sequence as shown in SEQ ID NO: 125, or an amino acid sequence having at least 90%, 91%, 92%, 93%, 94%, 95%, 96%, 97%, 98% or 99% identity to the amino acid sequence of SEQ ID NO: 125, or consists of SEQ ID NO: 125;

26) the heavy chain comprises the amino acid sequence as shown in SEQ ID NO: 121, or an amino acid sequence having at least 90%, 91%, 92%, 93%, 94%, 95%, 96%, 97%, 98% or 99% identity to the amino acid sequence of SEQ ID NO: 121, or consists of SEQ ID NO: 121, and the light chain comprises the amino acid sequence as shown in SEQ ID NO: 123, or an amino acid sequence having at least 90%, 91%, 92%, 93%, 94%, 95%, 96%, 97%, 98% or 99% identity to the amino acid sequence of SEQ ID NO: 123, or consists of SEQ ID NO: 123;

27) the heavy chain comprises the amino acid sequence as shown in SEQ ID NO: 121, or an amino acid sequence having at least 90%, 91%, 92%, 93%, 94%, 95%, 96%, 97%, 98% or 99% identity to the amino acid sequence of SEQ ID NO: 121, or consists of SEQ ID NO: 121, and the light chain comprises the amino acid sequence as shown in SEQ ID NO: 124, or an amino acid sequence having at least 90%, 91%, 92%, 93%, 94%, 95%, 96%, 97%, 98% or 99% identity to the amino acid sequence of SEQ ID NO: 124, or consists of SEQ ID NO: 124; or

28) the heavy chain comprises the amino acid sequence as shown in SEQ ID NO: 121, or an amino acid sequence having at least 90%, 91%, 92%, 93%, 94%, 95%, 96%, 97%, 98% or 99% identity to the amino acid sequence of SEQ ID NO: 121, or consists of SEQ ID NO: 121, and the light chain comprises the amino acid sequence as shown in SEQ ID NO: 125, or an amino acid sequence having at least 90%, 91%, 92%, 93%, 94%, 95%, 96%, 97%, 98% or 99% identity to the amino acid sequence of SEQ ID NO: 125, or consists of SEQ ID NO: 125.

In certain embodiments of any of the antibodies described above, the antibody is monoclonal.

In certain embodiments of any of the antibodies described above, the antibody is a full length antibody.

In one embodiment, the anti-ROR1 antibody of the invention is an intact antibody, such as an IgG1, IgG2, IgG3, IgG4 antibody. In another embodiment, the anti-ROR1 antibody of the invention encompass only the antigen binding portion thereof, such as: Fab, Fab'-SH, Fv, scFv or $(Fab')_2$ fragment.

In a second aspect, the present invention provides an antibody-drug conjugate targeting human ROR1, the antibody-drug conjugate having the following advantages:

(1) binding to target cells expressing ROR1 with high affinity;
(2) capable of entering cells through internalization and killing target cells; in some embodiments, the ADCs of the present invention have high internalization rate;
(3) treating, preventing, or ameliorating a disease associated with abnormal function or expression of ROR1 (e.g., cancer, such as hematologic malignancy, solid tumor) in a subject , or treating, preventing, or ameliorating one or more symptoms of the disease;
(4) reducing or inhibiting tumor growth or progression in a subject having a tumor expressing ROR1;
(5) inducing regression of ROR1-expressing tumors (e.g., long-term regression);
(6) exerting cytotoxic activity in cells expressing ROR1.

In one embodiment, the present application provides a conjugate comprising the anti-ROR1 antibody of the first aspect. In a specific embodiment, the molecules that can be conjugated to the anti-ROR1 antibody are, for example, cytotoxic agents, immunomodulators, imaging agents, fluorescent proteins, molecular markers, therapeutic proteins, biopolymers, and oligonucleotides, etc.

In one embodiment, the present invention provides an antibody-drug conjugate (ADC), comprising the anti-ROR1 antibody or antigen-binding fragment thereof according to the first aspect and at least one therapeutically active substance or pharmaceutically active ingredient, having the structure of Ab- (L- D)n, wherein: Ab is the antibody binding to ROR1 or antigen-binding fragment thereof as described in the first aspect of the present invention; L is a linker; D is a therapeutically active substance or a pharmaceutically active ingredient, and n represents an integer from 1 to 20, such as 1, 2, 3, 4, 5, etc..

In one embodiment, the antibody-drug conjugate comprises a plurality of D components, which may be a combination of different therapeutically active substances or pharmaceutically active ingredients, or a combination of the same therapeutically active substances or pharmaceutically active ingredients. In one embodiment, the antibody-drug conjugate has a drug/antibody ratio (DAR) from 1 to 20, such as a DAR value of 2, 3, 4, 5, 6, 7, 8, 9, 10, 11, 12, 13, 14 or 15. In one embodiment, the DAR is an average DAR. In one embodiment, the average DAR ranges from 1 to 15, *e.g.,* from 1 to 10, from 2 to 8, from 2 to 6 or from 3 to 5. In a specific embodiment, the average DAR is 3.7.

In a specific embodiment, the therapeutically active substance or pharmaceutically active ingredient is a cytotoxin, a plant toxin, a small molecule toxin, a radioactive isotope, maytansine alkaloids, or the like. In a specific embodiment, the cytotoxin is dolastatin and auristatin derivatives thereof, such as 0101(2- methylpropionyl -N-[(3R,4S,5S)-3-methoxy-1-{(2S)-2-[(1R,2R)-1-methoxy-2-methyl-3-oxy-3-{[(1S)-2-phenyl-1-(1,3 -thiazol-2-yl)-ethyl] amino}propyl]pyrrolidine-1-yl}-5-methyl-1-oxyheptane-4-yl]-N-methyl-L-valin amide), 8261 (2- methylpropionyl -N-[(3R,4S,SS)-1- f (2S)-2-[(1R,2R)-3- f [(1S)-1-carboxyl-2-phenethyl]amino}-1-methoxy-2-methyl-3 -oxypropyl]pyrrolidine-1-yl}-3-methoxy-5-methyl-1-oxyheptane-4-yl]-N-methyl-L-valinamide), Dolastatin 10, Dolastatin 15, auristatin E, auristatin PE, monomethyl auristatin D (MMAD), monomethyl auristatin E (MMAE), monomethyl auristatin F (MMAF), auristatin F phenylenediamine (AFP), auristatin EB (AEB), auristatin EFP (AEFP), auristatin F hydroxypropylamide (AFHPA). In another embodiment, the dolastatin and auristatin derivatives thereof are auristatin, dolastatin, MMAE, MMAF, auristatin F hydroxypropylamide or auristatin F phenylenediamine.

In one embodiment, the cytotoxin is covalently linked to the anti-ROR1 antibody or antigen-binding fragment thereof in a non-site-specific or in site-specific manner via a linker.

In one embodiment, the linker is selected from the group consisting of maleimido-hexanoyl-valine-citrulline-p-aminobenzyloxy (mc-vc-PAB), acetyl-lysine-valine-citrulline-p-aminobenzyloxycarbonyl (AcLys-VC-PABC), amino PEG6-propionyl, maleimidocaproyl (mc), maleimidopropionyl (MP), valine-citrulline (val-cit), alanine-phenylalanine (ala-phe), p-aminobenzyloxycarbonyl (PAB), N-succinimidyl 4-(2-pyridylthio) pentanoate (SPP), N-succinimidyl 4-(N-maleimidomethyl)-cyclohexane-1-carboxylate (SMCC), N-succinimidyl (4-iodo-acetyl) aminobenzoate (SIAB), N-succinimidyl-4-(2-pyridyldithio) butyrate (SPDB), N-succinimidyl 3-(pyridin-2-yldithio)-propionate (SPDP).

In one embodiment, the antibody-drug conjugate comprises the anti-ROR1 antibody or antigen-binding fragment thereof described in the first aspect and tubulin inhibitor ( MMAE). In a further embodiment, MMAE is conjugated to the thiol group of cysteine on the anti-ROR1 antibody via a MC-VC-PAB linker, having the structure of anti-ROR1 antibody-MC-VC-PAB-MMAE. IgG1 antibodies have 16 pairs of cysteine residues, which are present in the form of 12 intrachain and 4 interchain disulfide bonds. The interchain disulfide bonds are accessible solvent and can be reduced by reducing agents to form eight sulfhydryl groups, which then become conjugation targets (McCombs J, Owen S. Antibody drug conjugates: design and selection of linker, payload and conjugation chemistry. AAPS J. 2015;17:339-51).

In a specific embodiment, the antibody-drug conjugate comprises or consists of anti-ROR1 monoclonal antibody B62-H3L3 and MC-VC-PAB-MMAE. In a further embodiment, MMAE is conjugated to the thiol group of cysteine on B62-H3L3 via a MC-VC-PAB linker.

In another specific embodiment, the antibody-drug conjugate comprises or consists of anti-ROR1 monoclonal antibody B31-H3L3 and MC-VC-PAB-MMAE. In a further embodiment, MMAE is conjugated to the thiol group of cysteine on B31-H3L3 via a MC-VC-PAB linker.

In a third aspect, the present invention provides a pharmaceutical composition comprising (1) the antibody or antigen-binding fragment thereof of the first aspect or the antibody-drug conjugate of the second aspect, and (2) a pharmaceutically acceptable carrier.

In a fourth aspect, the present invention provides an isolated polynucleotide molecule encoding any one of the antibodies or antigen-binding fragments thereof described in the first aspect.

In a fifth aspect, the present invention provides a vector comprising the nucleic acid molecule of the fourth aspect. In one embodiment, the vector is an expression vector.

In a sixth aspect, the present invention provides a host cell comprising the vector of the fifth aspect or the nucleic acid molecule of the fourth invention. In some embodiments, the host cell is prokaryotic, such as *E. coli.* In other embodiments, the host cell is eukaryotic, such as HEK293 cell, CHO cell, yeast cell, or plant cell.

In a seventh aspect, the present invention provides a method for preventing or treating a disease associated with abnormal expression of ROR1 in a subject in need thereof, comprising administering to the subject a preventively or therapeutically effective amount of the antibody or antigen-binding fragment thereof of the present invention, or a preventively or therapeutically effective amount of the antibody-drug conjugate of the present invention, or a preventively or therapeutically effective amount of the pharmaceutical composition of the present invention.

In one embodiment, the disease associated with abnormal expression of ROR1 is a cancer that highly expresses ROR1, such as chronic lymphocytic leukemia (CLL), acute lymphocytic leukemia (ALL), mantle cell lymphoma, renal cell carcinoma, colon cancer, gastric cancer, breast cancer, neuroblastoma, lung cancer, head and neck cancer, and melanoma. In a specific embodiment, the lung cancer is non-small cell lung cancer. In a specific embodiment, the breast cancer is triple-negative breast cancer.

In some embodiments, the ADC molecules or pharmaceutical compositions of the present invention can also be administered in combination with one or more other therapies, such as treatment modalities and/or other therapeutic agents, for the uses described herein, such as for preventing and/or treating the relevant diseases or conditions mentioned herein.

In a specific embodiment, the present invention provides a method for killing cells expressing ROR1 or inhibiting the growth of cells expressing ROR1, comprising contacting the cells with an effective amount of the antibody or antigen-binding fragment thereof of the present invention, or an effective amount of the antibody-drug conjugate of the present invention, or an effective amount of the pharmaceutical composition of the present invention.

In an eighth aspect, the present invention provides use of an anti-ROR1 antibody or antigen-binding fragment thereof in the preparation of an antibody-drug conjugate for preventing or treating cancer.

The present invention also provides use of an antibody-drug conjugate comprising an anti-ROR1 antibody or antigen-binding fragment thereof in the preparation of a drug for preventing or treating cancer.

## DESCRIPTION OF THE DRAWINGS

Figure 1 shows the binding activity of each murine antibody clone Fab obtained in the present application binding to huROR1-HEK293 cells determined by FACS; Figure 1A shows the binding activity of clone B31 Fab, B32 Fab, B34 Fab, B39 Fab, B62 Fab, B74 Fab, C38 Fab, M71 Fab and M78 Fab lysate binding to huROR1-HEK293 cells ; Figure 1B shows the binding activity of C42 Fab and C77 Fab lysate binding to huROR1-HEK293 cells; NC represents negative control, and MFI represents median fluorescence intensity.

Figures 2A-2L show the SEC-HPLC results of the antibodies of the present application; Figure 2A shows the SEC-HPLC results of B31; Figure 2B shows the SEC-HPLC results of B32; Figure 2C shows the SEC-HPLC results of B34; Figure 2D shows the SEC-HPLC results of B39; Figure 2E shows the SEC-HPLC results of B62; Figure 2F shows the SEC-HPLC results of B74; Figure 2G shows the SEC-HPLC results of C38; Figure 2H shows the SEC-HPLC results of M71; Figure 2I shows the SEC-HPLC results of M78; Figure 2J shows the SEC-HPLC results of C42; Figure 2K shows the SEC-HPLC results of C77; Figure 2L shows the SEC-HPLC results of 99961.1.

Figures 3A-3B show the activity of the antibodies of the present application binding to the antigen protein huROR1-His determined by ELISA; Figure 3A shows the activity of B31, B32, B34, B39, B62 and B74 binding to the antigen protein huROR1-His; Figure 3B shows the binding activity of C38, C42, C77, M71 and M78 with the antigen protein huROR1-His.

Figures 4A-4B show the activity of the antibodies of the present application binding to A549 tumor cells determined by FACS; Figure 4A shows the binding activity of B31, B32, B34, B39, B62 and B74 to A549 tumor cells; Figure 4B shows the activity of C38, C42, C77, M71 and M78 binding to A549 tumor cells.

Figures 5A-5B show the activity of the antibodies of the present application binding to huROR1-HEK293 cells determined by FACS; Figure 5A shows the activity of B31, B32, B34, B39, B62 and B74 binding to huROR1-HEK293 cells; Figure 5B shows the activity of C38, C42, C77, M71 and M78 binding to huROR1-HEK293 cells.

Figures 6A-6B show the activity of the antibodies of the present application binding to the antigen protein MusROR1-His determined by ELISA; Figure 6A shows the activity of B31, B32, B34, B39 and B62 binding to the antigen protein MusROR1-His; Figure 6B shows the activity of B74, C38, C42, C77, M71 and M78 binding to the antigen protein MusROR1-His.

Figures 7A-7B show the activity of the antibodies of the present application binding to the antigen protein huROR2-His determined by ELISA; Figure 7A shows the activity of B31, B32, B34, B39 and B62 binding to the antigen protein huROR2-His; Figure 7B shows the activity of B74, C38, C42, C77, M71 and M78 binding to the antigen protein huROR2-His.

Figures 8A-8B show the internalization rate of the antibodies of the present application in huROR1-HEK293 cells determined by FACS; Figure 8A shows the internalization rate of B62 and C42 in huROR1-HEK293 cells; Figure 8B

shows the internalization rate of B31, B32, B34, B39, B74, C38, C77, M71 and M78 in huROR1-HEK293 cells.

Figures 9A-9F show the internalization rate of the antibodies of the present application in huROR1-HEK293 cells determined by Fab-Zap method; Figure 9A shows the internalization rate of B31 and B32 in huROR1-HEK293 cells; Figure 9B shows the internalization rate of B34 and B39 in huROR1-HEK293 cells; Figure 9C shows the internalization rate of B74 and C38 in huROR1-HEK293 cells; Figure 9D shows the internalization rate of C77 and M71 in huROR1-HEK293 cells; Figure 9E shows the internalization rate of M78 in huROR1-HEK293 cells; Figure 9F shows the internalization rate of B62 and C42 in huROR1-HEK293 cells.

Figures 10A-10E show the activity of humanized antibodies binding to the antigen protein huROR1-His determined by ELISA;Figure 10A shows the activity of B31-H2L2, B31-H1L1 and B31-H2L3 binding to the antigen protein huROR1-His; Figure 10B shows the activity of B31-H3L2, B31-H3L3 and B31-H3L4 binding to the antigen protein huROR1-His; Figure 10C shows the activity of B31-H4L2, B31-H4L3 and B31-H4L4 binding to the antigen protein huROR1-His; Figure 10D shows the activity of B62-H1L1, B62-H1L2, B62-H2L2 and B62-H2L3 binding to the antigen protein huROR1-His; Figure 10E shows the activity of B62-H2L4, B62-H3L2, B62-H3L3 and B62-H3L4 binding to the antigen protein huROR1-His.

Figures 11A-11B show the activity of humanized antibodies binding to A549 tumor cells determined by FACS; Figure 11A shows the activity of B31-H1L1, B31-H2L2, B31-H2L3, B31-H3L2, B31-H3L3, B31-H3L4, B31-H4L2, B31-H4L3 and B31-H4L4 binding to A549 tumor cells; Figure 11B shows the activity of B62-H1L1, B62-H1L2, B62-H2L2, B62-H2L3, B62-H2L4, B62-H3L2, B62-H3L3 and B62-H3L4 binding to A549 tumor cells.

Figure 12 shows the internalization rate of B31-H3L3 and B62-H3L3 in huROR1-HEK293 cells determined by Fab-Zap method.

Figures 13A-13B show the activity of the ADCs of the present application binding to A549 and HT29 tumor cells determined by FACS; Figure 13A shows the activity of B31-H3L3-MMAE and B62-H3L3-MMAE binding to A549 tumor cells; Figure 13B shows the activity of B31-H3L3-MMAE and B62-H3L3-MMAE binding to HT29 tumor cells.

Figures 14A-14D show the tumor cell killing rate of the ADCs of the present application detected by MTS method; Figure 14A shows the effect of B31-H3L3-MMAE on killing A549 tumor cells; Figure 14B shows the effect of B62-H3L3-MMAE on killing A549 tumor cells; Figure 14C shows the effect of B31-H3L3-MMAE on killing HT29 tumor cells; Figure 14D shows the effect of B62-H3L3-MMAE on killing HT29 tumor cells.

Figures 15A-15C show the effect of the ADCs of the present application on killing three tumor cells, Jeko-1, MDA-MB-468 and NCI-H1944, determined by CCK8 method; Figure 15A shows the effect of B31-H3L3-MMAE and B62-H3L3-MMAE on killing Jeko-1 cells; Figure 15B shows the effect of B31-H3L3-MMAE and B62-H3L3-MMAE on killing Jeko-1 cells; Figure 15C shows the effect of B31-H3L3-MMAE and B62-H3L3-MMAE on killing NCI-H1944 cells.

Figure 16 shows the antigen-dependent killing effect of the ADCs of the present application on huROR1-HEK293 cells measured by CCK8 method.

Figures 17A-17B show the internalization rate of the ADCs of the present application in A549 and HT-29 tumor cells determined by FACS; Figure 17A shows the internalization rate of B31-H3L3-MMAE and B62-H3L3-MMAE in A549 tumor cells; Figure 17B shows the internalization rate of B31-H3L3-MMAE and B62-H3L3-MMAE in HT-29 tumor cells.

Figures 18A-18C show the *in vivo* tumor-suppressing effect of the ADCs of the present application in HT-29 tumor mouse models; Figure 18A shows the changes in tumor volume in mice in different experimental groups during the experimental period, with arrows indicating the time points of drug administration; Figure 18B shows the changes in body weight of mice in different experimental groups during the experimental period; Figure 18C shows the tumor weight in mice in different experimental groups after the end of the experiment.

Figures 19A-19C show the *in vivo* tumor-suppressing effect of the ADCs of the present application in A549 tumor mouse models; Figure 19A shows the changes in tumor volume in mice in different experimental groups during the experimental period; Figure 19B shows the changes in body weight of mice in different experimental groups during the experimental period; Figure 19C shows the tumor weight in mice in different experimental groups after the end of the experiment.

Figures 20A-20B show the *in vivo* tumor-suppressing effect of the ADCs of the present application in NCI-N87 tumor mouse models; Figure 20A shows the changes in tumor volume in mice in different experimental groups during the experimental period, with arrows indicating the time points of drug administration; Figure 20B shows the changes in body weight of mice in different experimental groups during the experimental period.

Figures 21A-21B show the *in vivo* tumor-suppressing effect of the ADCs of the present application in MDA-MB-231 tumor mouse models; Figure 21A shows the changes in tumor volume in mice in different experimental groups during the experimental period, with arrows indicating the time points of drug administration; Figure 21B shows the changes in body weight of mice in different experimental groups during the experimental period.

Figures 22A-22B show the *in vivo* tumor-suppressing effect of the ADCs of the present application in MDA-MB-468 tumor mouse models; Figure 22A shows the changes in tumor volume in mice in different experimental groups during the experimental period, with arrows indicating the time points of drug administration; Figure 22B shows the changes

in body weight of mice in different experimental groups during the experimental period.

Figures 23A-23B show the *in vivo* tumor-suppressing effect of the ADCs of the present application in Jeko-1 tumor mouse models; Figure 23A shows the changes in tumor volume in mice in different experimental groups during the experimental period, with arrows indicating the time points of drug administration; Figure 23B shows the changes in body weight of mice in different experimental groups during the experimental period.

## DETAILED DESCRIPTION OF THE INVENTION

Before describing the present invention in detail, it is to be understood that this invention is not limited to the particular methods and experimental conditions described herein as such methods and conditions may vary. Additionally, the terminology used herein is for the purpose of describing particular embodiments only and is not intended to be limiting.

### I. Definitions

Unless otherwise defined, all technical and scientific terms used herein have the same meanings as commonly understood by one of ordinary skill in the art. For purposes of the present invention, the following terms are defined below.

The term "about" when used in connection with a numerical value is meant to encompass numerical values within the range between the lower limit of 10% less than the specified numerical value and the upper limit of 10% greater than the specified numerical value.

The term "and/or", when used in conjunction with two or more alternatives, should be understood to mean any one of the alternatives or any two or more of the alternatives.

As used herein, the term "comprising", "comprise", " including " or "include" means to include the stated elements, integers or steps, but does not exclude any other elements, integers or steps. When the term "comprising" or "including" is used herein, unless otherwise specified, it also encompasses the situation consisting of the stated elements, integers or steps. For example, when referring to an antibody variable region "comprising" a specific sequence, it is also intended to encompass an antibody variable region consisting of the specific sequence.

The term "ROR1" refers to any recombinant or naturally occurring form of Receptor Tyrosine Kinase-like Orphan Receptor 1 (ROR1), variant or homolog thereof, which maintains, for example, at least 50%, 80%, 90%, 95%, 96%, 97%, 98%, 99% or 100% of the activity of ROR1. The variant or homolog has at least 90%, 95%, 96%, 97%, 98%, 99% or 100% amino acid sequence identity to the naturally occurring ROR1 protein in the entire sequence or a partial sequence (e.g., 50, 100, 150 or 200 consecutive amino acids). In one embodiment, the ROR1 protein includes the amino acid sequence of Uniprot ID: Q01973.

ROR1 is highly expressed in the embryo, and then its expression level decreases significantly in the adult stage. However, it was found that the expression of ROR1 was significantly increased in various hematologic malignancies and solid tumors. Hematologic malignancies that highly express ROR1 include B-cell chronic lymphocytic leukemia (CLL), acute lymphocytic leukemia (ALL), non-Hodgkin lymphoma (NHL) and myeloid hematologic malignancy. Among solid tumors, cancers that express ROR1 include colon cancer, breast cancer, intestinal cancer, lung cancer, pancreatic cancer, ovarian cancer, etc.

The term "antibody" is used in the broadest sense herein and encompasses a variety of antibody structures, including but not limited to monoclonal antibodies, polyclonal antibodies, multispecific antibodies (e.g., bispecific antibodies), and antibody fragments, as long as they show desired antigen-binding activity. An intact antibody generally contains at least two full-length heavy chains and two full-length light chains, but may include fewer chains in some cases, for example, antibodies naturally occurring in camels may contain only heavy chains.

The term "anti-ROR1 antibody" refers to an antibody molecule that specifically binds to ROR1 and is capable of inhibiting ROR1 activity. An anti-ROR1 antibody can inhibit ROR1 activity relative to the absence of the ROR1 antibody, *e.g.*, by at least partially or completely blocking the stimulation of ROR1, reducing, preventing or delaying the activation of ROR1, or inactivating, desensitizing or down-regulating the signaling, activity or amount of ROR1. In some embodiments, the antibody can inhibit ROR1 activity by 10%, 20%, 30%, 40%, 50%, 60%, 70%, 80%, 90% or more compared to a control.

The term "antibody fragment" refers to a molecule other than an intact antibody, such molecule comprises a portion of the intact antibody and binds the antigen to which the intact antibody binds. Examples of antibody fragments include, but are not limited to, Fv, Fab, Fab', Fab'-SH, $F(ab')_2$; diabodies; linear antibodies; single-chain antibodies (e.g., scFv); single-domain antibodies; bivalent or bispecific antibodies or fragments thereof; camelid antibodies (heavy chain antibodies); and multispecific antibodies (e.g., bispecific antibodies) composed of antibody fragments.

The term "variable region" or "variable domain" refers to the domain of an antibody heavy or light chain that is involved in antibody binding to an antigen. The heavy and light chain variable domains of naturally occurring antibodies generally have similar structures, wherein each domain comprises four conserved framework regions (FRs) and three complementarity determining regions (see, *e.g.,* Kindt et al. Kuby Immunology, 6th ed., WH Freeman and Co. page 91 (2007)). A single VH or VL domain is sufficient to confer antigen binding specificity.

The term "Complementarity-determining regions" or "CDR regions" or "CDRs" are the regions in antibody variable domains, which are highly variable in sequence and form structurally defined loop ("hypervariable loop"), and/or include antigen-contacting residues ("antigen-contacting points"). The CDRs are mainly responsible for binding to antigenic epitopes. The CDRs in the variable domain are commonly designated as CDR1, CDR2, and CDR3, sequentially numbered from the N-terminus. The precise amino acid sequence boundaries of each CDR in a given variable region can be determined by using any one or a combination of various established CDR assignment scheme, including, for example, Chothia based on the three-dimensional structure of the antibody and the topology of the CDR loops (Chothia et al. (1989) Nature 342: 877-883, Al-Lazikani et al., "Standard conformations for the canonical structures of immunoglobulins", Journal of Molecular Biology, 273, 927-948(1997)), Kabat based on the variability of antibody sequences (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (http://imgt.cines.fr/), and North CDR definition based on affinity propagation clustering by using a large number of crystal structures.

Unless otherwise indicated, in the present invention, the term "CDRs" or "CDR sequences" encompass CDR sequences determined in any of the ways described above.

The CDRs can also be determined based on a reference CDR sequence (for example, any of the exemplary CDRs of the present invention) having the same AbM numbering position. In one embodiment, the CDRs of the antibody of the invention are positioned according to the AbM numbering scheme.

Unless otherwise indicated, in the present invention, when referring to residue positions in antibody variable regions and CDRs (including heavy chain variable region residues), we refer to those according to the AbM numbering system.

"Humanized" antibody refers to a chimeric antibody comprising amino acid residues from non-human CDRs and amino acid residues from human FRs. In some embodiments, all or substantially all CDRs *(e.g.,* CDRs) in a humanized antibody correspond to those of a non-human antibody, and all or substantially all FRs correspond to those of a human antibody. A humanized antibody may optionally contain at least a portion of antibody constant region derived from human antibody. The "humanized form" of an antibody (e.g., a non-human antibody) refers to an antibody that has been humanized.

As used herein, the term "binding" or "specific binding" mean that the binding is selective for an antigen and can be distinguished from unwanted or nonspecific interactions. The ability of an antigen binding site to bind a specific antigen can be determined by enzyme-linked immunosorbent assay (ELISA) or conventional binding assays known in the art such as by radioimmunoassay (RIA) or biofilm thin layer interferometry or MSD assay or surface plasmon resonance (SPR).

The term "half effective concentration ($EC_{50}$)" refers to the concentration of a drug, antibody or toxic agent that induces a response that is 50% between baseline and maximum after a specified exposure time.

The term "therapeutic agent" as used herein encompasses any substance effective in preventing or treating tumors, such as cancer, including chemotherapeutic agents, cytokines, angiogenesis inhibitors, cytotoxic agents, other antibodies, small molecule drugs, or immunomodulators (e.g., immunosuppressants).

The term "antibody-drug conjugate" or "ADC" refers to an antibody or antibody fragment covalently coupled to a therapeutically active substance or active pharmaceutical ingredient, such that the therapeutically active substance or active pharmaceutical ingredient is targeted to the binding target of the antibody to exhibit its pharmacological function. The therapeutically active substance or active pharmaceutical ingredient may be a cytotoxin capable of killing cells, preferably cancer cells, targeted by the ADC. The covalent attachment of therapeutically active substances, active pharmaceutical ingredients or cytotoxins can be performed in a non-site-specific manner using linkers, or in a site-specific manner.

The term "site-specific conjugation" refers to a method of specifically linking a therapeutically active substance or active pharmaceutical ingredient to a specific site of an antibody. In one embodiment, the conjugation is accomplished via a linker.

The term "cytotoxic agent" and "cytotoxin" are used interchangeably and refer to a substance that inhibits or disrupts cellular function and/or causes cell death or destruction in the present invention. In one embodiment, the cytotoxic agent may include, but is not limited to, bacterial toxins *(e.g.,* diphtheria toxin), plant toxins *(e.g.,* ricin), small molecule toxins, radioactive isotopes, maytansine alkaloids, and the like, specifically, such as anthracycline, camptothecin, combretastatin, dolastatin and their auristatin derivatives, duocarmycin, enediyne, geldanamycin, indolino-benzodiazepine dimer, maytansine, puromycin, pyrrolobenzodiazepine dimer, taxane, vinca alkaloid, tubulysin, hemiasterlin, spliceostatin, pladienolide and calicheamicin.

Any antibody-drug conjugate of the present invention can be prepared by conjugating the antibody to dolastatin and its auristatin derivatives. Dolastatin and its auristatin derivatives are important cytotoxins used in antibody drug conjugates (ADCs). They interfere with microtubule dynamics, cell division, etc., and have anti-tumor and anti-fungal activities. The modification of its skeleton has been widely reported in the literatures, mainly the modification of the terminal subunits: modification of P1 (N-terminus) and P5 (C-terminus). The modification of the central peptide subunit also results

in effective in vitro cytotoxicity of this type of substance. In an aspect, dolastatin and auristatin derivatives thereof can be, *e.g.,* 0101(2-methylpropionyl-N-[(3R,4S,SS)-3-methoxy-1-{(2S)-2-[(1R,2R)-1-methoxy-2-methyl-3-oxy-3-{[(1S)-2-phenyl-1-(1,3-thiazol-2-yl)-ethyl]amino}propyl]pyrrolidine-1-yl}-5-methyl-1-oxyheptane -4-yl]-N-methyl-L-valinamide), 8261 (2-methylpropionyl-N-[(3R,4S,SS)-1-{(2S)-2-[(1R,2R)-3-{[(1S)-1-carboxyl-2-phenethyl]amino}-1-methoxy-2-methyl-3-oxypropyl]pyrrolidine-1-yl}-3-methoxy-5-methyl-1-oxyheptane-4-yl]-N-methy l-L-valinamide), Dolastatin 10, Dolastatin 15, auristatin E, auristatin PE, monomethyl auristatin D (MMAD), monomethyl auristatin E (MMAE), monomethyl auristatin F (MMAF), auristatin F phenylenediamine (AFP), auristatin EB (AEB), auristatin EFP ( AEFP), auristatin F hydroxypropylamide (AF AFHPA), and other auristatins, e.g., auristatins described in US publication No. 20130129753.

Monomethyl auristatin (MMAE), also known as demethyl-auristatin E, is a well-known member of the auristatin compound family. It has the following structural formula:

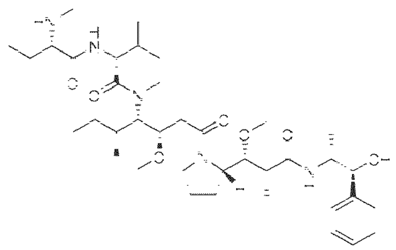

MMAE plays an effective inhibitory role in mitosis by inhibiting tubulin polymerization. It cannot be used as a drug due to its cytotoxicity, but it has been widely used to prepare antibody conjugates. MMAE was conjugated to monoclonal antibody (MAB) via a linker to form MMAE-MAB. Generally, MMAE-MAB targets tumor cells through antibodies, and the linker is cleaved after MMAE-MAB enters the tumor cells, thereby releasing MMAE, allowing it to exert its cytotoxic effect and kill tumor cells.

The terms "linker" and "connector" are used interchangeably in this application to refer to a chemical moiety that covalently links an antibody to a therapeutically active substance or active pharmaceutical ingredient in an ADC. In one embodiment, the linker may comprise amino acid residues that connect the antibody to the payload. The amino acid residues may form dipeptide, tripeptide, tetrapeptide, pentapeptide, hexapeptide, heptapeptide, octapeptide, nonapeptide, decapeptide, undecapeptide or dodecapeptide units. Amino acid residues include those occurring naturally as well as non-naturally occurring amino acid analogs, such as citrulline or β-amino acids, such as β-alanine, or ω-amino acids such as 4-amino-butyric acid.

According to the property, the linkers suitable for the present invention can be classified as linkers degradable by cathepsin, such as valine-citrulline (val-cit) linkers, cBu-Cit linkers and CX linkers; non-cleavable linkers such as SMCC linkers or MD linkers; acid-sensitive linkers, silicone-structured linkers, disulfide-carbamate linkers, MC-GGFG linkers, TRX linkers, galactoside-containing linkers, pyrophosphate linkers, near-infrared-sensitive linkers, ultraviolet-sensitive linkers such as PC4AP.

The linker of the present invention may also be a combination of one or more linkers. For example, a linker degradable by cathepsin may be combined with other types of linkers to form a new linker. Therefore, the "linker" described in the present invention encompasses a single type of linker, or a combination of different types of linkers, as long as it is capable of coupling the antibody of the present invention to a drug.

In a specific embodiment, the linker includes but is not limited to maleimido-hexanoyl-valine-citrulline-p-aminobenzyloxy (mc-vc-PAB), acetyl-lysine-valine-citrulline-p-aminobenzyloxycarbonyl (AcLys-VC-PABC), amino PEG6-propionyl, maleimidocaproyl (mc), maleimidopropionyl (MP), valine-citrulline (val-cit), alanine-phenylalanine (ala-phe), p-aminobenzyloxycarbonyl (PAB), N-succinimidyl 4-(2-pyridylthio) pentanoate (SPP), N-succinimidyl 4-(N-maleimidomethyl)-cyclohexane-1-carboxylate (SMCC), N-succinimidyl (4-iodo-acetyl) aminobenzoate (SIAB), N-succinimidyl-4-(2-pyridyldithio) butyrate (SPDB), N-succinimidyl 3-(pyridin-2-yldithio)-propionate (SPDP).

The term "load" or "drug loading" or "payload" refers to the average number of effective payloads per antibody within an ADC molecule ("payload" is used interchangeably with "therapeutically active substance or active pharmaceutical ingredient" herein). Drug loading can range from 1 to 20 therapeutically active substances or active pharmaceutical ingredients per antibody. The term "drug/antibody ratio" or "DAR" refers to the ratio of therapeutically active substance or active pharmaceutical ingredient (D) conjugated to an antibody to the antibody. The ADCs described herein typically have a DAR from 1 to 20, and in certain embodiments have a DAR from 1 to 8, from 2 to 8, from 2 to 6, from 2 to 5, from 2 to 18, from 4 to 16, from 5 to 12, from 6 to 10, from 3 to 8, from 4 to 6, from 6 to 10, and from 2 to 4. Representative DAR value is 2, 3, 4, 5, 6, 7, 8, 9, 10, 11, 12, 13, 14 or 15, usually expressed as a combination of the letter D and a number, where the number represents the numerical value of the DAR, for example, D2 represents the drug/antibody ratio, i.e., DAR value is 2. In some embodiments, the DAR is an average DAR, *i.e.,* characterized by a detection method (e.g., by conventional methods such as UV/visible spectroscopy, mass spectrometry, ELISA assay, and HPLC). Quantitative DAR values can

also be determined. DAR may be limited by the number of binding sites on the antibody. For example, where the binding site is cysteine thiol, the antibody may have only one or a few cysteine thiol groups or may have only one or a few sufficiently reactive thiol groups through which the linker unit may be attached. In some embodiments, the average DAR value of the conjugate of the invention is from 1 to 20, such as from 2 to 18, from 4 to 16, from 5 to 12, from 6 to 10, from 2 to 8, from 3 to 8, from 2 to 6, from 4 to 6, from 6 to 10, such as from 1.0 to 8.0, from 2.0 to 6.0, such as 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4 , 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9 or 10.0, and ranges with two of these values as endpoints.

The term "treatment" refers to slowing, interrupting, blocking, alleviating, stopping, reducing, or reversing the progression or severity of an existing symptom, disorder, condition, or disease. Desired therapeutic effects include, but are not limited to, preventing disease appearance or recurrence, alleviating symptoms, reducing any direct or indirect pathological consequences of the disease, preventing metastasis, reducing the rate of disease progression, ameliorating or palliating the disease state, and alleviating or improving prognosis. In some embodiments, the antibodies of the invention are used to delay the development of a disease or to slow the progression of a disease.

The term "prevention" includes the inhibition of the occurrence or progression of a disease or disorder or symptoms of a particular disease or disorder. In some embodiments, subjects with a family history of cancer are candidates for preventive regimens. Generally, in the context of cancer, the term "prevention" refers to the administration of a drug prior to the onset of signs or symptoms of cancer, particularly in subjects at risk of cancer.

The term "effective amount" refers to an amount or dose of an antibody or conjugate or composition of the invention to produce the desired effect in a patient in need of treatment or prevention after being administered to a patient in single or multiple doses. An effective amount can be readily determined by the attending physician skilled in the art, by taking into account various factors such as the species; body weight; age and general health condition of the mammals; the specific disease involved; the degree or severity of the disease; the response of the individual patient; the specific antibody to be administered; the mode of administration; the bioavailability profile of the preparation to be administered; the selected dosing regimen; and the use of any concomitant therapy.

The term "therapeutically effective amount" refers to an amount effective to achieve the desired therapeutic result, at necessary dosages and for periods of time necessary. A therapeutically effective amount of the antibody or antibody fragment or conjugate or composition thereof may vary according to factors such as the disease state, age, sex, and body weight of the individual, and the ability of the antibody or antibody portion to elicit a desired response in the individual. A therapeutically effective amount is also one in which any toxic or detrimental effects of the antibody or antibody fragment or conjugate or composition thereof are outweighed by the therapeutically beneficial effects. The "therapeutically effective amount" preferably inhibits a measurable parameter *(e.g.,* tumor growth rate, tumor volume, *etc.*) by at least about 20%, more preferably at least about 40%, even more preferably at least about 50%, 60% or 70%, and still more preferably at least about 80% or 90% relative to untreated subjects. The ability of a compound to inhibit a measurable parameter (e.g., cancer) can be evaluated in an animal model system in which the efficacy in human tumors is predictive.

The term "preventively effective amount" refers to an amount effective to achieve the desired preventive result, at necessary dosages and for periods of time necessary. Typically, since a preventive dose is used in subjects prior to or at an earlier stage of disease, the preventively effective amount will be less than the therapeutically effective amount.

The term "pharmaceutical composition" refers to a composition that allows active ingredients contained therein are in a form of maintaining their biological activity and does not contain additional ingredients unacceptably toxic to a subject who would be administered with the composition.

## II. Compositions of the Invention

In some embodiments, the present invention provides a composition comprising any anti-ROR1 antibody described herein, or an ADC molecule thereof, preferably a pharmaceutical composition. In one embodiment, the composition further comprises a pharmaceutically acceptable adjuvant, such as a pharmaceutically acceptable carrier, a pharmaceutically acceptable excipient, including a buffer, known in the art. In one embodiment, the composition (e.g., the pharmaceutical composition) comprises an anti-ROR1 antibody of the invention, or an ADC molecule thereof, in combination with one or more other therapeutic agents.

As used herein, "pharmaceutically acceptable carrier" includes any and all physiologically compatible solvents, dispersion media, isotonic agents and absorption delaying agents, and the like.

For the use of pharmaceutically acceptable adjuvant, see also "Handbook of Pharmaceutical Excipients", Eighth Edition, R.C. Rowe, P.J. Seskey and S.C. Owen, Pharmaceutical Press, London, Chicago.

The compositions of the present invention may be present in a variety of forms. These forms include, for example, liquid, semisolid, and solid dosage forms, such as liquid solutions (e.g., injectable solutions and infusible solutions), powders or suspensions, liposomes, and suppositories. The preferred form depends on the intended mode of administration and therapeutic use.

The administration route of the composition of the invention is based on the known methods, for example, orally, by intravenous injection, intraperitoneally, intracerebrally (intraparenchymal), intracerebroventricularly, intramuscularly, intraocularly, intraarterially, intraportally or intralesionally; by sustained release systems or by implanted devices. In certain embodiments, the compositions may be administered by bolus injection or by continuous infusion or by an implant device.

The subject can be a mammal, *e.g.,* a primate, preferably, a higher order primate, *e.g.,* a human (e.g., an individual having or at risk of having a disease described herein). In one embodiment, the subject has or is at risk of having a disease described herein (e.g., cancer). In certain embodiments, the subject is receiving or has received other treatments, such as chemotherapy treatment and/or radiation therapy. In some embodiments, the subject has previously received or is currently receiving immunotherapy.

A medicament comprising the antibody described herein can be prepared by mixing the anti-ROR1 antibody of the present invention, or ADC molecule thereof, having a desired purity with one or more optional pharmaceutically acceptable adjuvants, preferably in the form of a lyophilized preparation or an aqueous solution.

The pharmaceutical composition or formulation of the present invention may also contain more than one active ingredient as required for the particular indication to be treated, preferably those with complementary activities that do not adversely affect each other. For example, it is also desirable to provide other therapeutic agents, including chemotherapeutic agents, angiogenesis inhibitors, cytokines, cytotoxic agents, other antibodies, small molecule drugs, or immunomodulators (e.g., immune checkpoint inhibitors or agonists), and the like. The active ingredients are suitably present in combination in amounts effective for the intended purpose.

Sustained release preparations can be prepared. Suitable examples of sustained release preparations include semipermeable matrices of solid hydrophobic polymers containing the antibody, and the matrices are in the form of shaped articles, e.g. films, or microcapsules.

### III. Preparation of Antibodies and Antibody-Drug Conjugates of the Invention

In one embodiment, the present invention provides a method for preparing an anti-ROR1 antibody, wherein the method comprises culturing a host cell comprising a nucleic acid encoding an anti-ROR1 antibody or an expression vector comprising the nucleic acid under conditions suitable for expression of the nucleic acid encoding the anti-ROR1 antibody, and optionally isolating the anti-ROR1 antibody. In certain embodiment, the method further comprises recovering the anti-ROR1 antibody from the host cell (or host cell culture medium).

For recombinant production of the anti-ROR1 antibody of the present invention, a nucleic acid encoding the anti-ROR1 antibody of the present invention is first isolated and inserted into a vector for further cloning and/or expression in a host cell. Such nucleic acid is readily isolated and sequenced using conventional procedures, for example, by using oligonucleotide probes that are capable of binding specifically to the nucleic acid encoding the anti-ROR1 antibody of the invention.

The present anti-ROR1 antibodies prepared as described herein can be purified by techniques known in the prior art, such as high performance liquid chromatography, ion exchange chromatography, gel electrophoresis, affinity chromatography, size exclusion chromatography, and the like. The actual conditions used to purify a particular protein will also be dependent on factors such as net charge, hydrophobicity, hydrophilicity, etc., and these will be apparent to those skilled in the art. The purity of the anti-ROR1 antibody of the invention can be determined by any of a variety of well-known analytical methods, including size exclusion chromatography, gel electrophoresis, high performance liquid chromatography, and the like.

Various methods for conjugating cytotoxic or other therapeutic agents to antibodies have been described in the prior art. For example, conjugation can occur through the amino group of lysine side chains and the amino group at the N-terminus of the antibody, the carboxyl group of aspartic acid, glutamic acid and the C-terminus, or the activated cysteine sulfhydryl group in the antibody.

## EXAMPLES

The present invention is further illustrated according to the following examples, however, it should be understood that the examples are described in an illustrative rather than a limiting manner, and various modifications can be made by those skilled in the art.

The present invention will be implemented by conventional methods of chemistry, biochemistry, organic chemistry, molecular biology, microbiology, recombinant DNA technology, genetics, immunology and cell biology in the art, unless clearly indicated to the contrary.

**Example 1. Preparation and assessment of raw materials**

1.1 Preparation and assessment of ROR1 control antibody

Preparation of ROR1 control antibody: in the application, the anti-ROR1 antibody Cirmtuzumab is used as a positive control antibody. The gene for the target fragment was synthesized by General Biotech Co., Ltd. according to the sequence disclosed in WO/2019/173843. The gene was then cloned into the eukaryotic expression vector pcDNA3.4 (Invitrogen) via homologous recombination. The recombinant protein expression vector was transformed into E. coli DH5α, cultured overnight at 37°C, and plasmid DNA was extracted using an endotoxin-free plasmid extraction kit (OMEGA, D6950-01). The obtained expression vector, which expresses Cirmtuzumab, was designated with clone number 99961.1, and the expressed Cirmtuzumab will be referred to as 99961.1. The expression vector was transfected into 293 cells using ExpiFectamine™ CHO Transfection Reagent (Thermo Fisher, A29129). After 7 days, the cell culture supernatant was collected, centrifuged at 15,000 g for 10 minutes, and filtered through a 0.22 μm membrane. The antibody in the supernatant was purified using a Protein A/G affinity chromatography column. The target antibody was eluted with 100 mM glycine (pH 3.0) and then exchanged into PBS buffer using an ultrafiltration tube (Millipore, UFC901096).

Assessment of ROR1 control antibody: The activity of the prepared positive control antibody 99961.1 (IgG1) was detected with the purchased huROR1-His antigen protein (Kaixia Biotechnology, ROR-HM401). The specific method was as follows: a 96-well ELISA plate was coated with huROR1-His (2 μg/mL, 30 μL/well) at 4°C overnight; after washing three times, the plate was blocked with 5% skim milk prepared in PBS at room temperature for 1 hour; after washing three times, the plate was added the control antibody 99961.1 gradient diluted in PBS and incubated at room temperature for 1 hour; after washing, the plate was added the secondary antibody Anti-human-IgG-Kappa+Lambda-HRP (Millipore, AP502-P+AP506P) diluted with PBS (1:6000) and incubated at room temperature for 1 hour, the plate was then washed six times and added TMB for color development for 5-20 min. The data was read at OD450 with the microplate reader after the color development was terminated, and the data was processed and plotted using Graphpad prism. The results showed that the expressed control antibody 99961.1 could bind to the ROR1 protein and had normal anti-ROR1 activity.

1.2 Preparation and assessment of antigen protein

Preparation of antigen protein: Through genetic manipulation at the coding gene level, His tag or human Fc (SEQ ID NO: 51) tag was added to the C-terminus of the sequences of human ROR1 protein huROR1 ECD AA30-406 (Uniprot ID: Q01973), mouse ROR1 protein MusROR1 ECD AA30-406 (Uniprot ID: Q9Z139), and human ROR2 protein huROR2 ECD AA34-403 (Uniprot ID: Q01974). The obtained nucleic acid sequences were constructed into pcDNA3.4 vector, which was then transformed into *Escherichia coli* DH5α, cultured at 37°C overnight, and then the plasmid was extracted using an endotoxin-free plasmid extraction kit (OMEGA, D6950-01). The resulting plasmid was transiently transfected into HEK293 cells (ATCC® CRL-1573™) using the ExpiFectamine™ 293 Transfection Kit (Gibco™, A14524). After 7 days of expression, the cell culture supernatant was collected and the protein containing Fc-tagged was subjected to affinity purification using COLUMN XK16/20 (Cytiva). After purification, the target protein was eluted with 100 mM glycine (pH=3.0), concentrated, and subjected to buffer exchange, finally resulting in the antigen protein (huROR1-huFc). The His-tagged protein was subjected to affinity purification using Ni Smart Beads 6FF (Changzhou Tiandi Renhe Biotechnology Co., Ltd., SA036050), and then the target protein was eluted using imidazole gradient. The eluted proteins were respectively placed into PBS buffer through ultrafiltration concentration tubes (Millipore, UFC901096), and finally resulting in the antigen proteins (huROR1-His, MusROR1-His, huROR2-His).

Antigen assessment: The prepared antigens (huROR1-His, huROR1-huFc) were detected using the antibody 99961.1 (IgG1) obtained in Example 1.1, which passed quality inspection. The specific method was as follows: ELISA plate was coated with 2 μg/mL huROR1-His and huROR1-huFc respectively at 4°C overnight. The purchased antigen proteins huROR1-His and huROR1-huFc (Kaixia Biology, ROR-HM201) were used as positive controls. After washing three times, the plate was blocked with 5% skim milk prepared in PBS at room temperature for 1 hour; after washing three times, the plate was added antibody 99961.1 gradient diluted in PBS and incubated at room temperature for 1 hour; after washing, the plate was added the secondary antibody Anti-human-IgG-Kappa+Lambda-HRP (Millipore, AP502-P+AP506P) diluted with PBS (1:6000) and incubated at room temperature for 1 hour, the plate was then washed six times and added TMB for color development for 5-20 min. The data was read at OD450 with the microplate reader after the color development was terminated, and the data was processed and plotted using Graphpad prism. The results show that antibody 99961.1 can bind to the antigens huROR1-His and huROR1-huFc expressed in house with an affinity comparable to that of the purchased ROR1 antigen protein.

**Example 2. Construction and assessment of cell lines overexpressing human ROR1**

Construction of HEK293 cell line overexpressing human ROR1 (hereafter referred to as huROR1-HEK293): The

nucleic acid sequence encoding full-length human ROR1 (Uniprot ID: Q01973) was constructed into pLVX-puro plasmid (Clontech, Cat#632164). Then, the resulting plasmid was electroporated into HEK293 cells (ATCC® CRL-1573™) using an electroporator (Invitrogen, Neon™ Transfection System, MP922947). After electroporation, the obtained cells were transferred to DMEM medium (Gibco, 11995065) containing 10% FBS (Gibco, 15140-141) by volume and without antibiotics, and then transferred to 10×10 cm cell culture dishes for culture for 48 hours. The cells were then distributed into 96-well cell culture plate at an average density of $10^4$ cells/well, and puromycin was added at a final concentration of 2 μg/mL for screening pressure. After about 2 weeks, the cell lines that formed clones were picked up for identification.

Flow cytometric assay of huROR1-HEK293 cells: The above obtained cell lines in the logarithmic growth phase were digested and plated into 96-well plates. After washing with FACS buffer (1× PBS buffer containing 2% FBS by volume), the primary antibody (99961.1) gradient diluted in PBS was added and incubated at 4°C for 30 min. After washing, the prepared fluorescent secondary antibody anti-human IgG Fc (abcam, 98596) was added and incubated at 4°C for 30 min. Finally, the cells were detected by flow cytometer (Beckman, CytoFLEXAOO-1-1102). The rusults show that huROR1-HEK293 cell line with high expression of human ROR1 on the surface was obtained.

## Example 3 Animal immunization and immune library construction

3.1 Immunization regimen

Three Balb/C mice (Shanghai Lingchang Biotechnology Co., Ltd.) were cross-immunized with huROR1-huFc and huROR1-His antigens by subcutaneous injection and intraperitoneal injection, once every two weeks, for a total of four immunizations. One week after the fourth immunization, the blood was collected from mice for immune titer assay, and finally, huROR1-huFc was used for booster immunization once.

3.2 Assessment of serum antibody titers in mice after immunization

ELISA plate was coated with 2 μg/mL huROR1-His and huROR1-huFc respectively at 4°C overnight (30μL/well). After washing three times, the plate was blocked with 5% skim milk prepared in PBS at room temperature for 1 hour; after washing three times, the plate was added mouse serum gradient diluted in PBS, and added antibody 99961.1 as positive control, and incubated at room temperature for 1 hour; after washing, the plate was added the secondary antibody Goat-anti-mouse-IgG(1+2a+2b+3)-HRP (Jackson, 115-035-164) or Goat-anti-human-Kappa+Lambda-HRP (Millipore, AP502P+AP506P) diluted in PBS and incubated at room temperature for 1 hour, the plate was then washed six times and added TMB for color development for 5-20 min. The data was read at OD450 with the microplate reader after the color development was terminated, and the data was processed and plotted using Graphpad prism. The results showed that the serum titers of all three mice met the standards.

3.3 Construction of antibody gene phage display library

After the immunization was completed, the spleens were taken from mice, and the splenocytes were collected after grinding and filtering. 1 mL of TRIzol™ Reagent (Thermo Fisher, 15596026) was added to lyse the splenocytes, and the total RNAs were extracted by the phenol-chloroform method. The extracted RNAs were reverse transcribed into cDNAs using a reverse transcription kit (TaKaRa, 6210A). The cDNAs were then used as PCR templates and specific primers for the mouse antibody sequences were used to amplify the antibody light and heavy chain variable region genes respectively. The PCR product was digested with two enzymes NcoI and NotI to obtain the antibody gene fragments, which were inserted into the phage display vector and ligated with T4 ligase. The ligation products were recovered using a DNA recovery kit (Omega, D6492-02) and finally transformed into competent *Escherichia coli* SS320 (Lucigen, MC1061F) using Electroporator (Bio-Rad, MicroPulser). The electroporated bacteria were spread on a 2-YT ($C^+/K^+$ 2-YT) solid plate containing ampicillin and tetracycline, and the SS320 bacteria that were correctly transformed with the antibody plasmid were amplified and packaged using VSCM13 helper phage (purchased from Stratagene) to obtain a phage display library containing Fab sequences.

## Example 4 Screening of antibody gene phage display library

4.1 Cell-based screening of phage-displayed antibody gene library

hROR1-HEK293T cells were cultured in T25 flasks. When the cell growth density was close to 90%, the culture supernatant was removed and the cells were washed once with PBS (Yuanpei, B310KJ), then 2 mL of 4% paraformaldehyde (Shenggong, E672002-0500) was added for fixation for 0.5 h, and finally washed twice with PBS and used as the screening raw material. During screening, the phage display library was incubated with fixed hROR1-HEK293T cells at

room temperature for 1 hour, washed three times with 1×PBS, and then 2 mL of glycine-HCl (pH=2.0) was added and gently mixed for 10 minutes to elute the phage that specifically binds to human ROR1. The eluted supernatant then infected SS320 bacteria at logarithmic phase (Lucigen, 60512-1), allowed to stand for 30 minutes, and then cultured at 37°C, 220 rpm for 1 hour. VSCM13 helper phage was added, allowed to stand for 30 minutes, and continued to be cultured at 37°C, 220 rpm for 1 hour. After centrifugation, the cells were transfers to $C^+$ /$K^+$ 2-YT medium. The final phage was used for the second round of screening. The screening was repeated several times, and 10 clones were randomly selected for sequence analysis in each round to evaluate the library. After three rounds of screening, the sequences in the library were significantly enriched.

4.2 Immunotube-based and magnetic bead-based screening of antibody gene phage display library

The immunotube and magnetic bead were used to enrich specific antibodies against antigens, and the two methods complemented and verified each other.

Immunotube-based screening is a panning process, consisting of coating the antigen protein huROR1-His or huROR1-huFc on the surface of an immunotube with high adsorption capacity, adding the phage display antibody library to the immunotube to incubate with the antigen protein adsorbed on the surface of the immunotube, washing and eluting. After 2-4 rounds of panning, the specific monoclonal antibody Fab against the antigen is finally enriched. In this example, monoclonal antibody Fab against human ROR1 was enriched after three rounds of panning. For the specific method, refer to Example 2.4.2 in patent CN112250763B.

Magnetic bead-based screening is a panning process, consisting of labeling the antigen protein huROR1-His with biotin, binding the labeled antigen protein with magnetic beads coupled with streptavidin, incubating the antigen-bound magnetic beads with the antibody gene phage display library, washing and eluting. Usually specific monoclonal antibody against the antigen can be enriched in large quantities by 3-4 rounds of panning. In this example, biotin-labeled huROR1-His was used for phage display library screening, and monoclonal antibody Fab against human ROR1 was primarily screened after three rounds of panning. For the specific method, refer to Example 2.4.1 in patent CN112250763B.

4.3 Selection of single clones

The phage pool eluted in each round was tested by ELISA to evaluate the enrichment effect, and 10 clones were randomly selected from the phage pool in each round of screening for sequence analysis. The enrichment effect and the ratio of the sequencing repeatability were comprehensively analyzed to select the appropriate round for single clone selection.

The antigen protein huROR1-His was used for the ELISA monoclonal primary screening, and the antibody Fab binding to huROR1-His obtained from the primary screening was prepared as Fab lysate, and then the huROR1-overexpression HEK293 cells prepared in Example 2.1 were used for detection and verification by flow cytometry (FACS). A total of 11 antibody Fab molecules that specifically bind to human ROR1 were screened, and the obtained 11 mouse antibody Fabs were named according to the corresponding clone numbers (B62, B31, B32, B74, B34, B39, C38, C77, C42, M71 and M78). The specific FACS results are shown in Figures 1A-1B. The CDR amino acid sequences of the obtained mouse antibody Fabs are shown in Table 1. The CDR sequences are determined using the AbM definition.

Table 1. Amino acid sequences of CDR regions of murine antibodies

| Clone No. | SEQ ID NO | Sequence | |
|---|---|---|---|
| B62 | 1 | HCDR1 | GFTFSSYVMS |
| | 2 | HCDR2 | TINSNGGNTY |
| | 3 | HCDR3 | DSLGLRRRDYAMDY |
| | 28 | LCDR1 | VTSIDIDDDVN |
| | 29 | LCDR2 | AGNTLRP |
| | 30 | LCDR3 | LQSDNLPYT |

(continued)

| Clone No. | SEQ ID NO | Sequence | |
|---|---|---|---|
| B31 | 4 | HCDR1 | GFTFSRYAMS |
| | 5 | HCDR2 | SISRGGSTY |
| | 6 | HCDR3 | YFGNYDAMDY |
| | 31 | LCDR1 | KASQDINNYVS |
| | 32 | LCDR2 | RANRLVD |
| | 33 | LCDR3 | LQFDEFPYT |
| B32 | 7 | HCDR1 | GFAFSSYAMS |
| | 8 | HCDR2 | SINRDVTTY |
| | 9 | HCDR3 | GYYYGGGPYWFWDV |
| | 34 | LCDR1 | KASQDINSYLT |
| | 32 | LCDR2 | RANRLVD |
| | 33 | LCDR3 | LQFDEFPYT |
| B74 | 10 | HCDR1 | GFTFSIYAMS |
| | 11 | HCDR2 | SISGGGTAY |
| | 12 | HCDR3 | GYYYGGSPYWHFDV |
| | 35 | LCDR1 | KASQDINSYLS |
| | 32 | LCDR2 | RANRLVD |
| | 36 | LCDR3 | LQIDEFPYT |
| B34 | 4 | HCDR1 | GFTFSRYAMS |
| | 13 | HCDR2 | SISTGGSPY |
| | 14 | HCDR3 | GYYYNS SPHWYFAV |
| | 35 | LCDR1 | KASQDINSYLS |
| | 32 | LCDR2 | RANRLVD |
| | 37 | LCDR3 | LQYDEFPYT |
| B39 | 15 | HCDR1 | GFTFSTYAMS |
| | 16 | HCDR2 | SISTGASTY |
| | 17 | HCDR3 | EETTTAALYYAMDY |
| | 38 | LCDR1 | KASQDINNYLS |
| | 32 | LCDR2 | RANRLVD |
| | 33 | LCDR3 | LQFDEFPYT |
| C38 | 18 | HCDR1 | GFTFSSYAMS |
| | 8 | HCDR2 | SINRDVTTY |
| | 19 | HCDR3 | DYYYGS SLYYGMDY |
| | 31 | LCDR1 | KASQDINNYVS |
| | 32 | LCDR2 | RANRLVD |
| | 33 | LCDR3 | LQFDEFPYT |

(continued)

| Clone No. | SEQ ID NO | Sequence | |
|---|---|---|---|
| C77 | 20 | HCDR1 | GFSFSNYAMS |
| | 21 | HCDR2 | SINSGVTTY |
| | 12 | HCDR3 | GYYYGGSPYWHFDV |
| | 31 | LCDR1 | KASQDINNYVS |
| | 32 | LCDR2 | RANRLVD |
| | 33 | LCDR3 | LQFDEFPYT |
| C42 | 22 | HCDR1 | GFTFSNYIMS |
| | 23 | HCDR2 | TINSGGTNTY |
| | 24 | HCDR3 | YNGNYVWFAY |
| | 39 | LCDR1 | SASSSVSFIY |
| | 40 | LCDR2 | DTSNLAS |
| | 41 | LCDR3 | QQW SNYHFT |
| M71 | 4 | HCDR1 | GFTFSRYAMS |
| | 25 | HCDR2 | SINRGGTTY |
| | 26 | HCDR3 | DYYYGS SLYNGMDF |
| | 35 | LCDR1 | KASQDINSYLS |
| | 32 | LCDR2 | RANRLVD |
| | 33 | LCDR3 | LQFDEFPYT |
| M78 | 18 | HCDR1 | GFTFSSYAMS |
| | 25 | HCDR2 | SINRGGTTY |
| | 27 | HCDR3 | DYYYGSNLYNGMDY |
| | 35 | LCDR1 | KASQDINSYLS |
| | 32 | LCDR2 | RANRLVD |
| | 33 | LCDR3 | LQFDEFPYT |

**Example 5 Construction, expression and purification of antibody**

5.1 Construction of Plasmid

The VH coding sequence in the Fab sequence of the screened monoclonal antibodies B62, B31, B32, B74, B34, B39, C38, C77, C42, M71 and M78 was connected to the coding sequence of the human IgG1 heavy chain constant region ( SEQ ID NO: 52) to obtain the heavy chain coding sequence of the chimeric antibody, and the VL coding sequence in the Fab sequence was connected to the coding sequence of the kappa type (SEQ ID NO: 53) of the human light chain constant region (CL) to obtain the light chain coding sequence of the chimeric antibody. The antibody heavy and light chain coding sequence was respectively inserted into the eukaryotic expression vector plasmid pcDNA3.4 (Invitrogen), transformed into *Escherichia coli* DH5$\alpha$, and cultured at 37°C overnight. The plasmid was extracted using an endotoxin-free plasmid extraction kit (OMEGA, D6950-01) to obtain endotoxin-free antibody plasmid for eukaryotic expression.

5.2 Expression and Purification of Antibody

The full-length sequence of the antibody obtained above was expressed by the Expi CHO transient expression system (Thermo Fisher, A29133). The specific method was as follows: on the day of transfection, the CHO cell density was about $7\times10^6$ to $1\times10^7$ viable cells/mL, and the cell survival rate was >98%. At this time, the cells were adjusted to a final concentration of $6\times10^6$ cells/mL using fresh ExpiCHO expression medium pre-warmed at 37°C. The target plasmid was diluted with OptiPRO™ SFM pre-cooled at 4°C (1 $\mu$g plasmid was added to 1 mL of the culture medium), and

ExpiFectamine™ CHO reagent was diluted with OptiPRO™ SFM. The two was mixed in equal volumes and gently mixed by pipetting, resulting in ExpiFectamine™ CHO/plasmid DNA mixture, which was incubated at room temperature for 1-5 min, slowly added to the prepared cell suspension with gently shaking, and finally placed in a cell culture shaker and cultured at 37°C, 8% $CO_2$.

18-22 hours after transfection, ExpiCHO™ Enhancer reagent and ExpiCHO™ Feed reagent were added to the culture medium, and the flask was placed on a shaker at 32°C with 5% $CO_2$ to continue culturing. On day 5 after transfection, the same volume of ExpiCHO™ Feed reagent was added slowly, along with gently mixing the cell suspension. Seven days after transfection, the cell culture supernatant expressing the target protein was collected and centrifuged at 15,000 g for 10 min. The resulting supernatant was subjeted to affinity purification using MabSelect SuRe LX (GE, 17547403), and then the target protein was eluted with 100 mM sodium acetate (pH 3.0), followed by neutralization with 1 M Tris-HCl, and finally the resulting protein was placed into PBS buffer using ultrafiltration concentration tubes (Millipore, UFC901096).

**Example 6 Physical and chemical property of antibody**

In this example, the relative molecular weight and purity of the antibodies obtained above were detected by SDS-PAGE and SEC-HPLC.

6.1 Assessment of antibody by SDS-PAGE

Preparation of non-reducing solution: 1 μg of each obtained antibody and quality control product IPI (Ipilimumab) were separately added to 5×SDS loading buffer and 40 mM iodoacetamide, heated in a 75°C dry bath for 10 min, cooled to room temperature, and centrifuged at 12,000 rpm for 5 min to obtain the supernatant.

Preparation of reducing solution: 2 μg of each obtained antibody and quality control product IPI were separately added to 5×SDS loading buffer and 5 mM DTT, heated in a 100°C dry bath for 10 min, cooled to room temperature, and centrifuged at 12000 rpm for 5 min to obtain the supernatant. The supernatant was added to Bis-tris 4-15% gradient gel (GenScript) for gel electrophoresis and the protein bands were visualized by Coomassie brilliant blue staining.

The protein gel with chromogenic protein bands was scanned using EPSON V550 color scanner (decolorization was performed until the gel background was transparent), and the purity of the reduced and non-reduced bands was calculated using ImageJ according to the peak area normalization method.

The rusults show that the bands of each antibody in non-reducing gel were around 150kD, and the bands in reducing gel were around 55kD and 25kD, which were in line with the expected size. The purity of all antibodies obtained in this application detected by reducing gel was greater than 95% (Table 2).

6.2 Assessment of antibody monomer purity by SEC-HPLC

Preparation of materials: 1. Mobile phase: 150 mmol/L phosphate buffer, pH 7.4; 2. Sample preparation: Each antibody and quality control product IPI were diluted to 0.5 mg/mL with mobile phase solution, respectively. Agilent HPLC 1100 or Shimadzu LC2030C PLUS liquid chromatograph was used, column was XBridge BEH (SEC 3.5 μm, 7.8 mm I.D.×30 cm), Waters flow rate was set to 0.8 mL/min, injection volume was 20 μL, VWD detector wavelengths were 280 nm and 214 nm. Blank solution, IPI quality control solution and antibody sample solution were injected sequentially. The percentages of high molecular weight polymers, antibody monomers and low molecular weight substances in the samples were calculated according to the area normalization method.

The results are shown in Figures 2A-2K and Table 2. Except for antibody B74, the SEC monomer purity of the other antibodies was greater than 96%.

Table 2 Expression levels and physicochemical properties of antibodies obtained in this application

| Clone No. | SDS-PAGE (%) | SEC-HPLC (%) |
|---|---|---|
| 99961.1 | >95.0 | 98.68 |
| B31 | >95.0 | 96.45 |
| B62 | >95.0 | 99.1 |
| B32 | >95.0 | 98.79 |
| B34 | >95.0 | 99.29 |
| B39 | >95.0 | 98.6 |
| B74 | >95.0 | Abnormal peak shape |

(continued)

| Clone No. | SDS-PAGE (%) | SEC-HPLC (%) |
|---|---|---|
| C38 | >95.0 | 98.9 |
| C42 | >95.0 | 99.39 |
| C77 | >95.0 | 100 |
| M71 | >95.0 | 100 |
| M78 | >95.0 | 98.93 |

**Example 7 Assessment of binding activity of antibody to the antigen**

In this example, the binding profiles of 11 antibodies (B62, B31, B32, B34, B39, B74, C38, C42, C77, M71 and M78) to human ROR1 antigen protein huROR1-His were detected by the ELISA assay, and the binding abilities of the antibodies (B62, B31, B32, B34, B39, B74, C38, C42, C77, M71 and M78) to huROR1-HEK293 cells over-expressing human ROR1 and A549 tumor cells were also detected by the FACS assay. A549 tumor cells are a human non-small cell lung cancer cell line that over-expresses human ROR1.

7.1 Assessment of the binding ability of antibody to antigen protein huROR1-His by ELISA assay

96-well ELISA plate was coated with 2 μg/mL huROR1-His (30 μL/well) at 4°C overnight. The next day, the plate was washed three times with PBST and then blocked with 5% skim milk for 2 h. After washing with PBST for three times, the gradient dilution solution (3.00000, 0.33333, 0.11111, 0.03704, 0.01235, 0.00412, 0.00046, 0.00005 μg/mL) of each antibody and the positive control antibody 99961.1 were added and incubated for 1 h. After washing with PBST for three times, the secondary antibody Goat-anti-human Fc-HRP (abcam, ab97225) was added and incubated for 1 h. After incubation, the plate was washed with PBST for 6 times and TMB (SurModics, TMBS-1000-01) was added for color development. According to the color development results, 2 M HCl was added to terminate the reaction, and the data were read at OD450 using a microplate reader (Molecular Devices, SpecterMax 190).

The results are shown in Figures 3A-3B. All antibodies obtained in the present application have good affinity activity to antigen protein huROR1-His, and the affinity of each antibody is equivalent to 99961.1.

7.2 Assessment of the binding ability of antibody to huROR1-HEK293 and A549 tumor cells by FACS

In this example, the binding activity of the antibody was evaluated using two types of cells, huROR1-HEK293 cells over-expressing human ROR1 and A549 tumor cells.

The specific method was as follows: huROR1-HEK293 cells or A549 cells in the logarithmic growth phase were prepared to a single-cell suspension, the density was adjusted to $1 \times 10^6$ cells/mL, then 100 μL/well cells were added to a 96-well round-bottom plate, centrifuged at 4°C, 300 g, and the supernatant was removed. The gradient dilution solution of antibodies obtained in this application and the positive control antibody 99961.1 were added to the corresponding wells, thoroughly mixed and incubated at 4°C for 30 min. After the incubation, the cell mixture was washed three times and then 100 μL of 1:300 diluted secondary antibody Goat $F(ab')_2$ Anti-Human IgG-Fc (abcam, ab98596) was added. The mixture was incubated at 4°C in the dark for 30 min. After washing three times, the cells were detected by flow cytometry (Beckman, CytoFLEX AOO-1-1102).

The results are shown in the figure. On A549 cells (Figures 4A-4B), except for C42, the other antibodies all have affinity superior to that of the control antibody 99961.1; on huROR1-HEK293 cells (Figures 5A-5B), all the antibodies have affinity comparable to that of the control antibody 99961.1.

**Example 8 Assessment of cross-reactivity of antibody between species and homologous groups**

In this example, the cross-reactivity of each of the antibodies obtained in the present application was tested between species and homologous groups. The cross-activity of the antibodies obtained in the present application between species and homologous groups was identified with the mouse ROR1 antigen protein MusROR1-His and the human ROR2 antigen protein huROR2-His prepared in Example 1.2.

8.1 Assessment of cross-reactivity of antibody between species.

96-well ELISA plate was coated with 2 μg/mL MusROR1-His (30 μL/well) at 4°C overnight. The next day, the

plate was washed three times with PBST and then blocked with 5% skim milk for 2 h. After washing with PBST for three times, the gradient dilution solution (1.00000, 0.11111, 0.03704, 0.01235, 0.00412, 0.00137, 0.00015, 0.00002 μg/mL) of each antibody and the positive control antibody 99961.1 were added and incubated for 1 h. After washing with PBST for three times, the secondary antibody Goat-anti-human Fc-HRP (abcam, ab97225) was added and incubated for 1 h. After incubation, the plate was washed with PBST for 6 times and TMB (SurModics, TMBS-1000-01) was added for color development. According to the color development results, 2 M HCl was added to terminate the reaction, and the data were read at OD450 using a microplate reader (Molecular Devices, SpecterMax 190).

The results are shown in Figures 6A-6B and Table 3. B62, B32, C38 and C42 bind to the antigen protein MusROR1-His and show good cross-reactivity with mouse. The other antibodies and the positive control antibody 99961.1 do not bind to the mouse antigen protein. Therefore, the antibodies B62, B32, C38 and C42 obtained in the present application have a wide range of uses in experiments and tests based on mouse models.

8.2 Assessment of cross-reactivity of antibody of the present application between homologous groups

96-well ELISA plate was coated with 2 μg/mL huROR2-His (30 μL/well) at 4°C overnight. The next day, the plate was washed three times with PBST and then blocked with 5% skim milk for 2 h. After washing with PBST for three times, the gradient dilution solution of the antibody and positive control antibody 99961.1 were added and incubated for 1 h. After washing with PBST for three times, the secondary antibody Goat-anti-human Fc-HRP (abcam, ab97225) was added and incubated for 1 h. After incubation, the plate was washed with PBST for 6 times and TMB (SurModics, TMBS-1000-01) was added for color development. According to the color development results, 2 M HCl was added to terminate the reaction, and the data were read at OD450 using a microplate reader (Molecular Devices, SpecterMax 190).

The results are shown in Figures 7A-7B and Table 3. None of the antibodies bind to the antigen protein huROR2-His, indicating that the antibodies prepared in the present application can specifically bind to human ROR1.

Table 3 Cross-activity of antibody obtained in this application between species and homologous groups

| Clone No | Species | MusROR1-His | huROR2-His |
|---|---|---|---|
| B31 | murine | no binding | no binding |
| B62 | murine | binding | no binding |
| B32 | murine | binding | no binding |
| B34 | murine | no binding | no binding |
| B39 | murine | no binding | no binding |
| B74 | murine | no binding | no binding |
| C38 | murine | binding | no binding |
| C42 | murine | binding | no binding |
| C77 | murine | no binding | no binding |
| M71 | murine | no binding | no binding |
| M78 | murine | no binding | no binding |

**Example 9 Assessment of internalization rate of antibody**

In this example, the internalization rate of the antibodies obtained in this application was detected by using two detection methods, FACS and Fab-Zap. The FACS-based internalization detection is to detect the antibody internalization rate in a short period of time by using supersaturated antibody-binding cells; and the Fab-Zap method reflects the long-term cumulative effect of internalization rate by binding cells with different concentrations of Fab-Zap toxin-conjugated antibodies, and killing the target cells by the toxins entering the cells through internalization.

9.1 Assessment of internalization rate of the antibody of the present application by FACS assay

Antibody dilution: The test antibody was diluted with DMEM complete medium to a final concentration of 10.0000 μg/mL.

Cell treatment: huROR1-HEK293 cells were digested and added into complete DMEM medium. After thoroughly mixing, the cells was counted and determined for their viability. $10^6$ cells were taken and added to 1.5 mL centrifuge tubes,

centrifuged at 300 g for 5 minutes, the supernatant was discarded, 1 mL pre-cooled DMEM medium was used for re-suspension, centrifuged at 300 g for 5 minutes, and the supernatant was discarded.

Primary antibody incubation: 1000 μL of the pre-cooled diluted antibody was taken and added to the centrifuge tube containing cells to prepare the antibody-cell suspension, which was then added to a 96-well plate and incubated at 4°C.

Extracellular secondary antibody incubation: The suspension in the 96-well plate was quickly transfered to a second 96-well plate. 180 μL of pre-cooled FACS buffer was added to each well of the second 96-well plate and washed twice. Then, 100 μL of diluted secondary antibody FITC-labeled anit-huFc or RPE-labeled anit-huFc (the secondary antibody was diluted 1:150 using FACS buffer) was added to each well; incubated at 4°C for 30 min.

Cell fixation: After incubation, the supernatant was discarded by centrifugation, 180 μL of pre-cooled FACS buffer was added to each well, and the cells were washed twice. The supernatant was removed by centrifugation, and the cells were fixed with 100 μL of 4% paraformaldehyde in each well for 30 min at room temperature.

Cell membrane disruption: After fixation, 180 μL FACS buffer was added and washed twice. 100 μL of pre-warmed 0.5% Triton X-100 was added to each well and permeabilization for 5 min at room temperature.

Intracellular secondary antibody incubation: After washing the 96-well plate, 180 μL of pre-warmed Permeabilization Buffer (Invitrogen™ eBioscience™, 00-8333-56) was added to each well and washed twice. 100 μL of diluted secondary antibody RPE-labeled anit-huFc (diluted 1:150 using Perm buffer) was added to each well, and to be incubated at room temperature for 60 min.

Fluorescence Detection: After washing the 96-well plate, 100 μL FACS buffer was used for re-suspension, and detection was performed by flow cytometry.

Two groups of different fluorescence settings were used in the example to stain each sample. The first group was set as FITC+PE, that is, FITC secondary antibody was first used for staining the extracellular primary antibody, and then the PE secondary antibody was used for staining the intracellular primary antibody after the membrane was permeabilized. In this group, PE was the intracellular signal and FITC was the extracellular signal. The second group was set as PE+PE, that is, PE secondary antibody was first used for staining the extracellular primary antibody, and then the PE secondary antibody was used for staining the intracellular primary antibody after the membrane was permeabilized. In this group, PE was the sum of intracellular and extracellular signals. At the same time, both groups of samples were detected using FITC and PE channels, and the values of internalization rate were calculated as detection values for the PE channel. The specific formula is:

$$\text{Internalization rate} = \text{group 1 (FITC+PE) PE channel/group 2 (PE+PE) PE channel} \times 100\%.$$

The results are shown in figures 8A-8B. The results of this example show that, for all antibodies prepared in this application, the internalization rate is better than that of the control antibody 99961.1 in a short period of time (within 3 hours).

9.2 Assessment of internalization rate of the antibody by Fab-Zap method

The internalization activity of antibodies was detected in this experiment by using the cytotoxicity of antibody-mediated Fab-ZAP internalization. Fab-ZAP is a Fab fragment linked to saporin, a ribosomal inhibitor that can inhibit protein synthesis and cause cell death. The Fab-ZAP used in this experiment is a Fab fragment that can bind to the human Fc of the chimeric antibody. After incubation with the chimeric antibody, Fab-ZAP makes the chimeric antibody carry the toxin. When the chimeric antibody is internalized, the toxin enters the cell along with the chimeric antibody, resulting in cell death. Then, the activity of the cell is detected by MTS (Promega, G3580) to determine whether the antibody is internalized.

The detail experimental procedures were as follows: first, Fab-Zap was diluted to 0.4 nM with DMEM complete medium, and then each of the antibodies obtained in the application and the positive control antibody were gradiently diluted (0.020000, 0.006667, 0.002222, 0.000741, 0.000247, 0.000082, 0.000027, 0.000009 μg/mL) with the 0.4 nM Fab-Zap to prepare antibody dilution solutions. The huROR1-HEK293 cells in the logarithmic growth phase were made into a single cell suspension, the density was adjusted to $6\times10^6$ cells/mL, and 50 μL was inoculated into each well of a 96-well plate. Then, the above antibody dilution was taken and added into the cell culture plate, 50 μL per well, and the suspension was mixed thoroughly by pipetting. The cell culture plate was placed in a 37°C cell culture incubator and incubated for 48 hours. After the incubation, 7.5 μL of TritonX-100 solution was added to each well, gently mixed by tapping, and the cell culture plate was placed in a 37°C cell culture incubator for 0.5 h. Then, 20 μL of MTS was added to each well and incubated at 37°C for 1-4 hours. Finally, the cell culture plate was centrifuged at 1000 rpm for 5 min, and the data was read by a microplate reader at a detection wavelength of A492.

The results are shown in Figures 9A-9F, indicating that under the antibody concentration conditions specified in

this example, the internalization effect of the antibodies prepared in the present application is equivalent to that of the control antibody. Considering that cells will exhibit sensitive reaction to toxins only above a certain threshold, it is harder to widen the gap when there is little difference in accumulated toxins.

**Example 10 Assessment of affinity kinetics (Gator) of antibody**

In this example, the affinity of the antibodies obtained in the present application and the positive control antibody 99961.1 to the antigen protein huROR1-His was detected based on the Gator device.

First, Q buffer was prepared with PBS (10 mM pH7.4) (IgG-free, purchased from Jackson ImmunoResearch Lab) + 0.02% Tween 20 (purchased from thermo) + 0.2% BSA (purchased from source culture), and the stock solution of the test antibody was diluted with the prepared Q buffer to a working solution with a final concentration of 30 nM; the stock solution of the antigen protein huROR1-His was prepared with Q buffer into a working solution with multiple dilutions (480, 240, 120, 60, 30, 15, 7.5 nM), and detection and analysis was then performed with Gator instrument and its accompanying software, selecting the Advanced Kinetics experimental mode is used. The rusults are shown in Table 4.

The results show that except for C42 antibody, which has comparable affinity to the control antibody 99961.1, the other antibodies all have affinity superior to that of the control antibody 99961.1 by one order of magnitude or more. Among them, B62 antibody has affinity of 9.84E-10, superior to that of the control antibody 99961.1 by about two orders of magnitude.

Table 4 Affinity kinetic test result of antibody obtained in this application

| Clone No. | KD (M) | Ka (1/Ms) | Kd (1/s) | $R^2$ | Rmax (nm) |
|---|---|---|---|---|---|
| 99961.1 | 1.35E-08 | 5.29E+05 | 7.12E-03 | 0.995 | 0.669 |
| B31 | 5.67E-09 | 3.83E+05 | 2.17E-03 | 0.997 | 0.723 |
| B32 | 1.25E-09 | 3.01E+05 | 3.77E-04 | 0.994 | 0.874 |
| B34 | 2.52E-09 | 2.41E+05 | 6.09E-04 | 0.995 | 0.782 |
| B39 | 4.43E-09 | 3.04E+05 | 1.35E-03 | 0.995 | 1.060 |
| B62 | 9.84E-10 | 3.79E+05 | 3.73E-04 | 0.995 | 1.400 |
| B74 | 1.44E-09 | 3.23E+05 | 4.64E-04 | 0.995 | 1.080 |
| C38 | 1.34E-09 | 3.14E+05 | 4.22E-04 | 0.995 | 0.935 |
| C42 | 2.96E-08 | 1.10E+05 | 3.25E-03 | 0.998 | 0.652 |
| C77 | 2.47E-09 | 3.14E+05 | 7.74E-04 | 0.996 | 0.959 |
| M71 | 1.54E-09 | 3.51E+05 | 5.40E-04 | 0.995 | 0.916 |
| M78 | 1.86E-09 | 3.30E+05 | 6.14E-04 | 0.995 | 0.920 |

**Example 11 Analysis of affinity Kinetics (Biacore) of antibody**

In this example, the affinity of the antibodies obtained in the present application and the positive control antibody 99961.1 to the antigen protein huROR1-His was detected based on the Biacore device.

Protein coupling: The huROR1-His protein produced in Example 1.2 was diluted to 5.6 μg/mL with NaAc buffer, pH 5.0, the flow rate was set to 10 μL/min, the chip activation time was set to the default value of 420 s with a mixture of 1-ethyl-(3-dimethylaminopropyl)carbodiimide (EDC) and N-hydroxysuccinimide (NHS), and the antigen protein huROR1-His was fixed to a level of about 75 RU using a coupling mode with a preset coupling amount, and the activated groups that were not bound to the test sample were blocked with ethanolamine.

Sample test conditions: PBS buffer (pH 7.4) containing 0.05% Tween-20 was used as the running buffer, and the running buffer was used as the control test sample. A series of antibody concentrations (4 nM, 20 nM) were set. During sample analysis, the flow rate was set to 30 μL/min, the binding time was 120 s, and the dissociation time was 360 s. After the dissociation was completed, 10 mM Gly-HCl (pH 2.0) was used for regeneration for 20 s to completely remove the antibody bound to the ligand.

Parameter fitting: The experiment was run in multiple cycles, with the response signal taking the analysis time as the horizontal axis and the response value as the vertical axis. After double reference subtraction, the obtained data were fitted by BIAcore T200 analysis software. The fitting model adopted was 1:1 Langmuir binding model, and Affinity metrics such as the binding dissociation constant were determined.

The results are shown in Table 5. Antibody B62, with a KD of 6.39E-11, has affinity about 2 orders of magnitude better than that of control antibody 99961.1, the later has KD of 1.98E-9; in addition, the affinity of antibody B31 is comparable to that of control antibody 99961.1.

Table 5 Affinity kinetic test result of antibody obtained in this application

| Clone No. | ka (1/Ms) | kd (1/s) | t 1/2 (s) | KD (M) | Rmax (RU) | $Chi^2$ ($RU^2$) |
|---|---|---|---|---|---|---|
| 99961.1 | 6.94E+05 | 1.37E-03 | 504.4 | 1.98E-09 | Local Fit | 3.13 |
| B31 | 6.53E+05 | 8.17E-04 | 848.8 | 1.25E-09 | Local Fit | 1.61 |
| B62 | 1.21E+06 | 7.74E-05 | 8954.3 | 6.39E-11 | Local Fit | 0.92 |

**Example 12 Epitope grouping by affinity kinetics**

The antibodies obtained in this application and the positive control antibody 99961.1 were divided into various groups based on the epitope in this example by using the affinity kinetics method.

The detail experimental procedures were as follows: First, prepare Q buffer by mixing PBS (10 mM, pH 7.4) (IgG-free, purchased from Jackson ImmunoResearch Lab) with 0.02% Tween 20 (purchased from Thermo) and 0.2% BSA (purchased from Source culture). Dilute the antibody storage solution to a final concentration of 100 nM in the prepared Q buffer. Similarly, dilute the antigen huROR1-His storage solution to a 50 nM working solution in Q buffer. Detection and analysis were then conducted using the Gator instrument and its accompanying software, employing the Tandem settings based on the Epitope Binning experimental mode.

The specific detection and analysis steps were as follows:

Equilibration stage 1: The probe was equilibrated in Q buffer at 1000 rpm for 60 s;
Antigen immobilization stage: After the equilibration stage 1, the probe was immobilized in Q buffer containing 50 nM antigen at 400 rpm for 90 s;
Equilibrium stage 2: After the antigen immobilization stage, the probe was equilibrated in Q buffer at 1000 rpm for 60 s;
Antibody 1 binding stage: After the equilibrium stage 2 was completed, the probe was subjected to binding reaction in Q buffer containing 100 nM antibody 1 at 1000 rpm for 180 s;
Equilibrium stage 3: After the antibody 1 binding stage was completed, the probe was equilibrated in Q buffer at 1000 rpm for 30 s;
Antibody 2 binding stage: After the equilibrium stage 3 was completed, the probe was subjected to binding reaction in Q buffer containing 100 nM antibody 2 at 1000 rpm for 180 s;
Regeneration phase: After one assay, *i.e.* from equilibrium stage 1 to antibody 2 binding stage, was completed, the probe should be regenerated in Q buffer at 1000 rpm for 50 s before the next assay.

The results of the epitope grouping of each antibody obtained are shown in Table 6. The results show that the antibodies of the present application and the control antibody 99961.1 are divided into 2 epitope-based groups, in which only C42 and the control antibody share the same binding epitope, and the remaining antibodies have epitope different from that of the control antibody.

Table 6 Summary of epitope grouping results

| epitope grouping | Clone No. |
|---|---|
| 1 | 99961.1, C42 |
| 2 | B62, B32, C38, B74, M71, B34, M78, C77, B39, B31 |

**Example 13 Humanization of murine antibody**

The VH and VL sequences of the murine antibodies B31 and B62 screened in Example 4 were separately compared with known human antibody databases to find the human germline gene VH and VL sequences with the highest homology to the mouse VH and VL sequences respectively. The framework regions of the corresponding human germline gene VH and VL sequences (CDR and framework regions were defined by using AbM) were determined, and the complementarity determining region (CDR) sequences of the germline genes were replaced with the corresponding CDR sequences in the murine antibodies B31 and B62 of the present application. Then, with the help of computer prediction simulation, the murine amino acids in the framework regions of the murine antibodies B31 and B62 that have an important

effect on antigen binding were retained by back mutation. The amino acid sequences of the CDR regions of the humanized antibodies resulting from humanization of the murine antibodies B31 and B62 are shown in Table 7. Various antibodies resulting from humanization of B31 and B62 were constructed, expressed and purified by the method of Example 5, and the various antibody proteins resulting from humanization of B31 and B62 were identified by SDS-PAGE and SEC-HPLC. The results are shown in Table 8. The results show that each antibody exhibits band at around 150 kD in non-reducing gel, and bands at around 55 kD and 25 kD in reducing gel, which are consistent with the expected size. The purity of all antibodies obtained in this application is greater than 95% by reducing gel detection; the SEC monomer purity of all antibodies is greater than 96%.

Table 7 Amino acid sequences of CDR regions of humanized antibody

| Clone No. | SEQ ID NO | Sequence | |
|---|---|---|---|
| B31-H1L1, B31-H2L2, B31-H2L3, B31-H3L2, B31-H3L3, B31-H3L4, B31-H4L2, B31-H4L3, B31-H4L4 | 4 | HCDR 1 | GFTFSRYAMS |
| B31-H1L1, B31-H2L2, B31-H2L3, B31-H3L2, B31-H3L3, B31-H3L4, | 5 | HCDR 2 | SISRGGSTY |
| B31-H4L2, B31-H4L3, B31-H4L4 | 42 | HCDR 2 | AISRGGSTY |
| B31-H1L1, B31-H2L2, B31-H2L3, B31-H3L2, B31-H3L3, B31-H3L4, B31-H4L2, B31-H4L3, B31-H4L4 | 6 | HCDR 3 | YFGNYDAMDY |
| B31-H1L1, B31-H2L2, B31-H3L2, B31-H4L2 | 31 | LCDR1 | KASQDINNYVS |
| B31-H2L3, B31-H3L3, B31-H4L3 | 43 | LCDR1 | RASQDINNYVS |
| B31-H3L4, B31-H4L4 | 44 | LCDR1 | RASQDINNYLS |
| B31-H1L1, B31-H2L2, B31-H3L2, B31-H4L2 | 45 | LCDR2 | RANRLVD |
| B31-H2L3, B31-H3L3, B31-H4L3 | 46 | LCDR2 | RANRLVS |
| B31-H3L4, B31-H4L4 | 47 | LCDR2 | RANRLQS |
| B31-H1L1, B31-H2L2, B31-H2L3, B31-H3L2, B31-H3L3, B31-H3L4, B31-H4L2, B31-H4L3, B31-H4L4 | 48 | LCDR3 | LQFDEFPYT |
| B62-H1L1, B62-H1L2, B62-H2L2, B62-H2L3, B62-H2L4, B62-H3L2, B62-H3L3, B62-H3L4 | 1 | HCDR 1 | GFTFSSYVMS |
| B62-H1L1, B62-H1L2, B62-H2L2, B62-H2L3, B62-H2L4, B62-H3L2, B62-H3L3, B62-H3L4 | 2 | HCDR 2 | TINSNGGNTY |
| B62-H1L1, B62-H1L2, B62-H2L2, B62-H2L3, B62-H2L4, B62-H3L2, B62-H3L3, B62-H3L4 | 3 | HCDR 3 | DSLGLRRRDYAMDY |
| B62-H1L1, B62-H1L2, B62-H2L2, B62-H2L3, B62-H3L2, B62-H3L3 | 28 | LCDR1 | VTSIDIDDDVN |
| B62-H2L4, B62-H3L4 | 49 | LCDR1 | QTSIDIDDDVN |
| B62-H1L1, B62-H1L2, B62-H2L2, B62-H2L3, B62-H3L2, B62-H3L3 | 29 | LCDR2 | AGNTLRP |
| B62-H2L4, B62-H3L4 | 50 | LCDR2 | AGNTLET |

(continued)

| Clone No. | SEQ ID NO | Sequence | |
|---|---|---|---|
| B62-H1L1, B62-H1L2, B62-H2L2, B62-H2L3, B62-H2L4, B62-H3L2, B62-H3L3, B62-H3L4 | 30 | LCDR3 | LQSDNLPYT |

Table 8 Physicochemical property of humanized antibody

| Clone No. | SDS-PAGE(%) | SEC(%) |
|---|---|---|
| B31 | >95.0 | 100 |
| B31-H1L1 | >95.0 | 98.34 |
| B31-H2L2 | >95.0 | 100 |
| B31-H2L3 | >95.0 | 100 |
| B31-H3L2 | >95.0 | 100 |
| B31-H3L3 | >95.0 | 100 |
| B31-H3L4 | >95.0 | 100 |
| B31-H4L2 | >95.0 | 100 |
| B31-H4L3 | >95.0 | 99.1 |
| B31-H4L4 | >95.0 | 100 |
| B62 | >95.0 | 98.72 |
| B62-H1L1 | >95.0 | 100 |
| B62-H1L2 | >95.0 | 100 |
| B62-H2L2 | >95.0 | 100 |
| B62-H2L3 | >95.0 | 100 |
| B62-H2L4 | >95.0 | 96.73 |
| B62-H3L2 | >95.0 | 100 |
| B62-H3L3 | >95.0 | 100 |
| B62-H3L4 | >95.0 | 97.07 |

**Example 14 Assessment of antigen affinity of humanized antibody**

In this example, the affinity of the humanized antibody to the human ROR1 antigen protein huROR1-His was detected based on the ELISA method, and the affinity of the humanized antibody to A549 tumor cells was also detected based on the FACS assay. Specific testing method was referred to Example 7.

The ELISA results are shown in Figures 10A-10E. The humanized antibodies have affinity to the antigen protein comparable to that of the corresponding parent molecule and of the control antibody 99961.1.

The FACS results are shown in Figures 11A-11B. Except for B31-H3L4, B31-H4L3, B31-H4L2 and B31-H4L4, the other humanized antibodies have affinity to the antigen protein on A549 tumor cells comparable to that of the corresponding parent molecule and of the control antibody 9996.1.

**Example 15 Assessment of internalization rate of humanized antibody**

In this example, the humanized antibodies B31-H3L3 and B62-H3L3 in Example 13 were subjected to Fab-Zap-based internalization detection. The specific method was referred to Example 9. The results are shown in Figure 12. The internalization rate of B31-H3L3 and B62-H3L3 is comparable to that of the control antibody 99961.1.

**Example 16 Preparation of ADC**

In this example, antibodies B62-H3L3, B31-H3L3 and control antibody 99961.1 were conjugated with toxin

MMAE (a tubulin inhibitor with anticancer activity) to construct antibody-drug conjugates. MMAE was connected to the linker MC-VC-PAB to form MC-VC-PAB-MMAE, and the linker was covalently linked to the sulfhydryl group on the cysteine of the antibody, thereby conjugating the antibody to MMAE to obtain an antibody-drug conjugate. IgG1 antibodies have 16 pairs of cysteine residues, which are present in the form of 12 intrachain and 4 interchain disulfide bonds. The interchain disulfide bonds are solvent accessible and can be reduced by reducing agents to form eight sulfhydryl groups, which then become conjugation targets (McCombs J, Owen S. Antibody drug conjugates: design and selection of linker, payload and conjugation chemistry. AAPS J. 2015;17:339-51).

The specific preparation method was as follows:

Antibodies B62-H3L3, B31-H3L3 and control antibody 99961.1 were taken out of the -80°C refrigerator, thawed and transferred to 15 mL 30KD ultrafiltration centrifuge tubes separately, and coupling buffer (components per 1 L: $Na_2HPO_4 \cdot 2H_2O$ 6.86 g, $NaH_2PO_4 \cdot H_2O$ 1.58 g, purified water to a total of 1000 g, pH7.4) was added to a total of 15 mL. Then, the soloutions were centrifuged at 4500 rpm for about 30 min, concentrated to 2-3 mL, and replenished with dialysate (components per 1 L: histidine 0.73 g, histidine hydrochloride monohydrate 1.12 g, purified water to a total of 1000 g, pH6.0) to a final volume of 15 mL. The dialysis was repeated 8-10 times to obtain the antibody stock solution, and the antibody concentration was measure after dialysis.

The thiol group on the cysteine of the antibody was reduced by a reduction reaction system, which was formed by adding the antibody stock solution, 10 mM disulfide bond reducing agent TCEP stock solution (tris(2-carboxyethyl) phosphine hydrochloride stock solution, components per 1 L: $Na_2HPO_4 \cdot 2H_2O$ 6.86 g, $NaH_2PO_4 \cdot H_2O$ 1.58 g, purified water to a total of 1000 g), 10 mM DTPA stock solution (diethylenetriaminepentaacetic acid stock solution, contents per 1 L: DTPA 3.90 g, NaOH 1.20 g, purified water to a total of 1000 g) and coupling buffer in order. The amount of each component added to the reduction reaction system is shown in Table 9, so that the antibody concentration in the reduction reaction system is 5 mg/mL, the final concentration of DTPA is 1 mM, the molar ratio of TCEP to B62-H3L3 or B31-H3L3 is 2, and the molar ratio of TCEP to 9996.1 is 2.2. After thoroughly mixing, the reduction reaction system was placed in a 25°C constant temperature shaker, with a rotation speed of 400 rpm, for 2 h of reduction reaction.

MC-VC-PAB-MMAE was weighted and dissolved in DMSO to prepare a 5 mM MC-VC-PAB-MMAE stock solution. After the reduction reaction was completed, the MC-VC-PAB-MMAE stock solution was added to the reduction reaction system in an ice-water bath in order, and the added amount was as shown in Table 10, to prepare a coupling reaction system. After thoroughly mixing, the coupling reaction system was placed in a 25°C constant temperature shaker at 400 rpm and coupled for 1 h, to obtain a solution containing ADC.

After the coupling reaction was completed, the solution containing ADC was centrifuged and filtered to obtain the ADC sample, which was transferred into a 15 mL 30KD ultrafiltration centrifuge tube. Dialysate was added to 15 mL, centrifuged at 4500 rpm for 20 min to concentrate to 2-3 mL, and then dialysate was replenished to 15 mL again. The dialysis was repeated 8-10 times. The ADC samples after dialysis were subjected to SEC-HPLC detection, HIC-HPLC detection, concentration detection, free drug detection, etc. The rusults are shown in Table 11. The rusults show that the ADCs with a purity of more than 99% were obtained.

Table 9 Components of reduction reaction system

| Clone No. | Concentration of antibody stock solution (mg/mL) | Volume of antibody stock solution added (mL) | Antibody Amount (mg) | Volume of 10mM TCEP stock solution added (μL) | Volume of 10mM DTPA stock solution added (μL) | Volume of coupling buffer added (μL) |
|---|---|---|---|---|---|---|
| B62-H3L3 | 7.14 | 10.5 | 74.97 | 103.4 | 1499 | 2892 |
| B31-H3L3 | 9.28 | 5.0 | 46.4 | 64 | 928 | 3288 |
| 99961.1 | 8.62 | 9.0 | 77.58 | 117.7 | 1552 | 4846 |

Table 10 Amount of MC-VC-PAB-MMAE stock solution added

| Clone No. | B62-H3L3 | B31-H3L3 | 99961.1 |
|---|---|---|---|
| Molar ratio of MC-VC-PAB-MMAE to antibody | 6.0 | 6.0 | 6.0 |
| Volume of 5mM MC-VC-PAB-MMAE stock solution added (μL) | 620 | 384 | 642 |

Table 11 Rusults of ADC samples

| Clone No. | SEC-HPLC | Concentration (mg/mL) | Residue of free drug | HIC-HPLC | | | | | |
|---|---|---|---|---|---|---|---|---|---|
| | | | | Average DAR | D0% | D2% | D4% | D6% | D8% |
| B62-H3L3-MMAE | 99.9% | 9.9 | not detected | 3.5 | 6.40 | 34.11 | 41.47 | 13.76 | 4.26 |
| B31-H3L3-MMAE | 99.6% | 6.5 | not detected | 3.5 | 6.50 | 34.45 | 41.49 | 13.88 | 3.67 |
| 99961.1-MMAE | 99.8% | 7.1 | 0.03% | 3.8 | 4.61 | 30.76 | 42.30 | 16.36 | 5.97 |

**Example 17 Assessment of antigen-binding activity of ADC**

In this example, the ability of the prepared ADCs (B62-H3L3-MMAE and B31-H3L3-MMAE) binding to human ROR1 on tumor cells A549 and HT-29 (human colon cancer cells) was detected based on the FACS assay.

The specific method was as follows: A549 cells or HT-29 cells in the logarithmic growth phase were prepared to a single-cell suspension, the density was adjusted to 1 $\times$ $10^6$ cells/mL, 100 $\mu$L/well was added to a 96-well round-bottom plate, then centrifuged at 4°C, 300 g, and the supernatant was removed. The gradient dilution solutions (1.0000, 0.3333, 0.1111, 0.0370, 0.0123, 0.0041, 0.0014, 0.0001 $\mu$g/mL) of the antibodies of the present application (B62-H3L3 and B31-H3L3), ADCs of the present application (B62-H3L3-MMAE and B31-H3L3-MMAE), 99961.1, 99961.1-MMAE and negative control were added to the corresponding wells, respectively, mixed thoroughly and incubated at 4°C for 30 min. After the incubation, the cell mixture was washed three times and then 100 $\mu$L of 1:300 diluted secondary antibody Goat F(ab')$_2$ Anti-Human IgG-Fc (abcam, ab98596) was added. The mixture was incubated at 4°C in the dark for 30 min. After washing three times, the cells were detected by flow cytometry (Beckman, CytoFLEX AOO-1-1102).

The results are shown in Figures 13A-13B: The results of binding to A549 cells are shown in Figure 13A. After coupling, the activities of B62-H3L3-MMAE and 99961.1-MMAE binding to A549 cells are slightly inferior to those of the corresponding naked antibodies, and the activities of B62-H3L3 and B62-H3L3-MMAE binding to A549 cells are superior to those of the corresponding positive controls; The results of binding to HT-29 cells are shown in Figure 13B. The activities of the naked antibodies binding to HT-29 cells are equivalent to those of the corresponding ADCs , and the activities of B62-H3L3 and B62-H3L3-MMAE binding to HT-29 cells are slightly superior to those of the corresponding positive controls.

**Example 18. Assessment of ADC tumor cell killing effect based on the MTS assay**

In this example, A549 and HT-29 cells were used to detect the killing effect of the ADCs of the present application and the control ADC.

The specific method was as follows: A549 cells or HT-29 cells in the logarithmic growth phase were prepared to a single-cell suspension, the density of A549 was adjusted to 1 $\times$ $10^4$ cells/mL, and the density of HT-29 was adjusted to 1.5 $\times$ $10^4$ cells/mL, and 100 $\mu$L/well was added to a 96-well cell culture plate and cultured at 37°C, 5% $CO_2$ for 12 hours. Then, gradient diluted ADC samples (2000, 500, 250, 125, 62.5, 31.25, 15.625, 7.813, 1.953 nM) were added and cultured at 37°C, 5% $CO_2$ for 72 h (A549 cells) or 96 h (HT-29 cells). Then, 40 $\mu$L of MTS (Promega, G3580) was added to each well, incubated at 37°C for 1 h, and the data were read at OD492 using a microplate reader.

The results are shown in Figures 14A-14D: the killing effect of B31-H3L3-MMAE on two tumor cells A549 (Figure 14A) and HT-29 (Figure 14C) is slightly superior to that of the control 99961.1-MMAE, while the killing effect of B62-H3L3-MMAE on A549 (Figure 14B) and HT-29 (Figure 14D) is slightly inferior to that of the control 99961.1-MMAE.

**Example 19 Assessment of ADC tumor cell killing effect based on CCK8 assay**

In the example, Jeko-1 cells (human mantle cell lymphoma cells), MDA-MB-468 cells (human breast cancer cells) and NCI-H1944 cells (human lung cancer cells) were used to detect the killing effect of B31-H3L3-MMAE, B62-H3L3-MMAE and control ADC.

The specific method was as follows: Jeko-1 cells, MDA-MB-468 cells or NCI-H1944 cells in the logarithmic growth phase were prepared to a single-cell suspension, the density of Jeko-1 was adjusted to 1 $\times$ $10^5$ cells/mL, the density of MDA-MB-468 was adjusted to 2 $\times$ $10^5$ cells/mL and the density of NCI-H1944 was adjusted to 1.2 $\times$ $10^5$ cells/mL, and 90 $\mu$L/well single-cell suspension was added to a 96-well cell culture plate and cultured at 37°C, 5% $CO_2$ for 12 hours. Then, the gradiently diluted ADC samples (500, 158, 50, 15.8, 5, 1.58, 0.5, 0.158, and 0.05 nM) were added. The cells were cultured at 37°C and 5% $CO_2$ for 72 h. Then 10 $\mu$L of CCK8 (Bimake, B34304) was added to each well, incubated at 37°C for 1 h, and the data were read at OD450 using a microplate reader.

The experimental results are shown in Figures 15A-15C. The killing effects of B31-H3L3-MMAE and B62-H3L3-MMAE on three tumor cells, Jeko-1 (Figure 15A), MDA-MB-468 (Figure 15B) and NCI-H1944 (Figure 15C), are

comparable to those of the control 99961.1-MMAE, wherein the EC50 of B31-H3L3-MMAE on Jeko-1, MDA-MB-468 and NCI-H1944 is 38.24 nM, 15.24 nM and 117.3 nM, respectively, and the EC50 of B62-H3L3-MMAE on Jeko-1, MDA-MB-468 and NCI-H1944 is 50.14 nM, 28.64 nM and 128.6 nM, respectively.

**Example 20 Assessment of antigen-dependent killing effect of ADC based on CCK8 assay**

In this example, huROR1-HEK293 cells were used to detect the antigen-dependent killing effect of B31-H3L3-MMAE and control ADC.

The specific method was as follows: huROR1-HEK293 cells in the logarithmic growth phase were prepared to a single-cell suspension, the cell density was adjusted to $6 \times 10^4$ cells/mL, and 50 μL/well single-cell suspension was added to a 96-well cell culture plate. After culturing at 37°C, 5% $CO_2$ for 24 h, 50 μL of 100 μg/mL antibody B31-H3L3 or positive control antibody 99961.1 were added to each well. After incubation at 37°C for 2 h, the gradient dilution solution (42.6667, 21.3333, 10.6667, 5.3333) of the ADC B31-H3L3-MMAE or control ADC 99961.1-MMAE were added correspondingly. After culturing at 37°C, 5% $CO_2$ for 96 h, 30 μL of CCK8 (absin/Aibixin, abs50003) was added to each well and incubated at 37°C for 1-4h. Then, the plate was read at OD450 using a microplate reader. The system only adding ADC, but not adding antibody, was used as the control group.

The results are shown in Figure 16. The killing effect of the ADC on huROR1-HEK293 cells can be significantly competitively inhibited by the corresponding antibody, B31-H3L3 and 99961.1, demonstrating that the cell killing of the ADC prepared in the present application depends on the binding of the antibody connected to the ADC with the antigen on the cell surface, and is therefore antigen-dependent killing rather than non-specific killing by the connected toxin.

**Example 21 Assessment of internalization efficiency of ADC**

In this example, A549 and HT-29 tumor cells were used to detect the internalization efficiency of the ADC obtained in this application based on the FACS assay. For detail experimental methods, see Example 9.

The experimental results show that on A549 cells (Figure 17A), the internalization rate of B31-H3L3-MMAE is comparable to that of the control ADC 99961.1-MMA, the internalization rate of B62-H3L3-MMAE is slightly lower than that of the positive control ADC, and the internalization efficiencies of the ADCs are all higher than those of the corresponding antibodies. On HT-29 cells (Figure 17B), the internalization rate of B31-H3L3-MMAE is slightly higher than that of the positive control ADC, the internalization rate of B62-H3L3-MMAE is slightly lower than that of the positive control ADC, and the internalization efficiencies of the antibodies are generally lower than those of the corresponding ADCs.

**Example 22 Assessment of ADC inhibiting efficacy in mouse model bearing HT-29 tumor**

In this example, the anti-tumor effects of two candidate ADCs (B31-H3L3-MMAE and B62-H3L3-MMAE) in animals were verified, 99961.1-MMAE was used as a positive control, and the tumor cells used were colon cancer cells HT-29 (BNCC337732).

The specific method was as follows: 6-8 week-old female Balb/C nude mice weighing about 20 g (Beijing Weitong Lihua Laboratory Animal Technology Co., Ltd.) were used, and $1\times10^6$ HT-29 cells were injected subcutaneously unilaterally into each nude mouse. When the tumor volume reached about 100 $mm^3$, the mice were randomly grouped into cages. Totally 9 groups were divided, 6 tumor-bearing nude mice in each group, including a PBS negative control group, 5 candidate ADC groups (B31-H3L3-MMAE 0.4 mg/kg (mpk), B31-H3L3-MMAE 2 mg/kg, B31-H3L3-MMAE 10 mg/kg, B62-H3L3-MMAE 2 mg/kg and B62-H3L3-MMAE 10 mg/kg) and 3 positive control ADC groups (99961.1-MMAE 0.4 mg/kg, 99961.1-MMAE 2 mg/kg and 99961.1-MMAE 10 mg/kg). The drugs were administered by tail vein injection, twice a week, and the tumor volume was measured twice, for a total of 8 administrations/4 weeks (BIW*4). Tumor volume (V) was calculated as follows: $V = L \times W^2/2$ (where L is the longest tumor diameter and W is the shortest tumor diameter). One week after the end of the administration, the mice were euthanized, and the tumors were removed and the tumor weight was measured. The data of the tumor volume, tumor weight and mouse body weight change were analyzed and the Tumor growth inhibition was calculated. The results are shown in Figures 18A-18C and Table 12.

The experimental results show that there is no significant difference in the body weight of mice in each group, and there is no significant change in the body weight of mice in each group during the treatment, indicating that the mice have good tolerance to ADCs (Figure 18B); B31-H3L3-MMAE, B62-H3L3-MMAE and control ADC 99961.1-MMAE all show significant tumor inhibition effects under high-dose conditions (10 mpk). Under high-dose conditions, B31-H3L3-MMAE shows tumor inhibition effect equivalent to that of 99961.1-MMAE, and B62-H3L3-MMAE shows Tumor growth inhibition slightly weaker than that of the control ADC 99961.1-MMAE. Under lower dose conditions (2 mpk), the Tumor growth inhibition of B62-H3L3-MMAE is significantly better than that of the control ADC 99961.1-MMAE and of B62-H3L3-MMAE (Figure 18A, Figure 18C and Table 12).

Table 12 Tumor growth inhibition of ADC in mice

| | Tumor growth inhibition (TGI (%)) = (1-average tumor volume of experimental group/average tumor volume of PBS control group) × 100% | | | | | | | | |
|---|---|---|---|---|---|---|---|---|---|
| | Day for measurement | | | | | | | | |
| | 23 | 27 | 30 | 34 | 37 | 41 | 44 | 48 | 51 |
| PBS | 0 | 0 | 0 | 0 | 0 | 0 | 0 | 0 | 0 |
| 99961.1-MMAE 10 mpk | -15.19 | 37.35 | 44.00 | 69.99 | 82.23 | 85.78 | 90.30 | 88.83 | 89.35 |
| 99961.1-MMAE 2 mpk | -5.80 | -10.51 | -9.85 | 1.17 | 14.41 | 5.62 | 13.72 | 17.82 | 15.90 |
| 99961.1-MMAE 0.4 mpk | 7.60 | 0.08 | 4.71 | -2.64 | 9.28 | 2.61 | 7.32 | 8.34 | 11.08 |
| B31-H3L3-MM AE 10 mpk | -5.56 | 17.16 | 36.56 | 59.72 | 73.42 | 83.01 | 85.35 | 85.16 | 85.53 |
| B31-H3L3-MM AE 2 mpk | -7.50 | 8.48 | 6.86 | 0.99 | 4.88 | 6.70 | 9.97 | 14.09 | 15.37 |
| B31-H3L3-MM AE 0.4 mpk | -2.50 | 8.22 | -0.94 | -5.59 | 4.75 | 9.43 | 12.34 | 17.92 | 16.05 |
| B62-H3L3-MM AE 10 mpk | 20.27 | 29.04 | 23.90 | 48.45 | 63.35 | 67.65 | 68.50 | 73.86 | 73.87 |
| B62-H3L3-MM AE 2 mpk | -1.05 | 1.92 | -2.38 | 4.20 | 17.04 | 16.26 | 23.06 | 27.19 | 33.54 |

**Example 23 Assessment of ADC inhibiting efficacy in mouse model bearing A549 tumor**

In this example, the anti-tumor effects of two candidate ADCs (B31-H3L3-MMAE and B62-H3L3-MMAE) in animals were detected. The tumor cells used were non-small cell lung cancer cells A549 (Shanghai Cell Bank, Chinese Academy of Sciences, C2107019), and 99961.1-MMAE was used as a positive control.

The specific method was as follows: 6-8 week-old female nude mice weighing about 18-20 g (Balb/C, Beijing Weitong Lihua Laboratory Animal Technology Co., Ltd.) were used, and $1\times10^6$ A549 cells were injected subcutaneously unilaterally on the right dorsal side of each nude mouse. When the tumor volume reached about 100 $mm^3$, the mice were randomly grouped into cages. Totally 4 groups were divided, 6 tumor-bearing nude mice in each group, including a PBS negative control group, 2 ADC groups (B31-H3L3-MMAE 10 mpk (mg/kg) and B62-H3L3-MMAE 10 mpk) and 1 positive control ADC group (99961.1-MMAE 10 mpk). The drugs were administered by tail vein injection, twice a week, and the tumor volume was measured twice, for a total of 6 administrations/3 weeks (BIW*3). Tumor volume (V) was calculated as follows: $V = L \times W^2/2$ (where L is the longest tumor diameter and W is the shortest tumor diameter). After a certain period of observation after the end of the drug administration, the mice were euthanized, the tumors were removed and the tumor weight was measured. The data of the tumor volume, tumor weight and mouse body weight change were analyzed, and the Tumor growth inhibition was calculated. The results are shown in Figures 19A-19C and Table 13.

The experimental results show that there is no significant change in the body weight of mice in each group during the treatment, indicating that the mice have good tolerance to the ADCs (Figure 19B); B62-H3L3-MMAE shows an anti-tumor effect comparable to that of the positive control ADC at the same dose, and B31-H3L3-MMAE shows an anti-tumor effect superior to that of the positive control ADC (Figures 19A, 19C and Table 13).

Table 13 Tumor growth inhibition of ADC in mice

| | Tumor growth inhibition (TGI (%)) = (1-average tumor volume of experimental group/average tumor volume of PBS control group) × 100% | | | |
|---|---|---|---|---|
| Day for measurement | PBS | 99961.1-MMAE 10 mpk | B31-H3L3-MMAE 10 mpk | B62-H3L3-MMAE 10 mpk |
| 27 | 0 | 13.93 | 8.75 | 18.82 |
| 31 | 0 | 24.23 | 32.35 | 33.29 |
| 34 | 0 | 25.74 | 40.25 | 30.91 |
| 38 | 0 | 32.76 | 49.82 | 40.03 |
| 41 | 0 | 43.24 | 51.16 | 37.77 |
| 45 | 0 | 40.56 | 47.39 | 35.15 |
| 48 | 0 | 37.81 | 43.98 | 36.38 |

**Example 24 Assessment of ADC inhibiting efficacy in mouse model bearing NCI-N87 tumor**

In this example, the anti-tumor effect of a candidate ADC (B31-H3L3-MMAE) in animals was detected. The tumor cells used were gastric cancer cells NCI-N87 (Shanghai Cell Bank of the Chinese Academy of Sciences, C2009021, P3), and 99961.1-MMAE was used as a positive control.

The specific method was as follows: 6-8 week-old female nude mice weighing about 18-20 g (Balb/C, Beijing Weitong Lihua Laboratory Animal Technology Co., Ltd.) were used, and $1\times10^6$ NCI-N87 cells were injected subcutaneously on the dorsal side of each nude mouse. When the tumor volume reached about 100 $mm^3$, the mice were randomly grouped into cages. Totally 5 groups were divided, 10 tumor-bearing nude mice in each group, including a PBS negative control group, 2 ADC groups (B31-H3L3-MMAE 5 mpk (mg/kg) and B31-H3L3-MMAE 10 mpk) and 2 positive control ADC groups (99961.1-MMAE 5 mpk and 99961.1-MMAE 10 mpk). The drugs were administered by tail vein injection, once a week, and the tumor volume was measured twice, for a total of 3 administrations/3 weeks (BIW*3). Tumor volume (V) was calculated as follows: $V = L \times W^2/2$ (where L is the longest tumor diameter and W is the shortest tumor diameter). The data of the tumor volume and mouse body weight change were analyzed, and the Tumor growth inhibition was calculated. The results are shown in Figures 20A-20B and Table 14.

The experimental results show that there is no significant change in the body weight of mice in each group during the treatment, indicating that the mice have good tolerance to ADC (Figure 20B); compared with the PBS group, each dose group has a significant tumor inhibition effect, and the B31-H3L3-MMAE molecule at 10 mpk has tumor inhibition effect comparable to that of 99961.1-MMAE molecule (Figure 20A and Table 14).

Table 14 Tumor growth inhibition of ADC in mice

| | Tumor growth inhibition (TGI (%)) = (1-average tumor volume of experimental group/average tumor volume of PBS control group) × 100% | | | | |
|---|---|---|---|---|---|
| Day for measurement | PBS | 99961.1-MMAE 10 mpk | 99961.1-MMAE 5 mpk | B31-H3L3-MMAE 10 mpk | B31-H3L3-MMAE 5 mpk |
| 9 | 0 | 8.56 | 11.62 | 9.34 | -1.37 |
| 13 | 0 | 70.46 | 60.38 | 70.29 | 50.63 |
| 16 | 0 | 85.05 | 76.07 | 85.24 | 66.26 |
| 20 | 0 | 93.11 | 86.75 | 93.39 | 78.22 |
| 23 | 0 | 96.15 | 91.1 | 96.64 | 83.03 |
| 27 | 0 | 97.36 | 94.75 | 98.1 | 90.91 |
| 30 | 0 | 98.27 | 95.93 | 98.59 | 91.62 |
| 34 | 0 | 98.61 | 97.67 | 98.9 | 94.93 |
| 37 | 0 | 98.87 | 97.75 | 99.04 | 93.89 |
| 41 | 0 | 99.28 | 98.28 | 99.11 | 95.15 |

(continued)

| | Tumor growth inhibition (TGI (%)) = (1-average tumor volume of experimental group/average tumor volume of PBS control group) × 100% | | | | |
|---|---|---|---|---|---|
| Day for measur ement | PBS | 99961.1-MMAE 10 mpk | 99961.1-MMAE 5 mpk | B31-H3L3-MMAE 10 mpk | B31-H3L3-MMAE 5 mpk |
| 44 | 0 | 99.40 | 98.2 | 99.31 | 94.2 |
| 48 | 0 | 99.40 | 98.09 | 99.48 | 94.68 |
| 51 | 0 | 99.20 | 97.86 | 99.31 | 91.52 |

**Example 25 Assessment of ADC inhibiting efficacy in mouse model bearing MDA-MB-231 tumor**

In this example, the anti-tumor effect of a candidate ADC (B31-H3L3-MMAE) in animals was detected. The tumor cells used were triple-negative breast cancer cells MDA-MB-231 (Shanghai Cell Bank of the Chinese Academy of Sciences, C2006040), and 99961.1-MMAE was used as a positive control.

The specific method was as follows: 6-8 week-old female nude mice weighing about 20-22 g (NSG, Beijing Weitong Lihua Laboratory Animal Technology Co., Ltd.) were used, and $1\times10^6$ MDA-MB-231 cells were injected subcutaneously on the dorsal side of each nude mouse. When the tumor volume reached about 100 $mm^3$, the mice were randomly grouped into cages. Totally 5 groups were divided, 10 tumor-bearing nude mice in each group, including a PBS negative control group, 2 ADC groups (B31-H3L3-MMAE 5 mpk (mg/kg) and B31-H3L3-MMAE 10 mpk) and 2 positive control ADC groups (99961.1-MMAE 5 mpk and 99961.1-MMAE 10 mpk). The drugs were administered by tail vein injection, once a week, and the tumor volume was measured twice, for a total of 3 administrations/3 weeks (BIW*3). Tumor volume (V) was calculated as follows: $V = L \times W^2/2$ (where L is the longest tumor diameter and W is the shortest tumor diameter). The data of the tumor volume and mouse body weight change were analyzed, and the Tumor growth inhibition was calculated. The results are shown in Figures 21A-21B and Table 15.

The rusults show that there was no significant difference in the early stage of drug administration among the mice in each group. The PBS group started to show body weight loss on day 35, which was expected to be due to tumor overloading. There is no significant change in the body weight of mice in the ADC group and the positive control group, indicating that the mice have good tolerance to ADCs (Figure 21B). Compared with the PBS group, the positive control and the test ADC high-dose groups have considerable anti-tumor effect, showing significant effect. Under low-dose condition, the test ADC (B31-H3L3-MMAE) shows an anti-tumor effect superior to that of the positive control ADC. The low-dose groups show a certain tumor tissue rebound after drug withdrawal (Figure 21A and Table 15).

Table 15 Tumor growth inhibition of ADC in mice

| | Tumor growth inhibition (TGI (%)) = (1-average tumor volume of experimental group/average tumor volume of PBS control group) × 100% | | | | |
|---|---|---|---|---|---|
| Day for measure ment | PBS | 99961.1-MMAE 10 mpk | 99961.1-MMAE 5 mpk | B31-H3L3-MMAE 10 mpk | B31-H3L3-MMAE 5 mpk |
| 21 | 0 | 27.12 | 25.14 | 29.51 | 24.01 |
| 25 | 0 | 59.78 | 41.55 | 59.33 | 50.35 |
| 28 | 0 | 76.74 | 61.45 | 74.57 | 67.87 |
| 32 | 0 | 87.51 | 73.37 | 87.43 | 79.37 |
| 35 | 0 | 91.65 | 79.35 | 91.32 | 85.76 |
| 39 | 0 | 94.82 | 84.93 | 94.55 | 89.98 |
| 42 | 0 | 95.56 | 85.01 | 95.56 | 90.53 |
| 46 | 0 | 96.13 | 79.9 | 95.87 | 88.81 |
| 49 | 0 | 96.25 | 76.4 | 96.03 | 86.62 |
| 53 | 0 | 91.58 | 31.55 | 91.85 | 58.88 |
| 56 | 0 | 96.60 | 67.66 | 96.60 | 79.79 |

**Example 26 Assessment of ADC inhibiting efficacy in mouse model bearing MDA-MB-468 tumor**

In this example, the anti-tumor effect of a candidate ADC (B31-H3L3-MMAE) in animals was detected. The tumor cells used were triple-negative breast cancer cells MDA-MB-468 (Shanghai Cell Bank of the Chinese Academy of Sciences, TCHu136), and 99961.1-MMAE was used as a positive control.

The specific method was as follows: 6-8 week-old female nude mice weighing about 21-25 g (NOD SCID, Zhejiang Weitong Lihua Laboratory Animal Technology Co., Ltd.) were used, and $1\times10^7$ MDA-MB-468 cells were injected subcutaneously on the dorsal side of each nude mouse. When the tumor volume reached about 200 $mm^3$, the mice were randomly grouped into cages. Totally 5 groups were divided, 6 tumor-bearing nude mice in each group, including a PBS negative control group, 2 ADC groups (B31-H3L3-MMAE 5 mpk (mg/kg) (QW*3), B31-H3L3-MMAE 10 mpk (QW*3) and 2 positive control ADC groups (99961.1-MMAE 5 mpk (QW*3), 99961.1-MMAE 10 mpk (QW*3); totally 2 groups with 3 tumor-bearing nude mice in each group, including B31-H3L3-MMAE 10 mpk (single dose) and 99961.1-MMAE 10 mpk (single dose), and the administration method was tail vein injection, for a total of 3 administrations/3 weeks (QW*3) or single dose. Tumor volume (V) was calculated as follows: $V = L \times W^2/2$ (where L is the longest tumor diameter and W is the shortest tumor diameter). The data of the tumor volume and mouse body weight change were analyzed, and the Tumor growth inhibition was calculated. The results are shown in Figures 22A-22B and Tables 16-17.

The experimental results show that there is no significant change in the body weight of mice in each group during the treatment period, indicating that the mice have good tolerance to ADCs (Figure 22B); The Tumor growth inhibition of the group administered with 10 mpk B31-H3L3-MMAE by QW*3 is slightly higher than that of the group administered with 10 mpk 99961.1-MMAE by QW*3 (Table 17), but all mice achieve complete remission (CR) approximately 18 days after administration; the tumor inhibition effect of the group administered with 5 mg/kg B31-H3L3-MMAE by QW*3 is superior to that of the group administered with 5 mg/kg 99961.1-MMAE by QW*3, and 4/6 mice achieve CR on day 25 after administration. The single dose 10 mg/kg B31-H3L3-MMAE group shows a superior tumor inhibition effect compared to the single dose 10 mg/kg 99961.1-MMAE group, with the B31-H3L3-MMAE group achieving CR in all mice by 18 days post-administration, while the 99961.1-MMAE group experienced tumor recurrence by 20 days post-administration. In this experiment, tumor recurrence was not observed in all B31-H3L3-MMAE groups ( figure 22A and Tables 16-17).

Table 16 Tumor growth inhibition of ADC in mice

| | Tumor growth inhibition (TGI (%)) = (1-average tumor volume of experimental group/average tumor volume ofPBS control group) × 100% | | | | | | |
|---|---|---|---|---|---|---|---|
| Day for measurement | PBS | 99961.1-MMAE 10 mpk, QW*3 | 99961.1-MMAE 5 mpk, QW*3 | B31-MMAE 10 mpk, QW*3 | B31-MMAE 5 mpk, QW*3 | 99961.1-MMAE 10 mpk, Single dose | B31-MMAE 10 mpk, Single dose |
| 35 | 0 | 38.24 | 38.52 | 46.53 | 38.37 | 21.17 | 29.08 |
| 38 | 0 | 60.49 | 56.66 | 70.18 | 52.31 | 45.57 | 50.62 |
| 42 | 0 | 84.96 | 68.58 | 91.94 | 74.83 | 74.09 | 84.41 |
| 45 | 0 | 94.6 | 84.17 | 99.44 | 84.77 | 78.07 | 94.13 |
| 49 | 0 | 100 | 92.84 | 100 | 93.52 | 77.26 | 100 |
| 51 | 0 | 100 | 96.69 | 100 | 96.83 | 74.59 | 100 |
| 56 | 0 | 100 | 98.21 | 100 | 98.02 | 64.31 | 100 |

Table 17 Complete remission rate of ADC in mice

| | Complete remission (CR (%)) | | | | | | |
|---|---|---|---|---|---|---|---|
| Day for measurement | PBS | 99961.1-MMAE 10 mpk, QW*3 | 99961.1-MMAE 5 mpk, QW*3 | B31-MMAE 10 mpk, QW*3 | B31-MMAE 5 mpk, QW*3 | 99961.1-MMAE 10 mpk, Single dose | B31-MMAE 10 mpk, Single dose |
| 38 | 0 | - | - | - | - | - | - |
| 42 | 0 | - | - | 1/6 | - | - | - |
| 45 | 0 | 1/6 | - | 5/6 | - | - | 1/3 |

(continued)

| | Complete remission (CR (%)) | | | | | | |
|---|---|---|---|---|---|---|---|
| Day for measurement | PBS | 99961.1-MMAE 10 mpk, QW*3 | 99961.1-MMAE 5 mpk, QW*3 | B31-MMAE 10 mpk, QW*3 | B31-MMAE 5 mpk, QW*3 | 99961.1-MMAE 10 mpk, Single dose | B31-MMAE 10 mpk, Single dose |
| 49 | 0 | 6/6 | 1/6 | 6/6 | 2/6 | - | 3/3 |
| 51 | 0 | 6/6 | 4/6 | 6/6 | 4/6 | - | 3/3 |
| 56 | 0 | 6/6 | 6/6 | 6/6 | 4/6 | - | 3/3 |

**Example 27 Assessment of ADC inhibiting efficacy in mouse model bearing Jeko-1 tumor**

In this example, the anti-tumor effect of a candidate ADC (B31-H3L3-MMAE) in animals was detected. The tumor cells used were mantle cell lymphoma Jeko-1 cells (ATCC, CRL-3006), and 99961.1-MMAE was used as a positive control.

The specific method was as follows: 6-8 week-old female nude mice weighing about 21-25 g (BALB/c, Zhejiang Weitong Lihua Laboratory Animal Technology Co., Ltd.) were used, and $1\times10^7$ Jeko-1 cells were injected subcutaneously on the dorsal side of each nude mouse. When the tumor volume reached about 100 $mm^3$, the mice were randomly grouped into cages. Totally 9 groups were divided, 7 tumor-bearing nude mice per group, including a PBS negative control group, 4 ADC groups (B31-H3L3-MMAE 2.5 mpk (mg/kg) (QW*3), B31-H3L3-MMAE 10 mpk (QW*3), B31-H3L3-MMAE 10 mpk (single dose) and B31-H3L3-MMAE 2.5+3.5 mpk (Q2W*2, *i.e.,* 2.5 mpk on day 15 and 3.5 mpk on day 29, abbreviated as D15 2.5 mpk+D29 3.5 mpk)) and 4 positive control ADC groups (99961.1-MMAE 2.5 mpk (QW*3), 99961.1-MMAE 10 mpk (QW*3), 99961.1-MMAE 10 mpk (single dose) and 99961.1-MMAE 2.5+3.5 mpk (Q2W*2, *i.e.* 2.5 mpk on day 15 and 3.5 mpk on day 29, abbreviated as D15 2.5 mpk+D29 3.5 mpk)), the administration method was tail vein injection, a total of 3 administrations/3 weeks (QW*3) or single dose (single dose) or 2 administrations/3 weeks (Q2W*2). Tumor volume (V) was calculated as follows: $V = L \times W^2/2$ (where L is the longest tumor diameter and W is the shortest tumor diameter). The data of the tumor volume and mouse body weight change were analyzed, and the Tumor growth inhibition was calculated. The results are shown in Figures 23A-23B and Tables 18-19.

The experimental results show that there is no significant change in the body weight of mice in each group during the treatment, indicating that the mice have good tolerance to ADC (Figure 23B); mice in the QW*3 administration groups, B31-H3L3-MMAE-10mpk and 99961.1-MMAE-10mpk dosage groups have equivalent tumor growth inhibition rate TGI of about 96%, and the tumor recurrence was not observed 23 days after administration; the B31-H3L3-MMAE-2.5mpk and 99961.1-MMAE-2.5mpk dosage groups have equivalent anti-tumor effect, with TGI of 60.47% and 59.22%, respectively. In the single-dose groups, B31-H3L3-MMAE (10 mpk) and 99961.1-MMAE (10 mpk) dosage groups have TGI of 79.24% and 91.74%, respectively, and no tumor recurrence was observed 23 days after administration. In the Q2W*2 dosing groups, the B31-H3L3-MMAE (2.5+3.5 mpk) dosage group has the tumor inhibition effect comparable to that of the 99961.1-MMAE (2.5+3.5 mpk) group, with TGI of 34.96% and 34.60%, respectively (figure 23A and Tables 18-19).

Table 18 Tumor growth inhibition of ADC in mice

| | Tumor growth inhibition (TGI (%)) = (1-average tumor volume of experimental group/average tumor volume of PBS control group) × 100% | | | | |
|---|---|---|---|---|---|
| Day for mea sure men t | PBS | 99961.1-MMAE 10 mpk, QW*3 | 99961.1-MMAE 2.5 mpk, QW*3 | B31-H3L3-MMAE 10 mpk, QW*3 | B31-H3L3-MMAE 2.5 mpk, QW*3 |
| 17 | 0 | 2.81 | 2.04 | 3.63 | 6.14 |
| 20 | 0 | 40.69 | 15.40 | 26.96 | 13.78 |
| 22 | 0 | 62.44 | 10.31 | 52.36 | 10.83 |
| 24 | 0 | 69.94 | 4.20 | 61.53 | 20.13 |
| 27 | 0 | 86.34 | 29.20 | 83.08 | 40.94 |
| 29 | 0 | 90.97 | 44.98 | 89.03 | 47.48 |
| 31 | 0 | 94.42 | 53.01 | 94.30 | 53.56 |
| 34 | 0 | 95.57 | 55.18 | 96.02 | 58.44 |

(continued)

| | Tumor growth inhibition (TGI (%)) = (1-average tumor volume of experimental group/average tumor volume of PBS control group) × 100% | | | | |
|---|---|---|---|---|---|
| Day for mea sure men t | PBS | 99961.1-MMAE 10 mpk, QW*3 | 99961.1-MMAE 2.5 mpk, QW*3 | B31-H3L3-MMAE 10 mpk, QW*3 | B31-H3L3-MMAE 2.5 mpk, QW*3 |
| 36 | 0 | 96.24 | 58.47 | 95.68 | 61.76 |
| 38 | 0 | 95.38 | 60.82 | 96.28 | 64.21 |
| 41 | 0 | 95.77 | 59.22 | 95.44 | 60.47 |

Table 19 Tumor growth inhibition of ADC in mice

| | Tumor growth inhibition (TGI (%)) = (1-average tumor volume of experimental group/average tumor volume ofPBS control group) × 100% | | | | |
|---|---|---|---|---|---|
| Day for measur ement | PBS | 99961.1-MMAE 10 mpk, Single dose | 99961.1-MMAE 2.5(D15)/3.5(D29 ) mpk, Q2W*2 | B31-H3L3-MMAE 10 mpk, Single dose | B31-H3L3-MMAE 2.5(D15)/3.5(D29) mpk, Q2W*2 |
| 17 | 0 | 5.32 | 2.85 | 6.54 | 4.77 |
| 20 | 0 | 44.15 | 16.28 | 28.99 | 18.97 |
| 22 | 0 | 61.50 | 9.92 | 45.06 | 7.83 |
| 24 | 0 | 70.14 | 5.65 | 59.45 | 4.25 |
| 27 | 0 | 82.01 | 6.58 | 74.96 | 13.01 |
| 29 | 0 | 90.01 | 7.92 | 86.03 | 9.77 |
| 31 | 0 | 91.95 | 13.64 | 90.48 | 11.05 |
| 34 | 0 | 93.67 | 18.47 | 89.15 | 24.86 |
| 36 | 0 | 92.85 | 18.18 | 86.44 | 36.94 |
| 38 | 0 | 92.51 | 32.36 | 84.62 | 42.42 |
| 41 | 0 | 91.74 | 34.60 | 79.24 | 34.96 |

Sequence Listing

| SEQ ID NO: | Sequence description | Sequence information |
|---|---|---|
| 51 | human IgG1 Fc | EPKSCDKTHTCPPCPAPELLGGPSVFLFPPKPKDTLMISRTPEVTCV VVDVSHEDPEVKFNWYVDGVEVHNAKTKPREEQYNSTYRVVSVL TVLHQDWLNGKEYKCKVSNKALPAPIEKTISKAKGQPREPQVYTL PPSRDELTKNQVSLTCLVKGFYPSDIAVEWESNGQPENNYKTTPPV LDSDGSFFLYSKLTVDKSRWQQGNVFSCSVMHEALHNHYTQKSLS LSPGK |

(continued)

| SEQ ID NO: | Sequence description | Sequence information |
|---|---|---|
| 52 | hIgG1 heavy chain constant region | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 53 | CL (KAPPA) | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 54 | B31 VL | DIQMNQSPSSMYASLGERVTITCKASQDINNYVSWFQQKPGKSPKTLIYRANRLVDGVPSRFSGSGSGQDYSFTISSLEYEDMGIYYCLQFDEFPYTFGGGTKLEIK |
| 55 | B31 VH | EVMLVESGGGLVKPGGSLKLSCAASGFTFSRYAMSWVRQTPEKRLEWVASISRGGSTYYPDSVKGRFTISRDNARNILYLQMSSLRSEDTAMYYCASYFGNYDAMDYWGQGTSVTVSS |
| 56 | B62 VL | DIVLTQSPASLSMAIGEKVTIRCVTSIDIDDDVNWYQQKPGKPPKLLISAGNTLRPGVPSRFSSSGYGTDFVFTIENMLSEDVADYYCLQSDNLPYTFGGGTKLEIK |
| 57 | B62 VH | DVKLVESGGGLVQPGGSLKLSCAASGFTFSSYVMSWVRQTPDKRLELVATINSNGGNTYYPDSVKGRFTISRDNAKNTLYLQMSSLKSEDTAMYYCARDSLGLRRRDYAMDYWGQGTSVTVSS |
| 58 | B32 VL | DIQITQSPSSMYASLGERVTITCKASQDINSYLTWFQQKPGKSPKTLIYRANRLVDGVPSRFSGSGSGQDYSLTISSLEYEDMGIYYCLQFDEFPYTFGGGTKLEIK |
| 59 | B32 VH | EVKLMESGGGLVKPGGSLKLSCAASGFAFSSYAMSWVRQTPDKRLEWVASINRDVTTYYPDSMKGRFTISRDNARNILYLQMSSLRSEDTAMYYCARGYYYGGGPYWHFDVWGAGTTVTVSS |
| 60 | B74 VL | DIKMTQSPSSMYASLGERVTITCKASQDINSYLSWFQQKPGKSPKTLIYRANRLVDGVPSRFSGSGSGQDYSLTISSLEYEDIGIYYCLQIDEFPYTFGGGTKLEIK |

(continued)

| SEQ ID NO: | Sequence description | Sequence information |
|---|---|---|
| 61 | B74 VH | EVMLVESGGNLVKPGGSLKLSCAASGFTFSIYAMSWVRQTPEKRLEWVASISGGGTAYYPDSVKGRFTISRDNARNILYLQMSSLRSEDTAIYYCARGYYYGGSPYWHFDVWGAGTTVTVSS |
| 62 | B34 VL | DIQMTQSPSSLSASLGERVTITCKASQDINSYLSWFQQKPGKSPKTLIYRANRLVDGVPSRFSGSGSGQDYSLTISSLEYEDMGIYYCLQYDEFPYTFGGGTKLEIK |
| 63 | B34 VH | EVQLVESGGGLVQPKGSLKLSCAASGFTFSRYAMSWVRQTPEKRLEWVASISTGGSPYYPDSVKGRFTISRDNARNILYLQMSSLKSEDTAMYYCARGYYYNSSPHWYFAVWGAGTSVTVSS |
| 64 | B39 VL | DIKITQSPSSMYASLGERVTITCKASQDINNYLSWFQQKPGKSPKTLIYRANRLVDGVPSRFSGSGSGQDYSLTISSLEYEDMGIYYCLQFDEFPYTFGGGTKLEIK |
| 65 | B39 VH | EVKLVESGGGLVKPGGSLKLSCAASGFTFSTYAMSWVRQTPEKRLEWVASISTGASTYYPDSVKGRFTISRDNARNILYLQMSSLRSEDTAMYYCAREETTTAALYYAMDYWGQGTSVTVSS |
| 66 | C38, C77 VL | DIQMTQSPSSMHASLGERVTITCKASQDINNYVSWFQQKPGKSPKTLIYRANRLVDGVPSRFSGSGSGQDYSFTISSLEYEDMGIYYCLQFDEFPYTFGGGTKLEIK |
| 67 | C38 VH | EVMLVESGGGLVRPGGSLKLSCAASGFTFSSYAMSWVRQTPDKRLEWVASINRDVTTYYPDSVKGRFTISRDNARNILYLQMSSLRSEDTAMYYCARDYYYGSSLYYGMDYWGQGTSVTVSS |
| 68 | C77 VH | EVKLVESGGGLVKPGGSLKLSCAASGFSFSNYAMSWVRQTPEKRLEWVASINSGVTTYYPDSVKGRFTVSRDNARNILYLHMTGLRSEDTAIYYCSRGYYYGGSPYWHFDVWGAGTTVTVSS |
| 69 | C42 VL | DIVLTQSPAIMSASPGEKVTMTCSASSSVSFIYWYQQKPGSSPRLLIYDTSNLASGVPVRFSGSGSGTSYSLTISRMEAEDAATYYCQQWSNYHFTFGSGTKLEIK |
| 70 | C42 VH | EVMLVESGGGLVKPGGSLKLSCAASGFTFSNYIMSWVRQTPEKRLEWVATINSGGTNTYYPDSVKGRFTISRDNAKNSLYLQMNSLRSEDTALYYCTRYNGNYVWFAYWGQGTLVTVSA |
| 71 | M71 VL | DIQMTQSPSSMYASLGERVTITCKASQDINSYLSWFQQKPGKSPKTLIYRANRLVDGVPSRFSGSGSGQDYSLTISSLESEDMGIYYCLQFDEFPYTFGGGTKLEIK |

(continued)

| SEQ ID NO: | Sequence description | Sequence information |
|---|---|---|
| 72 | M71 VH | EVMLVESGGGLVKPGGSLKLSCAASGFTFSRYAMSWVRQTPEKRLEWVASINRGGTTYYPDSVKGRFTISRDNARDILYLQMSSLRSEDTAIYYCARDYYYGSSLYNGMDFWGQGTSVIVSS |
| 73 | M78 VL | DIKITQSPSSMYASLGERVTITCKASQDINSYLSWFQQKPGKSPKTLIYRANRLVDGVPSRFSGSGSGQDYSLTISSLEYEDMGIYYCLQFDEFPYTFGGGTKLEIK |
| 74 | M78 VH | EVMLVESGGGLVRPGGSLKLSCAASGFTFSSYAMSWVRQSPEKRLEWVASINRGGTTYYPDSVKGRFTISRDNARDILYLQMSSLRSEDTAIYYCARDYYYGSNLYNGMDYWGQGTSVIVSS |
| 75 | B31-H1L1 VH | EVQLVESGGGLVQPGGSLRLSCAASGFTFSRYAMSWVRQTPGKRLEWVASISRGGSTYYPDSVKGRFTISRDNAKNTLYLQMSSLRAEDTAMYYCASYFGNYDAMDYWGQGTTVTVSS |
| 76 | B31-H2L2, B31-H2L3 VH | EVQLVESGGGLVQPGGSLRLSCAASGFTFSRYAMSWVRQAPGKGLEWVASISRGGSTYYADSVKGRFTISRDNAKNTLYLQMSSLRAEDTAVYYCASYFGNYDAMDYWGQGTTVTVSS |
| 77 | B31-H3L2, B31-H3L3, B31-H3L4 VH | EVQLVESGGGLVQPGGSLRLSCAASGFTFSRYAMSWVRQAPGKGLEWVASISRGGSTYYADSVKGRFTISRDNSKNTLYLQMNSLRAEDTAVYYCASYFGNYDAMDYWGQGTTVTVSS |
| 78 | B31-H4L2, B31-H4L3, B31-H4L4 VH | EVQLVESGGGLVQPGGSLRLSCAASGFTFSRYAMSWVRQAPGKGLEWVSAISRGGSTYYADSVKGRFTISRDNSKNTLYLQMNSLRAEDTAVYYCASYFGNYDAMDYWGQGTTVTVSS |
| 79 | B31-H1L1 VL | DIQMTQSPSSLSASVGDRVTITCKASQDINNYVSWFQQKPGKAPKTLIYRANRLVDGVPSRFSGSGSGQDYTFTISSLQPEDFATYYCLQFDEFPYTFGGGTKLEIK |
| 80 | B31-H2L2, B31-H3L2, B31-H4L2 VL | DIQMTQSPSSLSASVGDRVTITCKASQDINNYVSWFQQKPGKAPKSLIYRANRLVDGVPSRFSGSGSGTDYTFTISSLQPEDFATYYCLQFDEFPYTFGGGTKLEIK |
| 81 | B31-H2L3, B31-H3L3, B31-H4L3 VL | DIQMTQSPSSLSASVGDRVTITCRASQDINNYVSWFQQKPGKAPKSLIYRANRLVSGVPSRFSGSGSGTDFTFTISSLQPEDFATYYCLQFDEFPYTFGGGTKLEIK |
| 82 | B31-H3L4, B31-H4L4 VL | DIQMTQSPSSLSASVGDRVTITCRASQDINNYLSWFQQKPGKAPKSLIYRANRLQSGVPSRFSGSGSGTDFTLTISSLQPEDFATYYCLQFDEFPYTFGGGTKLEIK |

(continued)

| SEQ ID NO: | Sequence description | Sequence information |
|---|---|---|
| 83 | B62-H1L1, B62-H1L2 VH | EVQLVESGGGLVQPGGSLRLSCAASGFTFSSYVMSWVRQTPGKRLELVATINSNGGNTYYPDSVKGRFTISRDNAKNTLYLQMSSLRAEDTAMYYCARDSLGLRRRDYAMDYWGQGTTVTVSS |
| 84 | B62-H2L2, B62-H2L3, B62-H2L4 VH | EVQLVESGGGLVQPGGSLRLSCAASGFTFSSYVMSWVRQAPGKGLELVATINSNGGNTYYPDSVKGRFTISRDNAKNTLYLQMGSLRAEDTAMYYCARDSLGLRRRDYAMDYWGQGTTVTVSS |
| 85 | B62-H3L2, B62-H3L3, B62-H3L4 VH | EVQLVESGGGLVQPGGSLRLSCAASGFTFSSYVMSWVRQAPGKGLEYVATINSNGGNTYYADSVKGRFTISRDNSKNTLYLQMGSLRAEDTAMYYCARDSLGLRRRDYAMDYWGQGTTVTVSS |
| 86 | B62-H1L1 VL | DIQLTQSPSSLSASVGDRVTIRCVTSIDIDDDVNWYQQKPGKAPKLLISAGNTLRPGVPSRFSSSGYGTDFTFTISSMQPEDIATYYCLQSDNLPYTFGGGTKLEIK |
| 87 | B62-H1L2, B62-H2L2, B62-H3L2 VL | DIQLTQSPSSLSASVGDRVTIRCVTSIDIDDDVNWYQQKPGKAPKLLISAGNTLRPGVPSRFSGSGSGTDFTFTISSLQPEDIATYYCLQSDNLPYTFGGGTKLEIK |
| 88 | B62-H2L3, B62-H3L3 VL | DIQMTQSPSSLSASVGDRVTITCVTSIDIDDDVNWYQQKPGKAPKLLISAGNTLRPGVPSRFSGSGSGTDFTFTISSLQPEDIATYYCLQSDNLPYTFGGGTKLEIK |
| 89 | B62-H2L4, B62-H3L4 VL | DIQMTQSPSSLSASVGDRVTITCQTSIDIDDDVNWYQQKPGKAPKLLISAGNTLETGVPSRFSGSGSGTDFTFTISSLQPEDIATYYCLQSDNLPYTFGGGTKLEIK |
| 90 | B31 light chain | DIQMNQSPSSMYASLGERVTITCKASQDINNYVSWFQQKPGKSPKTLIYRANRLVDGVPSRFSGSGSGQDYSFTISSLEYEDMGIYYCLQFDEFPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| SEQ ID NO: | Sequence description | Sequence information |
|---|---|---|
| 91 | B31 heavy chain | EVMLVESGGGLVKPGGSLKLSCAASGFTFSRYAMSWVRQTPEKRLEWVASISRGGSTYYPDSVKGRFTISRDNARNILYLQMSSLRSEDTAMYYCASYFGNYDAMDYWGQGTSVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 92 | B62 light chain | DIVLTQSPASLSMAIGEKVTIRCVTSIDIDDDVNWYQQKPGKPPKLLISAGNTLRPGVPSRFSSSGYGTDFVFTIENMLSEDVADYYCLQSDNLPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 93 | B62 heavy chain | DVKLVESGGGLVQPGGSLKLSCAASGFTFSSYVMSWVRQTPDKRLELVATINSNGGNTYYPDSVKGRFTISRDNAKNTLYLQMSSLKSEDTAMYYCARDSLGLRRRDYAMDYWGQGTSVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 94 | B32 light chain | DIQITQSPSSMYASLGERVTITCKASQDINSYLTWFQQKPGKSPKTLIYRANRLVDGVPSRFSGSGSGQDYSLTISSLEYEDMGIYYCLQFDEFPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| SEQ ID NO: | Sequence description | Sequence information |
|---|---|---|
| 95 | B32 heavy chain | EVKLMESGGGLVKPGGSLKLSCAASGFAFSSYAMSWVRQTPDKRLEWVASINRDVTTYYPDSMKGRFTISRDNARNILYLQMSSLRSEDTAMYYCARGYYYGGGPYWHFDVWGAGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 96 | B74 light chain | DIKMTQSPSSMYASLGERVTITCKASQDINSYLSWFQQKPGKSPKTLIYRANRLVDGVPSRFSGSGSGQDYSLTISSLEYEDIGIYYCLQIDEFPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 97 | B74 heavy chain | EVMLVESGGNLVKPGGSLKLSCAASGFTFSIYAMSWVRQTPEKRLEWVASISGGGTAYYPDSVKGRFTISRDNARNILYLQMSSLRSEDTAIYYCARGYYYGGSPYWHFDVWGAGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 98 | B34 light chain | DIQMTQSPSSLSASLGERVTITCKASQDINSYLSWFQQKPGKSPKTLIYRANRLVDGVPSRFSGSGSGQDYSLTISSLEYEDMGIYYCLQYDEFPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| SEQ ID NO: | Sequence description | Sequence information |
|---|---|---|
| 99 | B34 heavy chain | EVQLVESGGGLVQPKGSLKLSCAASGFTFSRYAMSWVRQTPEKRLEWVASISTGGSPYYPDSVKGRFTISRDNARNILYLQMSSLKSEDTAMYYCARGYYYNSSPHWYFAVWGAGTSVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 100 | B39 light chain | DIKITQSPSSMYASLGERVTITCKASQDINNYLSWFQQKPGKSPKTLIYRANRLVDGVPSRFSGSGSGQDYSLTISSLEYEDMGIYYCLQFDEFPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 101 | B39 heavy chain | EVKLVESGGGLVKPGGSLKLSCAASGFTFSTYAMSWVRQTPEKRLEWVASISTGASTYYPDSVKGRFTISRDNARNILYLQMSSLRSEDTAMYYCAREETTTAALYYAMDYWGQGTSVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 102 | C38, C77 light chain | DIQMTQSPSSMHASLGERVTITCKASQDINNYVSWFQQKPGKSPKTLIYRANRLVDGVPSRFSGSGSGQDYSFTISSLEYEDMGIYYCLQFDEFPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| SEQ ID NO: | Sequence description | Sequence information |
|---|---|---|
| 103 | C38 heavy chain | EVMLVESGGGLVRPGGSLKLSCAASGFTFSSYAMSWVRQTPDKRLEWVASINRDVTTYYPDSVKGRFTISRDNARNILYLQMSSLRSEDTAMYYCARDYYYGSSLYYGMDYWGQGTSVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 104 | C77 heavy chain | EVKLVESGGGLVKPGGSLKLSCAASGFSFSNYAMSWVRQTPEKRLEWVASINSGVTTYYPDSVKGRFTVSRDNARNILYLHMTGLRSEDTAIYYCSRGYYYGGSPYWHFDVWGAGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 105 | C42 light chain | DIVLTQSPAIMSASPGEKVTMTCSASSSVSFIYWYQQKPGSSPRLLIYDTSNLASGVPVRFSGSGSGTSYSLTISRMEAEDAATYYCQQWSNYHFTFGSGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 106 | C42 heavy chain | EVMLVESGGGLVKPGGSLKLSCAASGFTFSNYIMSWVRQTPEKRLEWVATINSGGTNTYYPDSVKGRFTISRDNAKNSLYLQMNSLRSEDTALYYCTRYNGNYVWFAYWGQGTLVTVSAASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO: | Sequence description | Sequence information |
|---|---|---|
| 107 | M71 light chain | DIQMTQSPSSMYASLGERVTITCKASQDINSYLSWFQQKPGKSPKTLIYRANRLVDGVPSRFSGSGSGQDYSLTISSLESEDMGIYYCLQFDEFPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 108 | M71 heavy chain | EVMLVESGGGLVKPGGSLKLSCAASGFTFSRYAMSWVRQTPEKRLEWVASINRGGTTYYPDSVKGRFTISRDNARDILYLQMSSLRSEDTAIYYCARDYYYGSSLYNGMDFWGQGTSVIVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 109 | M78 light chain | DIKITQSPSSMYASLGERVTITCKASQDINSYLSWFQQKPGKSPKTLIYRANRLVDGVPSRFSGSGSGQDYSLTISSLEYEDMGIYYCLQFDEFPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 110 | M78 heavy chain | EVMLVESGGGLVRPGGSLKLSCAASGFTFSSYAMSWVRQSPEKRLEWVASINRGGTTYYPDSVKGRFTISRDNARDILYLQMSSLRSEDTAIYYCARDYYYGSNLYNGMDYWGQGTSVIVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO: | Sequence description | Sequence information |
|---|---|---|
| 111 | B31-H1L1 heavy chain | EVQLVESGGGLVQPGGSLRLSCAASGFTFSRYAMSWVRQTPGKRLEWVASISRGGSTYYPDSVKGRFTISRDNAKNTLYLQMSSLRAEDTAMYYCASYFGNYDAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 112 | B31-H2L2, B31-H2L3 heavy chain | EVQLVESGGGLVQPGGSLRLSCAASGFTFSRYAMSWVRQAPGKGLEWVASISRGGSTYYADSVKGRFTISRDNAKNTLYLQMSSLRAEDTAVYYCASYFGNYDAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 113 | B31-H3L2, B31-H3L3, B31-H3L4 heavy chain | EVQLVESGGGLVQPGGSLRLSCAASGFTFSRYAMSWVRQAPGKGLEWVASISRGGSTYYADSVKGRFTISRDNSKNTLYLQMNSLRAEDTAVYYCASYFGNYDAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| SEQ ID NO: | Sequence description | Sequence information |
|---|---|---|
| 114 | B31-H4L2, B31-H4L3, B31-H4L4 heavy chain | EVQLVESGGGLVQPGGSLRLSCAASGFTFSRYAMSWVRQAPGKGL EWVSAISRGGSTYYADSVKGRFTISRDNSKNTLYLQMNSLRAEDT AVYYCASYFGNYDAMDYWGQGTTVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALHNHYTQKSLSLSPGK |
| 115 | B31-H1L1 light chain | DIQMTQSPSSLSASVGDRVTITCKASQDINNYVSWFQQKPGKAPKT LIYRANRLVDGVPSRFSGSGSGQDYTFTISSLQPEDFATYYCLQFDE FPYTFGGGTKLEIKRTVAAPSVFIFPPSDEQLKSGTASVVCLLNNFY PREAKVQWKVDNALQSGNSQESVTEQDSKDSTYSLSSTLTLSKAD YEKHKVYACEVTHQGLSSPVTKSFNRGEC |
| 116 | B31-H2L2, B31-H3L2, B31-H4L2 light chain | DIQMTQSPSSLSASVGDRVTITCKASQDINNYVSWFQQKPGKAPKS LIYRANRLVDGVPSRFSGSGSGTDYTFTISSLQPEDFATYYCLQFDE FPYTFGGGTKLEIKRTVAAPSVFIFPPSDEQLKSGTASVVCLLNNFY PREAKVQWKVDNALQSGNSQESVTEQDSKDSTYSLSSTLTLSKAD YEKHKVYACEVTHQGLSSPVTKSFNRGEC |
| 117 | B31-H2L3, B31-H3L3, B31-H4L3 light chain | DIQMTQSPSSLSASVGDRVTITCRASQDINNYVSWFQQKPGKAPKS LIYRANRLVSGVPSRFSGSGSGTDFTFTISSLQPEDFATYYCLQFDEF PYTFGGGTKLEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| 118 | B31-H3L4, B31-H4L4 light chain | DIQMTQSPSSLSASVGDRVTITCRASQDINNYLSWFQQKPGKAPKS LIYRANRLQSGVPSRFSGSGSGTDFTLTISSLQPEDFATYYCLQFDEF PYTFGGGTKLEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |

(continued)

| SEQ ID NO: | Sequence description | Sequence information |
|---|---|---|
| 119 | B62-H1L1, B62-H1L2 heavy chain | EVQLVESGGGLVQPGGSLRLSCAASGFTFSSYVMSWVRQTPGKRLELVATINSNGGNTYYPDSVKGRFTISRDNAKNTLYLQMSSLRAEDTAMYYCARDSLGLRRRDYAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 120 | B62-H2L2, B62-H2L3, B62-H2L4 heavy chain | EVQLVESGGGLVQPGGSLRLSCAASGFTFSSYVMSWVRQAPGKGLELVATINSNGGNTYYPDSVKGRFTISRDNAKNTLYLQMGSLRAEDTAMYYCARDSLGLRRRDYAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 121 | B62-H3L2, B62-H3L3, B62-H3L4 heavy chain | EVQLVESGGGLVQPGGSLRLSCAASGFTFSSYVMSWVRQAPGKGLEYVATINSNGGNTYYADSVKGRFTISRDNSKNTLYLQMGSLRAEDTAMYYCARDSLGLRRRDYAMDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 122 | B62-H1L1 light chain | DIQLTQSPSSLSASVGDRVTIRCVTSIDIDDDVNWYQQKPGKAPKLLISAGNTLRPGVPSRFSSSGYGTDFTFTISSMQPEDIATYYCLQSDNLPYTFGGGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |

(continued)

| SEQ ID NO: | Sequence description | Sequence information |
|---|---|---|
| 123 | B62-H1L2, B62-H2L2, B62-H3L2 light chain | DIQLTQSPSSLSASVGDRVTIRCVTSIDIDDDVNWYQQKPGKAPKL LISAGNTLRPGVPSRFSGSGSGTDFTFTISSLQPEDIATYYCLQSDNL PYTFGGGTKLEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| 124 | B62-H2L3, B62-H3L3 light chain | DIQMTQSPSSLSASVGDRVTITCVTSIDIDDDVNWYQQKPGKAPKL LISAGNTLRPGVPSRFSGSGSGTDFTFTISSLQPEDIATYYCLQSDNL PYTFGGGTKLEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |
| 125 | B62-H2L4, B62-H3L4 light chain | DIQMTQSPSSLSASVGDRVTITCQTSIDIDDDVNWYQQKPGKAPKL LISAGNTLETGVPSRFSGSGSGTDFTFTISSLQPEDIATYYCLQSDNL PYTFGGGTKLEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKSFNRGEC |

## Claims

**1.** An anti-ROR1 antibody specifically binding to ROR1 and antigen-binding fragment thereof, comprising:

1) three heavy chain CDRs (HCDR1, HCDR2, HCDR3) comprised in the heavy chain variable region as shown in SEQ ID NO: 57 and three light chain CDRs (LCDR1, LCDR2, LCDR3) comprised in the light chain variable region as shown in SEQ ID NO: 56;
2) three heavy chain CDRs (HCDR1, HCDR2, HCDR3) comprised in the heavy chain variable region as shown in SEQ ID NO: 55 and three light chain CDRs (LCDR1, LCDR2, LCDR3) comprised in the light chain variable region as shown in SEQ ID NO: 54;
3) three heavy chain CDRs (HCDR1, HCDR2, HCDR3) comprised in the heavy chain variable region as shown in SEQ ID NO: 59 and three light chain CDRs (LCDR1, LCDR2, LCDR3) comprised in the light chain variable region as shown in SEQ ID NO: 58;
4) three heavy chain CDRs (HCDR1, HCDR2, HCDR3) comprised in the heavy chain variable region as shown in SEQ ID NO: 61 and three light chain CDRs (LCDR1, LCDR2, LCDR3) comprised in the light chain variable region as shown in SEQ ID NO: 60;
5) three heavy chain CDRs (HCDR1, HCDR2, HCDR3) comprised in the heavy chain variable region as shown in SEQ ID NO: 63 and three light chain CDRs (LCDR1, LCDR2, LCDR3) comprised in the light chain variable region as shown in SEQ ID NO: 62;
6) three heavy chain CDRs (HCDR1, HCDR2, HCDR3) comprised in the heavy chain variable region as shown in SEQ ID NO: 65 and three light chain CDRs (LCDR1, LCDR2, LCDR3) comprised in the light chain variable region as shown in SEQ ID NO: 64;
7) three heavy chain CDRs (HCDR1, HCDR2, HCDR3) comprised in the heavy chain variable region as shown in SEQ ID NO: 67 and three light chain CDRs (LCDR1, LCDR2, LCDR3) comprised in the light chain variable region as shown in SEQ ID NO: 66;
8) three heavy chain CDRs (HCDR1, HCDR2, HCDR3) comprised in the heavy chain variable region as shown in SEQ ID NO: 68 and three light chain CDRs (LCDR1, LCDR2, LCDR3) comprised in the light chain variable region as shown in SEQ ID NO: 66;

9) three heavy chain CDRs (HCDR1, HCDR2, HCDR3) comprised in the heavy chain variable region as shown in SEQ ID NO: 70 and three light chain CDRs (LCDR1, LCDR2, LCDR3) comprised in the light chain variable region as shown in SEQ ID NO: 69;
10) three heavy chain CDRs (HCDR1, HCDR2, HCDR3) comprised in the heavy chain variable region as shown in SEQ ID NO: 72 and three light chain CDRs (LCDR1, LCDR2, LCDR3) comprised in the light chain variable region as shown in SEQ ID NO: 71;
11) three heavy chain CDRs (HCDR1, HCDR2, HCDR3) comprised in the heavy chain variable region as shown in SEQ ID NO: 74 and three light chain CDRs (LCDR1, LCDR2, LCDR3) comprised in the light chain variable region as shown in SEQ ID NO: 73;
12) three heavy chain CDRs (HCDR1, HCDR2, HCDR3) comprised in the heavy chain variable region as shown in SEQ ID NO: 75 and three light chain CDRs (LCDR1, LCDR2, LCDR3) comprised in the light chain variable region as shown in SEQ ID NO: 79;
13) three heavy chain CDRs (HCDR1, HCDR2, HCDR3) comprised in the heavy chain variable region as shown in SEQ ID NO: 76 and three light chain CDRs (LCDR1, LCDR2, LCDR3) comprised in the light chain variable region as shown in SEQ ID NO: 81;
14) three heavy chain CDRs (HCDR1, HCDR2, HCDR3) comprised in the heavy chain variable region as shown in SEQ ID NO: 77 and three light chain CDRs (LCDR1, LCDR2, LCDR3) comprised in the light chain variable region as shown in SEQ ID NO: 82;
15) three heavy chain CDRs (HCDR1, HCDR2, HCDR3) comprised in the heavy chain variable region as shown in SEQ ID NO: 78 and three light chain CDRs (LCDR1, LCDR2, LCDR3) comprised in the light chain variable region as shown in SEQ ID NO: 80;
16) three heavy chain CDRs (HCDR1, HCDR2, HCDR3) comprised in the heavy chain variable region as shown in SEQ ID NO: 78 and three light chain CDRs (LCDR1, LCDR2, LCDR3) comprised in the light chain variable region as shown in SEQ ID NO: 81;
17) three heavy chain CDRs (HCDR1, HCDR2, HCDR3) comprised in the heavy chain variable region as shown in SEQ ID NO: 78 and three light chain CDRs (LCDR1, LCDR2, LCDR3) comprised in the light chain variable region as shown in SEQ ID NO: 82; or
18) three heavy chain CDRs (HCDR1, HCDR2, HCDR3) comprised in the heavy chain variable region as shown in SEQ ID NO: 84 and three light chain CDRs (LCDR1, LCDR2, LCDR3) comprised in the light chain variable region as shown in SEQ ID NO: 89.

**2.** An anti-ROR1 antibody specifically binding to ROR1 and antigen-binding fragment thereof, comprising:

1) HCDR1, HCDR2 and HCDR3 as shown in sequence SEQ ID NO: 1, 2 and 3; and LCDR1, LCDR2 and LCDR3 as shown in sequence SEQ ID NO: 28, 29 and 30;
2) HCDR1, HCDR2 and HCDR3 as shown in sequence SEQ ID NO: 4, 5 and 6; and LCDR1, LCDR2 and LCDR3 as shown in sequence SEQ ID NO: 31, 32 and 33;
3) HCDR1, HCDR2 and HCDR3 as shown in sequence SEQ ID NO: 7, 8 and 9; and LCDR1, LCDR2 and LCDR3 as shown in sequence SEQ ID NO: 34, 32 and 33;
4) HCDR1, HCDR2 and HCDR3 as shown in sequence SEQ ID NO: 10, 11 and 12; and LCDR1, LCDR2 and LCDR3 as shown in sequence SEQ ID NO: 35, 32 and 36;
5) HCDR1, HCDR2 and HCDR3 as shown in sequence SEQ ID NO: 4, 13 and 14; and LCDR1, LCDR2 and LCDR3 as shown in sequence SEQ ID NO: 35, 32 and 37;
6) HCDR1, HCDR2 and HCDR3 as shown in sequence SEQ ID NO: 15, 16 and 17; and LCDR1, LCDR2 and LCDR3 as shown in sequence SEQ ID NO: 38, 32 and 33;
7) HCDR1, HCDR2 and HCDR3 as shown in sequence SEQ ID NO: 18, 8 and 19; and LCDR1, LCDR2 and LCDR3 as shown in sequence SEQ ID NO: 31, 32 and 33;
8) HCDR1, HCDR2 and HCDR3 as shown in sequence SEQ ID NO: 20, 21 and 12; and LCDR1, LCDR2 and LCDR3 as shown in sequence SEQ ID NO: 31, 32 and 33;
9) HCDR1, HCDR2 and HCDR3 as shown in sequence SEQ ID NO: 22, 23 and 24; and LCDR1, LCDR2 and LCDR3 as shown in sequence SEQ ID NO: 39, 40 and 41;
10) HCDR1, HCDR2 and HCDR3 as shown in sequence SEQ ID NO: 4, 25 and 26; and LCDR1, LCDR2 and LCDR3 as shown in sequence SEQ ID NO: 35, 32 and 33;
11) HCDR1, HCDR2 and HCDR3 as shown in sequence SEQ ID NO: 18, 25 and 27; and LCDR1, LCDR2 and LCDR3 as shown in sequence SEQ ID NO: 35, 32 and 33;
12) HCDR1, HCDR2 and HCDR3 as shown in sequence SEQ ID NO: 4, 5 and 6; and LCDR1, LCDR2 and LCDR3 as shown in sequence SEQ ID NO: 31, 45 and 48;
13) HCDR1, HCDR2 and HCDR3 as shown in sequence SEQ ID NO: 4, 5 and 6; and LCDR1, LCDR2 and LCDR3

as shown in sequence SEQ ID NO: 43, 46 and 48;
14) HCDR1, HCDR2 and HCDR3 as shown in sequence SEQ ID NO: 4, 5 and 6; and LCDR1, LCDR2 and LCDR3 as shown in sequence SEQ ID NO: 44, 47 and 48;
15) HCDR1, HCDR2 and HCDR3 as shown in sequence SEQ ID NO: 4, 42 and 6; and LCDR1, LCDR2 and LCDR3 as shown in sequence SEQ ID NO: 31, 45 and 48;
16) HCDR1, HCDR2 and HCDR3 as shown in sequence SEQ ID NO: 4, 42 and 6; and LCDR1, LCDR2 and LCDR3 as shown in sequence SEQ ID NO: 43, 46 and 48;
17) HCDR1, HCDR2 and HCDR3 as shown in sequence SEQ ID NO: 4, 42 and 6; and LCDR1, LCDR2 and LCDR3 as shown in sequence SEQ ID NO: 44, 47 and 48; or
18) HCDR1, HCDR2 and HCDR3 as shown in sequence SEQ ID NO: 1, 2 and 3; and LCDR1, LCDR2 and LCDR3 as shown in sequence SEQ ID NO: 49, 50 and 30.

**3.** The anti-ROR1 antibody and antigen-binding fragment thereof according to claim 1 or 2, comprising a heavy chain variable region and a light chain variable region, wherein:

1) the heavy chain variable region comprises the amino acid sequence as shown in SEQ ID NO: 57, or amino acid sequence having at least 90% identity to the amino acid sequence of SEQ ID NO: 57 and comprising HCDR1, HCDR2 and HCDR3 as shown in sequence SEQ ID NO: 1, 2 and 3, or consists of SEQ ID NO: 57, and the light chain variable region comprises the amino acid sequence as shown in SEQ ID NO: 56, or amino acid sequence having at least 90% identity to the amino acid sequence of SEQ ID NO: 56 and comprising LCDR1, LCDR2 and LCDR3 as shown in sequence SEQ ID NO: 28, 29 and 30, or consists of SEQ ID NO: 56;
2) the heavy chain variable region comprises the amino acid sequence as shown in SEQ ID NO: 55, or amino acid sequence having at least 90% identity to the amino acid sequence of SEQ ID NO: 55 and comprising HCDR1, HCDR2 and HCDR3 as shown in sequence SEQ ID NO: 4, 5 and 6, or consists of SEQ ID NO: 55, and the light chain variable region comprises the amino acid sequence as shown in SEQ ID NO: 54, or amino acid sequence having at least 90% identity to the amino acid sequence of SEQ ID NO: 54 and comprising LCDR1, LCDR2 and LCDR3 as shown in sequence SEQ ID NO: 31, 32 and 33, or consists of SEQ ID NO: 54;
3) the heavy chain variable region comprises the amino acid sequence as shown in SEQ ID NO: 59, or amino acid sequence having at least 90% identity to the amino acid sequence of SEQ ID NO: 59 and comprising HCDR1, HCDR2 and HCDR3 as shown in sequence SEQ ID NO: 7, 8 and 9, or consists of SEQ ID NO: 59, and the light chain variable region comprises the amino acid sequence as shown in SEQ ID NO: 58, or amino acid sequence having at least 90% identity to the amino acid sequence of SEQ ID NO: 58 and comprising LCDR1, LCDR2 and LCDR3 as shown in sequence SEQ ID NO: 34, 32 and 33, or consists of SEQ ID NO: 58;
4) the heavy chain variable region comprises the amino acid sequence as shown in SEQ ID NO: 61, or amino acid sequence having at least 90% identity to the amino acid sequence of SEQ ID NO: 61 and comprising HCDR1, HCDR2 and HCDR3 as shown in sequence SEQ ID NO: 10, 11 and 12, or consists of SEQ ID NO: 61, and the light chain variable region comprises the amino acid sequence as shown in SEQ ID NO: 60, or amino acid sequence having at least 90% identity to the amino acid sequence of SEQ ID NO: 60 and comprising LCDR1, LCDR2 and LCDR3 as shown in sequence SEQ ID NO: 35, 32 and 36, or consists of SEQ ID NO: 60;
5) the heavy chain variable region comprises the amino acid sequence as shown in SEQ ID NO: 63, or amino acid sequence having at least 90% identity to the amino acid sequence of SEQ ID NO: 63 and comprising HCDR1, HCDR2 and HCDR3 as shown in sequence SEQ ID NO: 4, 13 and 14, or consists of SEQ ID NO: 63, and the light chain variable region comprises the amino acid sequence as shown in SEQ ID NO: 62, or amino acid sequence having at least 90% identity to the amino acid sequence of SEQ ID NO: 62 and comprising LCDR1, LCDR2 and LCDR3 as shown in sequence SEQ ID NO: 35, 32 and 37, or consists of SEQ ID NO: 62;
6) the heavy chain variable region comprises the amino acid sequence as shown in SEQ ID NO: 65, or amino acid sequence having at least 90% identity to the amino acid sequence of SEQ ID NO: 65 and comprising HCDR1, HCDR2 and HCDR3 as shown in sequence SEQ ID NO: 15, 16 and 17, or consists of SEQ ID NO: 65, and the light chain variable region comprises the amino acid sequence as shown in SEQ ID NO: 64, or amino acid sequence having at least 90% identity to the amino acid sequence of SEQ ID NO: 64 and comprising LCDR1, LCDR2 and LCDR3 as shown in sequence SEQ ID NO: 38, 32 and 33, or consists of SEQ ID NO: 64;
7) the heavy chain variable region comprises the amino acid sequence as shown in SEQ ID NO: 67, or amino acid sequence having at least 90% identity to the amino acid sequence of SEQ ID NO: 67 and comprising HCDR1, HCDR2 and HCDR3 as shown in sequence SEQ ID NO: 18, 8 and 19, or consists of SEQ ID NO: 67, and the light chain variable region comprises the amino acid sequence as shown in SEQ ID NO: 66, or amino acid sequence having at least 90% identity to the amino acid sequence of SEQ ID NO: 66 and comprising LCDR1, LCDR2 and LCDR3 as shown in sequence SEQ ID NO: 31, 32 and 33, or consists of SEQ ID NO: 66;
8) the heavy chain variable region comprises the amino acid sequence as shown in SEQ ID NO: 68, or amino acid

sequence having at least 90% identity to the amino acid sequence of SEQ ID NO: 68 and comprising HCDR1, HCDR2 and HCDR3 as shown in sequence SEQ ID NO: 20, 21 and 12, or consists of SEQ ID NO: 68, and the light chain variable region comprises the amino acid sequence as shown in SEQ ID NO: 66, or amino acid sequence having at least 90% identity to the amino acid sequence of SEQ ID NO: 66 and comprising LCDR1, LCDR2 and LCDR3 as shown in sequence SEQ ID NO: 31, 32 and 33, or consists of SEQ ID NO: 66;
9) the heavy chain variable region comprises the amino acid sequence as shown in SEQ ID NO: 70, or amino acid sequence having at least 90% identity to the amino acid sequence of SEQ ID NO: 70 and comprising HCDR1, HCDR2 and HCDR3 as shown in sequence SEQ ID NO: 22, 23 and 24, or consists of SEQ ID NO: 70, and the light chain variable region comprises the amino acid sequence as shown in SEQ ID NO: 69, or amino acid sequence having at least 90% identity to the amino acid sequence of SEQ ID NO: 69 and comprising LCDR1, LCDR2 and LCDR3 as shown in sequence SEQ ID NO: 39, 40 and 41, or consists of SEQ ID NO: 69;
10) the heavy chain variable region comprises the amino acid sequence as shown in SEQ ID NO: 72, or amino acid sequence having at least 90% identity to the amino acid sequence of SEQ ID NO: 72 and comprising HCDR1, HCDR2 and HCDR3 as shown in sequence SEQ ID NO: 4, 25 and 26, or consists of SEQ ID NO: 72, and the light chain variable region comprises the amino acid sequence as shown in SEQ ID NO: 71, or amino acid sequence having at least 90% identity to the amino acid sequence of SEQ ID NO: 71 and comprising LCDR1, LCDR2 and LCDR3 as shown in sequence SEQ ID NO: 35, 32 and 33, or consists of SEQ ID NO: 71;
11) the heavy chain variable region comprises the amino acid sequence as shown in SEQ ID NO: 74, or amino acid sequence having at least 90% identity to the amino acid sequence of SEQ ID NO: 74 and comprising HCDR1, HCDR2 and HCDR3 as shown in sequence SEQ ID NO: 18, 25 and 27, or consists of SEQ ID NO: 74, and the light chain variable region comprises the amino acid sequence as shown in SEQ ID NO: 73, or amino acid sequence having at least 90% identity to the amino acid sequence of SEQ ID NO: 73 and comprising LCDR1, LCDR2 and LCDR3 as shown in sequence SEQ ID NO: 35, 32 and 33, or consists of SEQ ID NO: 73;
12) the heavy chain variable region comprises the amino acid sequence as shown in SEQ ID NO: 75, or amino acid sequence having at least 90% identity to the amino acid sequence of SEQ ID NO: 75 and comprising HCDR1, HCDR2 and HCDR3 as shown in sequence SEQ ID NO: 4, 5 and 6, or consists of SEQ ID NO: 75, and the light chain variable region comprises the amino acid sequence as shown in SEQ ID NO: 79, or amino acid sequence having at least 90% identity to the amino acid sequence of SEQ ID NO: 79 and comprising LCDR1, LCDR2 and LCDR3 as shown in sequence SEQ ID NO: 31, 45 and 48, or consists of SEQ ID NO: 79;
13) the heavy chain variable region comprises the amino acid sequence as shown in SEQ ID NO: 76, or amino acid sequence having at least 90% identity to the amino acid sequence of SEQ ID NO: 76 and comprising HCDR1, HCDR2 and HCDR3 as shown in sequence SEQ ID NO: 4, 5 and 6, or consists of SEQ ID NO: 76, and the light chain variable region comprises the amino acid sequence as shown in SEQ ID NO: 80, or amino acid sequence having at least 90% identity to the amino acid sequence of SEQ ID NO: 80 and comprising LCDR1, LCDR2 and LCDR3 as shown in sequence SEQ ID NO: 31, 45 and 48, or consists of SEQ ID NO: 80;
14) the heavy chain variable region comprises the amino acid sequence as shown in SEQ ID NO: 76, or amino acid sequence having at least 90% identity to the amino acid sequence of SEQ ID NO: 76 and comprising HCDR1, HCDR2 and HCDR3 as shown in sequence SEQ ID NO: 4, 5 and 6, or consists of SEQ ID NO: 76, and the light chain variable region comprises the amino acid sequence as shown in SEQ ID NO: 81, or amino acid sequence having at least 90% identity to the amino acid sequence of SEQ ID NO: 81 and comprising LCDR1, LCDR2 and LCDR3 as shown in sequence SEQ ID NO: 43, 46 and 48, or consists of SEQ ID NO: 81;
15) the heavy chain variable region comprises the amino acid sequence as shown in SEQ ID NO: 77, or amino acid sequence having at least 90% identity to the amino acid sequence of SEQ ID NO: 77 and comprising HCDR1, HCDR2 and HCDR3 as shown in sequence SEQ ID NO: 4, 5 and 6, or consists of SEQ ID NO: 77, and the light chain variable region comprises the amino acid sequence as shown in SEQ ID NO: 80, or amino acid sequence having at least 90% identity to the amino acid sequence of SEQ ID NO: 80 and comprising LCDR1, LCDR2 and LCDR3 as shown in sequence SEQ ID NO: 31, 45 and 48, or consists of SEQ ID NO: 80;
16) the heavy chain variable region comprises the amino acid sequence as shown in SEQ ID NO: 77, or amino acid sequence having at least 90% identity to the amino acid sequence of SEQ ID NO: 77 and comprising HCDR1, HCDR2 and HCDR3 as shown in sequence SEQ ID NO: 4, 5 and 6, or consists of SEQ ID NO: 77, and the light chain variable region comprises the amino acid sequence as shown in SEQ ID NO: 81, or amino acid sequence having at least 90% identity to the amino acid sequence of SEQ ID NO: 81 and comprising LCDR1, LCDR2 and LCDR3 as shown in sequence SEQ ID NO: 43, 46 and 48, or consists of SEQ ID NO: 81;
17) the heavy chain variable region comprises the amino acid sequence as shown in SEQ ID NO: 77, or amino acid sequence having at least 90% identity to the amino acid sequence of SEQ ID NO: 77 and comprising HCDR1, HCDR2 and HCDR3 as shown in sequence SEQ ID NO: 4, 5 and 6, or consists of SEQ ID NO: 77, and the light chain variable region comprises the amino acid sequence as shown in SEQ ID NO: 82, or amino acid sequence having at least 90% identity to the amino acid sequence of SEQ ID NO: 82 and comprising LCDR1, LCDR2 and

LCDR3 as shown in sequence SEQ ID NO: 44, 47 and 48, or consists of SEQ ID NO: 82;

18) the heavy chain variable region comprises the amino acid sequence as shown in SEQ ID NO: 78, or amino acid sequence having at least 90% identity to the amino acid sequence of SEQ ID NO: 78 and comprising HCDR1, HCDR2 and HCDR3 as shown in sequence SEQ ID NO: 4, 42 and 6, or consists of SEQ ID NO: 78, and the light chain variable region comprises the amino acid sequence as shown in SEQ ID NO: 80, or amino acid sequence having at least 90% identity to the amino acid sequence of SEQ ID NO: 80 and comprising LCDR1, LCDR2 and LCDR3 as shown in sequence SEQ ID NO: 31, 45 and 48, or consists of SEQ ID NO: 80;

19) the heavy chain variable region comprises the amino acid sequence as shown in SEQ ID NO: 78, or amino acid sequence having at least 90% identity to the amino acid sequence of SEQ ID NO: 78 and comprising HCDR1, HCDR2 and HCDR3 as shown in sequence SEQ ID NO: 4, 42 and 6, or consists of SEQ ID NO: 78, and the light chain variable region comprises the amino acid sequence as shown in SEQ ID NO: 81, or amino acid sequence having at least 90% identity to the amino acid sequence of SEQ ID NO: 81 and comprising LCDR1, LCDR2 and LCDR3 as shown in sequence SEQ ID NO: 43, 46 and 48, or consists of SEQ ID NO: 81;

20) the heavy chain variable region comprises the amino acid sequence as shown in SEQ ID NO: 78, or amino acid sequence having at least 90% identity to the amino acid sequence of SEQ ID NO: 78 and comprising HCDR1, HCDR2 and HCDR3 as shown in sequence SEQ ID NO: 4, 42 and 6, or consists of SEQ ID NO: 78, and the light chain variable region comprises the amino acid sequence as shown in SEQ ID NO: 82, or amino acid sequence having at least 90% identity to the amino acid sequence of SEQ ID NO: 82 and comprising LCDR1, LCDR2 and LCDR3 as shown in sequence SEQ ID NO: 44, 47 and 48, or consists of SEQ ID NO: 82;

21) the heavy chain variable region comprises the amino acid sequence as shown in SEQ ID NO: 83, or amino acid sequence having at least 90% identity to the amino acid sequence of SEQ ID NO: 83 and comprising HCDR1, HCDR2 and HCDR3 as shown in sequence SEQ ID NO: 1, 2 and 3, or consists of SEQ ID NO: 83, and the light chain variable region comprises the amino acid sequence as shown in SEQ ID NO: 86, or amino acid sequence having at least 90% identity to the amino acid sequence of SEQ ID NO: 86 and comprising LCDR1, LCDR2 and LCDR3 as shown in sequence SEQ ID NO: 28, 29 and 30, or consists of SEQ ID NO: 86;

22) the heavy chain variable region comprises the amino acid sequence as shown in SEQ ID NO: 83, or amino acid sequence having at least 90% identity to the amino acid sequence of SEQ ID NO: 83 and comprising HCDR1, HCDR2 and HCDR3 as shown in sequence SEQ ID NO: 1, 2 and 3, or consists of SEQ ID NO: 83, and the light chain variable region comprises the amino acid sequence as shown in SEQ ID NO: 87, or amino acid sequence having at least 90% identity to the amino acid sequence of SEQ ID NO: 87 and comprising LCDR1, LCDR2 and LCDR3 as shown in sequence SEQ ID NO: 28, 29 and 30, or consists of SEQ ID NO: 87;

23) the heavy chain variable region comprises the amino acid sequence as shown in SEQ ID NO: 84, or amino acid sequence having at least 90% identity to the amino acid sequence of SEQ ID NO: 84 and comprising HCDR1, HCDR2 and HCDR3 as shown in sequence SEQ ID NO: 1, 2 and 3, or consists of SEQ ID NO: 84, and the light chain variable region comprises the amino acid sequence as shown in SEQ ID NO: 87, or amino acid sequence having at least 90% identity to the amino acid sequence of SEQ ID NO: 87 and comprising LCDR1, LCDR2 and LCDR3 as shown in sequence SEQ ID NO: 28, 29 and 30, or consists of SEQ ID NO: 87;

24) the heavy chain variable region comprises the amino acid sequence as shown in SEQ ID NO: 84, or amino acid sequence having at least 90% identity to the amino acid sequence of SEQ ID NO: 84 and comprising HCDR1, HCDR2 and HCDR3 as shown in sequence SEQ ID NO: 1, 2 and 3, or consists of SEQ ID NO: 84, and the light chain variable region comprises the amino acid sequence as shown in SEQ ID NO: 88, or amino acid sequence having at least 90% identity to the amino acid sequence of SEQ ID NO: 88 and comprising LCDR1, LCDR2 and LCDR3 as shown in sequence SEQ ID NO: 28, 29 and 30, or consists of SEQ ID NO: 88;

25) the heavy chain variable region comprises the amino acid sequence as shown in SEQ ID NO: 84, or amino acid sequence having at least 90% identity to the amino acid sequence of SEQ ID NO: 84 and comprising HCDR1, HCDR2 and HCDR3 as shown in sequence SEQ ID NO: 1, 2 and 3, or consists of SEQ ID NO: 84, and the light chain variable region comprises the amino acid sequence as shown in SEQ ID NO: 89, or amino acid sequence having at least 90% identity to the amino acid sequence of SEQ ID NO: 89 and comprising LCDR1, LCDR2 and LCDR3 as shown in sequence SEQ ID NO: 49, 50 and 30, or consists of SEQ ID NO: 89;

26) the heavy chain variable region comprises the amino acid sequence as shown in SEQ ID NO: 85, or amino acid sequence having at least 90% identity to the amino acid sequence of SEQ ID NO: 85 and comprising HCDR1, HCDR2 and HCDR3 as shown in sequence SEQ ID NO: 1, 2 and 3, or consists of SEQ ID NO: 85, and the light chain variable region comprises the amino acid sequence as shown in SEQ ID NO: 87, or amino acid sequence having at least 90% identity to the amino acid sequence of SEQ ID NO: 87 and comprising LCDR1, LCDR2 and LCDR3 as shown in sequence SEQ ID NO: 28, 29 and 30, or consists of SEQ ID NO: 87;

27) the heavy chain variable region comprises the amino acid sequence as shown in SEQ ID NO: 85, or amino acid sequence having at least 90% identity to the amino acid sequence of SEQ ID NO: 85 and comprising HCDR1, HCDR2 and HCDR3 as shown in sequence SEQ ID NO: 1, 2 and 3, or consists of SEQ ID NO: 85, and the light

chain variable region comprises the amino acid sequence as shown in SEQ ID NO: 88, or amino acid sequence having at least 90% identity to the amino acid sequence of SEQ ID NO: 88 and comprising LCDR1, LCDR2 and LCDR3 as shown in sequence SEQ ID NO: 28, 29 and 30, or consists of SEQ ID NO: 88; or

28) the heavy chain variable region comprises the amino acid sequence as shown in SEQ ID NO: 85, or amino acid sequence having at least 90% identity to the amino acid sequence of SEQ ID NO: 85 and comprising HCDR1, HCDR2 and HCDR3 as shown in sequence SEQ ID NO: 1, 2 and 3, or consists of SEQ ID NO: 85, and the light chain variable region comprises the amino acid sequence as shown in SEQ ID NO: 89, or amino acid sequence having at least 90% identity to the amino acid sequence of SEQ ID NO: 89 and comprising LCDR1, LCDR2 and LCDR3 as shown in sequence SEQ ID NO: 49, 50 and 30, or consists of SEQ ID NO: 89.

**4.** The isolated anti-ROR1 antibody or antigen-binding fragment thereof according to any one of claims 1 to 3, wherein the antigen-binding fragment is selected from the group consisting of Fab, Fab'-SH, Fv (*e.g.,* scFv) or (Fab')2 fragment.

**5.** The isolated anti-ROR1 monoclonal antibody or antigen-binding fragment thereof according to any one of claims 1 to 4, comprising a constant region sequence, wherein at least a part of the constant region sequence is human consensus constant region sequence.

**6.** The antibody or antigen-binding fragment thereof according to any one of claims 1 to 5, wherein the antibody heavy chain constant region comprises the amino acid sequence as shown in SEQ ID NO: 52, and the light chain constant region comprises the amino acid sequence as shown in SEQ ID NO: 53.

**7.** The antibody or antigen-binding fragment thereof according to any one of claims 1 to 6, comprising a heavy chain and a light chain, wherein:

1) the heavy chain comprises the amino acid sequence as shown in SEQ ID NO: 93, or amino acid sequence having at least 90% identity to the amino acid sequence of SEQ ID NO: 93, or consists of SEQ ID NO: 93, and the light chain comprises the amino acid sequence as shown in SEQ ID NO: 92, or amino acid sequence having at least 90% identity to the amino acid sequence of SEQ ID NO: 92, or consists of SEQ ID NO: 92;

2) the heavy chain comprises the amino acid sequence as shown in SEQ ID NO: 91, or amino acid sequence having at least 90% identity to the amino acid sequence of SEQ ID NO: 91, or consists of SEQ ID NO: 91, and the light chain comprises the amino acid sequence as shown in SEQ ID NO: 90, or amino acid sequence having at least 90% identity to the amino acid sequence of SEQ ID NO: 90, or consists of SEQ ID NO: 90;

3) the heavy chain comprises the amino acid sequence as shown in SEQ ID NO: 95, or amino acid sequence having at least 90% identity to the amino acid sequence of SEQ ID NO: 95, or consists of SEQ ID NO: 95, and the light chain comprises the amino acid sequence as shown in SEQ ID NO: 94, or amino acid sequence having at least 90% identity to the amino acid sequence of SEQ ID NO: 94, or consists of SEQ ID NO: 94;

4) the heavy chain comprises the amino acid sequence as shown in SEQ ID NO: 97, or amino acid sequence having at least 90% identity to the amino acid sequence of SEQ ID NO: 97, or consists of SEQ ID NO: 97, and the light chain comprises the amino acid sequence as shown in SEQ ID NO: 96, or amino acid sequence having at least 90% identity to the amino acid sequence of SEQ ID NO: 96, or consists of SEQ ID NO: 96;

5) the heavy chain comprises the amino acid sequence as shown in SEQ ID NO: 99, or amino acid sequence having at least 90% identity to the amino acid sequence of SEQ ID NO: 99, or consists of SEQ ID NO: 99, and the light chain comprises the amino acid sequence as shown in SEQ ID NO: 98, or amino acid sequence having at least 90% identity to the amino acid sequence of SEQ ID NO: 98, or consists of SEQ ID NO: 98;

6) the heavy chain comprises the amino acid sequence as shown in SEQ ID NO: 101, or amino acid sequence having at least 90% identity to the amino acid sequence of SEQ ID NO: 101, or consists of SEQ ID NO: 101, and the light chain comprises the amino acid sequence as shown in SEQ ID NO: 100, or amino acid sequence having at least 90% identity to the amino acid sequence of SEQ ID NO: 100, or consists of SEQ ID NO: 100;

7) the heavy chain comprises the amino acid sequence as shown in SEQ ID NO: 103, or amino acid sequence having at least 90% identity to the amino acid sequence of SEQ ID NO: 103, or consists of SEQ ID NO: 103, and the light chain comprises the amino acid sequence as shown in SEQ ID NO: 102, or amino acid sequence having at least 90% identity to the amino acid sequence of SEQ ID NO: 102, or consists of SEQ ID NO: 102;

8) the heavy chain comprises the amino acid sequence as shown in SEQ ID NO: 104, or amino acid sequence having at least 90% identity to the amino acid sequence of SEQ ID NO: 104, or consists of SEQ ID NO: 104, and the light chain comprises the amino acid sequence as shown in SEQ ID NO: 102, or amino acid sequence having at least 90% identity to the amino acid sequence of SEQ ID NO: 102, or consists of SEQ ID NO: 102;

9) the heavy chain comprises the amino acid sequence as shown in SEQ ID NO: 106, or amino acid sequence

having at least 90% identity to the amino acid sequence of SEQ ID NO: 106, or consists of SEQ ID NO: 106, and the light chain comprises the amino acid sequence as shown in SEQ ID NO: 105, or amino acid sequence having at least 90% identity to the amino acid sequence of SEQ ID NO: 105, or consists of SEQ ID NO: 105;
10) the heavy chain comprises the amino acid sequence as shown in SEQ ID NO: 108, or amino acid sequence having at least 90% identity to the amino acid sequence of SEQ ID NO: 108, or consists of SEQ ID NO: 108, and the light chain comprises the amino acid sequence as shown in SEQ ID NO: 107, or amino acid sequence having at least 90% identity to the amino acid sequence of SEQ ID NO: 107, or consists of SEQ ID NO: 107;
11) the heavy chain comprises the amino acid sequence as shown in SEQ ID NO: 110, or amino acid sequence having at least 90% identity to the amino acid sequence of SEQ ID NO: 110, or consists of SEQ ID NO: 110, and the light chain comprises the amino acid sequence as shown in SEQ ID NO: 109, or amino acid sequence having at least 90% identity to the amino acid sequence of SEQ ID NO: 109, or consists of SEQ ID NO: 109;
12) the heavy chain comprises the amino acid sequence as shown in SEQ ID NO: 111, or amino acid sequence having at least 90% identity to the amino acid sequence of SEQ ID NO: 111, or consists of SEQ ID NO: 111, and the light chain comprises the amino acid sequence as shown in SEQ ID NO: 115, or amino acid sequence having at least 90% identity to the amino acid sequence of SEQ ID NO: 115, or consists of SEQ ID NO: 115;
13) the heavy chain comprises the amino acid sequence as shown in SEQ ID NO: 112, or amino acid sequence having at least 90% identity to the amino acid sequence of SEQ ID NO: 112, or consists of SEQ ID NO: 112, and the light chain comprises the amino acid sequence as shown in SEQ ID NO: 116, or amino acid sequence having at least 90% identity to the amino acid sequence of SEQ ID NO: 116, or consists of SEQ ID NO: 116;
14) the heavy chain comprises the amino acid sequence as shown in SEQ ID NO: 112, or amino acid sequence having at least 90% identity to the amino acid sequence of SEQ ID NO: 112, or consists of SEQ ID NO: 112, and the light chain comprises the amino acid sequence as shown in SEQ ID NO: 117, or amino acid sequence having at least 90% identity to the amino acid sequence of SEQ ID NO: 117, or consists of SEQ ID NO: 117;
15) the heavy chain comprises the amino acid sequence as shown in SEQ ID NO: 113, or amino acid sequence having at least 90% identity to the amino acid sequence of SEQ ID NO: 113, or consists of SEQ ID NO: 113, and the light chain comprises the amino acid sequence as shown in SEQ ID NO: 116, or amino acid sequence having at least 90% identity to the amino acid sequence of SEQ ID NO: 116, or consists of SEQ ID NO: 116;
16) the heavy chain comprises the amino acid sequence as shown in SEQ ID NO: 113, or amino acid sequence having at least 90% identity to the amino acid sequence of SEQ ID NO: 113, or consists of SEQ ID NO: 113, and the light chain comprises the amino acid sequence as shown in SEQ ID NO: 117, or amino acid sequence having at least 90% identity to the amino acid sequence of SEQ ID NO: 117, or consists of SEQ ID NO: 117;
17) the heavy chain comprises the amino acid sequence as shown in SEQ ID NO: 113, or amino acid sequence having at least 90% identity to the amino acid sequence of SEQ ID NO: 113, or consists of SEQ ID NO: 113, and the light chain comprises the amino acid sequence as shown in SEQ ID NO: 118, or amino acid sequence having at least 90% identity to the amino acid sequence of SEQ ID NO: 118, or consists of SEQ ID NO: 118;
18) the heavy chain comprises the amino acid sequence as shown in SEQ ID NO: 114, or amino acid sequence having at least 90% identity to the amino acid sequence of SEQ ID NO: 114, or consists of SEQ ID NO: 114, and the light chain comprises the amino acid sequence as shown in SEQ ID NO: 116, or amino acid sequence having at least 90% identity to the amino acid sequence of SEQ ID NO: 116, or consists of SEQ ID NO: 116;
19) the heavy chain comprises the amino acid sequence as shown in SEQ ID NO: 114, or amino acid sequence having at least 90% identity to the amino acid sequence of SEQ ID NO: 114, or consists of SEQ ID NO: 114, and the light chain comprises the amino acid sequence as shown in SEQ ID NO: 117, or amino acid sequence having at least 90% identity to the amino acid sequence of SEQ ID NO: 117, or consists of SEQ ID NO: 117;
20) the heavy chain comprises the amino acid sequence as shown in SEQ ID NO: 114, or amino acid sequence having at least 90% identity to the amino acid sequence of SEQ ID NO: 114, or consists of SEQ ID NO: 114, and the light chain comprises the amino acid sequence as shown in SEQ ID NO: 118, or amino acid sequence having at least 90% identity to the amino acid sequence of SEQ ID NO: 118, or consists of SEQ ID NO: 118;
21) the heavy chain comprises the amino acid sequence as shown in SEQ ID NO: 119, or amino acid sequence having at least 90% identity to the amino acid sequence of SEQ ID NO: 119, or consists of SEQ ID NO: 119, and the light chain comprises the amino acid sequence as shown in SEQ ID NO: 122, or amino acid sequence having at least 90% identity to the amino acid sequence of SEQ ID NO: 122, or consists of SEQ ID NO: 122;
22) the heavy chain comprises the amino acid sequence as shown in SEQ ID NO: 119, or amino acid sequence having at least 90% identity to the amino acid sequence of SEQ ID NO: 119, or consists of SEQ ID NO: 119, and the light chain comprises the amino acid sequence as shown in SEQ ID NO: 123, or amino acid sequence having at least 90% identity to the amino acid sequence of SEQ ID NO: 123, or consists of SEQ ID NO: 123;
23) the heavy chain comprises the amino acid sequence as shown in SEQ ID NO: 120, or amino acid sequence having at least 90% identity to the amino acid sequence of SEQ ID NO: 120, or consists of SEQ ID NO: 120, and the light chain comprises the amino acid sequence as shown in SEQ ID NO: 123, or amino acid sequence having

at least 90% identity to the amino acid sequence of SEQ ID NO: 123, or consists of SEQ ID NO: 123;
24) the heavy chain comprises the amino acid sequence as shown in SEQ ID NO: 120, or amino acid sequence having at least 90% identity to the amino acid sequence of SEQ ID NO: 120, or consists of SEQ ID NO: 120, and the light chain comprises the amino acid sequence as shown in SEQ ID NO: 124, or amino acid sequence having at least 90% identity to the amino acid sequence of SEQ ID NO: 124, or consists of SEQ ID NO: 124;
25) the heavy chain comprises the amino acid sequence as shown in SEQ ID NO: 120, or amino acid sequence having at least 90% identity to the amino acid sequence of SEQ ID NO: 120, or consists of SEQ ID NO: 120, and the light chain comprises the amino acid sequence as shown in SEQ ID NO: 125, or amino acid sequence having at least 90% identity to the amino acid sequence of SEQ ID NO: 125, or consists of SEQ ID NO: 125;
26) the heavy chain comprises the amino acid sequence as shown in SEQ ID NO: 121, or amino acid sequence having at least 90% identity to the amino acid sequence of SEQ ID NO: 121, or consists of SEQ ID NO: 121, and the light chain comprises the amino acid sequence as shown in SEQ ID NO: 123, or amino acid sequence having at least 90% identity to the amino acid sequence of SEQ ID NO: 123, or consists of SEQ ID NO: 123;
27) the heavy chain comprises the amino acid sequence as shown in SEQ ID NO: 121, or amino acid sequence having at least 90% identity to the amino acid sequence of SEQ ID NO: 121, or consists of SEQ ID NO: 121, and the light chain comprises the amino acid sequence as shown in SEQ ID NO: 124, or amino acid sequence having at least 90% identity to the amino acid sequence of SEQ ID NO: 124, or consists of SEQ ID NO: 124; or
28) the heavy chain comprises the amino acid sequence as shown in SEQ ID NO: 121, or amino acid sequence having at least 90% identity to the amino acid sequence of SEQ ID NO: 121, or consists of SEQ ID NO: 121, and the light chain comprises the amino acid sequence as shown in SEQ ID NO: 125, or amino acid sequence having at least 90% identity to the amino acid sequence of SEQ ID NO: 125, or consists of SEQ ID NO: 125.

**8.** An antibody-drug conjugate having the structure of Ab-(L-D)n, wherein Ab is an anti-ROR1 antibody or antigen-binding fragment thereof as described in any one of claims 1 to 7, L is a linker, D is a therapeutically active substance or a pharmaceutically active ingredient, and n represents an integer from 1 to 20.

**9.** The antibody-drug conjugate of claim 8, wherein the therapeutically active substance or pharmaceutically active ingredient is a cytotoxin, a plant toxin, a small molecule toxin, or a radioactive isotope.

**10.** The antibody-drug conjugate according to claim 8 or 9, wherein the therapeutically active substance or pharmaceutically active ingredient is Dolastatin and auristatin derivative thereof, such as Dolastatin 10, Dolastatin 15, auristatin E, auristatin PE, monomethyl auristatin D (MMAD), monomethyl auristatin E (MMAE), monomethyl auristatin F (MMAF) , auristatin F phenylenediamine (AFP), auristatin EB (AEB), auristatin EFP (AEFP), auristatin F hydroxypropylamide (AF HPA).

**11.** The antibody-drug conjugate according to any one of claims 8 to 10, wherein the dolastatin and auristatin derivatives thereof are MMAE, MMAF, auristatin F hydroxypropylamide or auristatin F phenylenediamine.

**12.** The antibody-drug conjugate according to any one of claims 8 to 11, wherein the linker is linker degradable by cathepsin, (such as valine-citrulline (val-cit) linker, cBu-Cit linker and CX linker); non-cleavable linker (such as SMCC linker or MD linker); acid-sensitive linker, silicone-structured linker, disulfide-carbamate linker, MC-GGFG linker, TRX linker, galactoside-containing linker, pyrophosphate linker, near-infrared-sensitive linker, ultraviolet-sensitive linker (such as PC4AP).

**13.** The antibody-drug conjugate according to any one of claims 8 to 12, wherein the linker is selected from the group consisting of maleimido-hexanoyl-valine-citrulline-p-aminobenzyloxy (mc-vc-PAB), acetyl-lysine-valine-citrulline-p-aminobenzyloxycarbonyl (AcLys-VC-PABC), amino PEG6-propionyl, maleimidocaproyl (mc), maleimidopropionyl (MP), valine-citrulline (val-cit), alanine-phenylalanine (ala-phe), p-aminobenzyloxycarbonyl (PAB), N-succinimidyl 4-(2-pyridylthio) pentanoate (SPP), N-succinimidyl 4-(N-maleimidomethyl)-cyclohexane-1-carboxylate (SMCC), N-succinimidyl (4-iodo-acetyl) aminobenzoate (SIAB), N-succinimidyl-4-(2-pyridyldithio) butyrate (SPDB), N-succinimidyl 3-(pyridin-2-yldithio)-propionate (SPDP).

**14.** The antibody-drug conjugate of any one of claims 8 to 13, wherein the linker is MC-VC-PAB, SMCC or MC-GGFG.

**15.** The antibody-drug conjugate according to any one of claims 8 to 14, wherein the Ab comprises:

(1) HCDR1, HCDR2 and HCDR3 as shown in sequence SEQ ID NO: 1, 2 and 3, and LCDR1, LCDR2 and LCDR3 as shown in sequence SEQ ID NO: 28, 29 and 30, or

(2) HCDR1, HCDR2 and HCDR3 as shown in sequence SEQ ID NO: 4, 5 and 6, and LCDR1, LCDR2 and LCDR3 as shown in sequence SEQ ID NO: 43, 46 and 48.

**16.** The antibody-drug conjugate according to any one of claims 8 to 15, wherein the Ab comprises a heavy chain variable region and a light chain variable region, wherein

(1) the heavy chain variable region comprises the amino acid sequence as shown in SEQ ID NO: 85, and the light chain variable region comprises the amino acid sequence as shown in SEQ ID NO: 88, or
(2) the heavy chain variable region comprises the amino acid sequence as shown in SEQ ID NO: 77, and the light chain variable region comprises the amino acid sequence as shown in SEQ ID NO: 81.

**17.** The antibody-drug conjugate according to any one of claims 8 to 16, wherein the Ab comprises:

(1) a heavy chain of the amino acid sequence as shown in SEQ ID NO: 121 and a light chain of the amino acid sequence as shown in SEQ ID NO: 124; or
(2) a heavy chain of the amino acid sequence as shown in SEQ ID NO: 113 and a light chain of the amino acid sequence as shown in SEQ ID NO: 117.

**18.** The antibody-drug conjugate according to any one of claims 15 to 17, wherein the linker is MC-VC-PAB and the cytotoxin is MMAE.

**19.** A pharmaceutical composition comprising:

(1) the anti-ROR1 antibody or antigen-binding fragment thereof according to any one of claims 1 to 7, or the antibody-drug conjugate according to any one of claims 8 to 18, and;
(2) pharmaceutically acceptable carrier.

**20.** An isolated polynucleotide molecule encoding the anti-ROR1 antibody or antigen-binding fragment thereof according to any one of claims 1 to 7.

**21.** A vector comprising the nucleic acid molecule according to claim 20, preferably, the vector is an expression vector.

**22.** A host cell comprising the vector of claim 21 or the nucleic acid molecule of claim 20.

**23.** Use of the anti-ROR1 antibody or antigen-binding fragment thereof according to any one of claims 1 to 7 in the manufature of an antibody-drug conjugate for preventing or treating cancer, or in the manufature of a medicament for preventing or treating a disease associated with abnormal expression of ROR1.

**24.** Use of the antibody-drug conjugate according to any one of claims 8 to 18 in the manufature of a medicament for preventing or treating a disease associated with abnormal expression of ROR1.

**25.** A method for killing cells expressing ROR1 or inhibiting the growth of cells expressing ROR1, comprising contacting the cells with an effective amount of the antibody or antigen-binding fragment thereof according to any one of claims 1 to 7, or an effective amount of the antibody-drug conjugate according to any one of claims 8 to 18, or an effective amount of the pharmaceutical composition according to claim 19.

**26.** A method for preventing or treating a disease associated with abnormal expression of ROR1 in a subject in need thereof, comprising administering to the subject a preventively or therapeutically effective amount of the antibody or antigen-binding fragment thereof according to any one of claims 1 to 7, or a preventively or therapeutically effective amount of the antibody-drug conjugate according to any one of claims 8 to 18, or a preventively or therapeutically effective amount of the pharmaceutical composition according to claim 19.

**27.** The use according to claim 24 or the method according to claim 26, wherein the disease associated with abnormal expression of ROR1 is cancer with high expression of ROR1.

**28.** The use or method according to claim 27, wherein the cancer with high expression of ROR1 is chronic lymphocytic leukemia (CLL), acute lymphocytic leukemia (ALL), mantle cell lymphoma, renal cell carcinoma, colon cancer, breast cancer, neuroblastoma, lung cancer, gastric cancer, head and neck cancer, and melanoma.

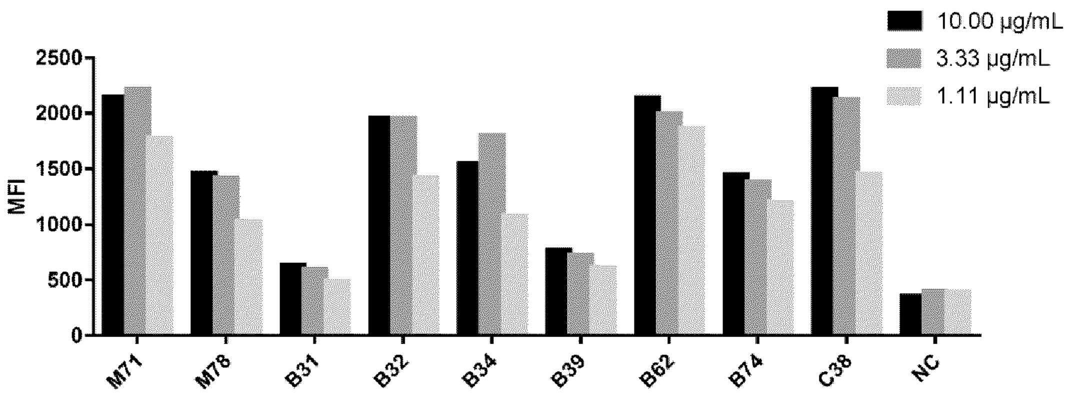


Figure 1A

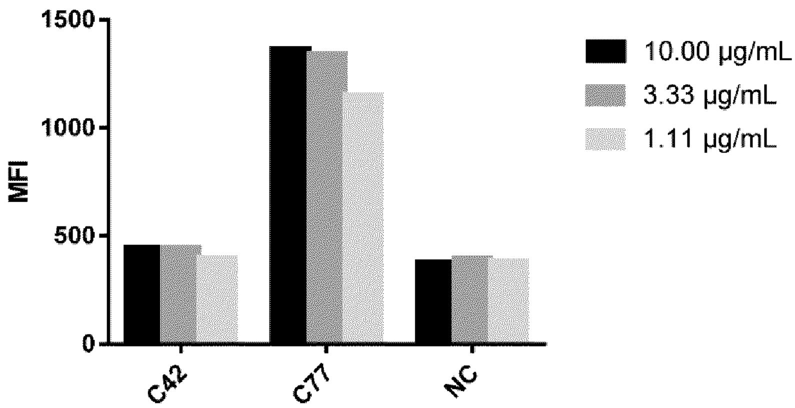


Figure 1B

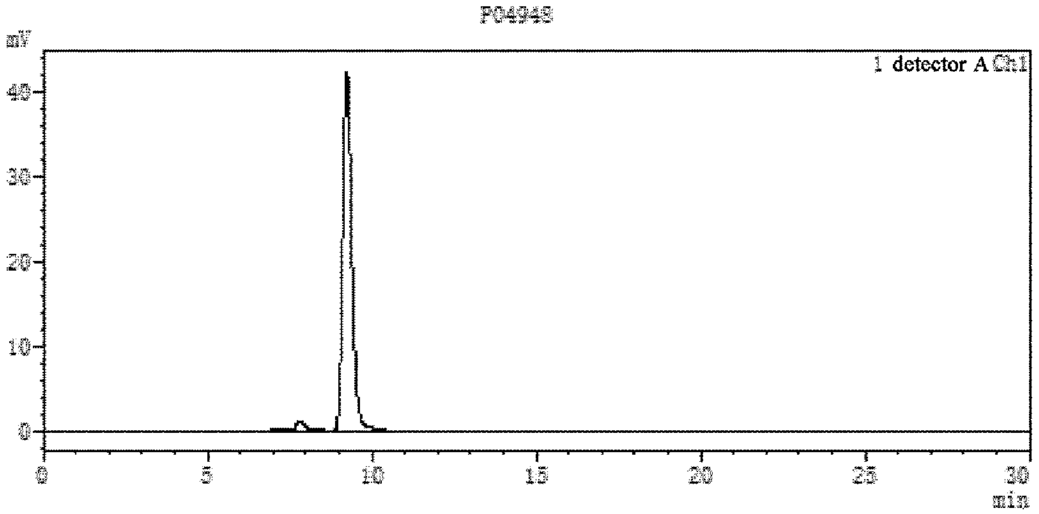


Figure 2A

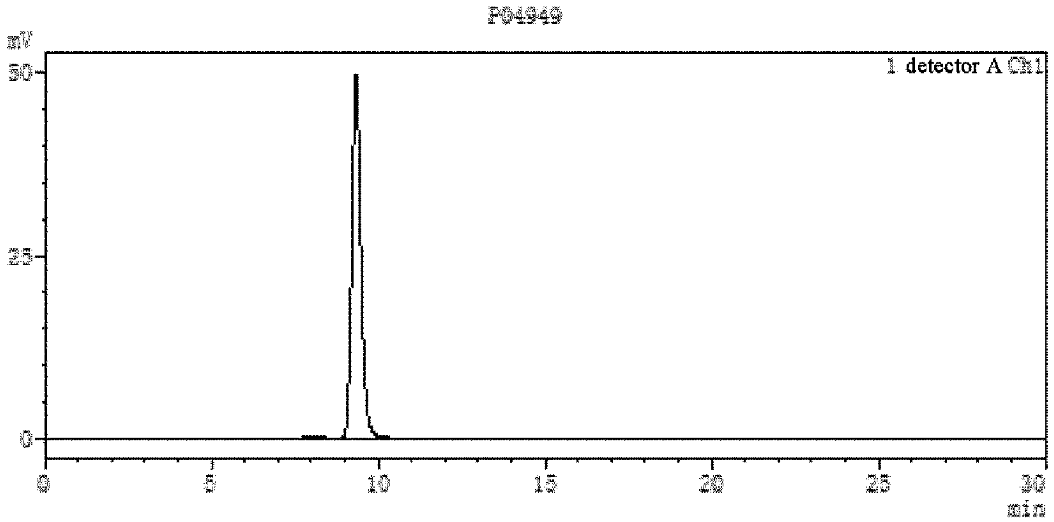


Figure 2B

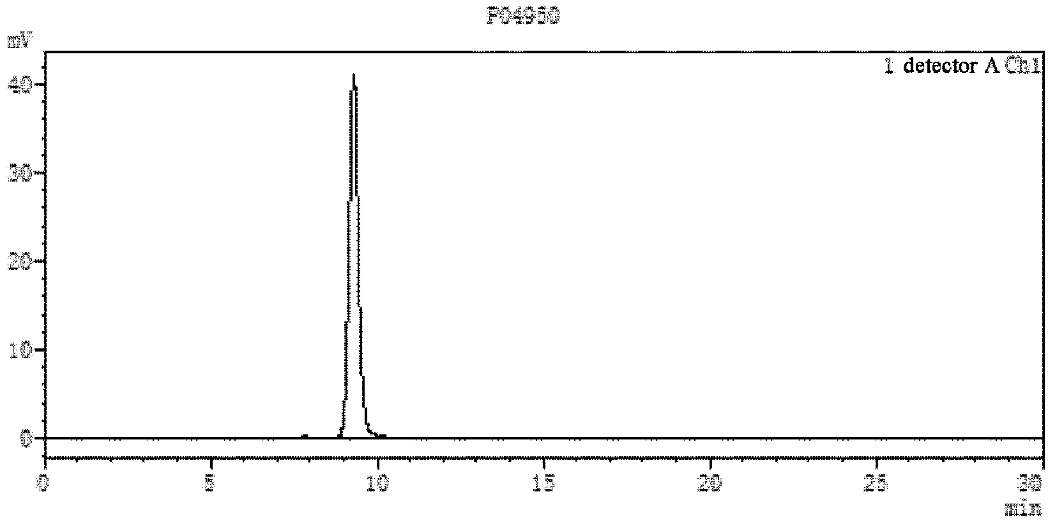


Figure 2C

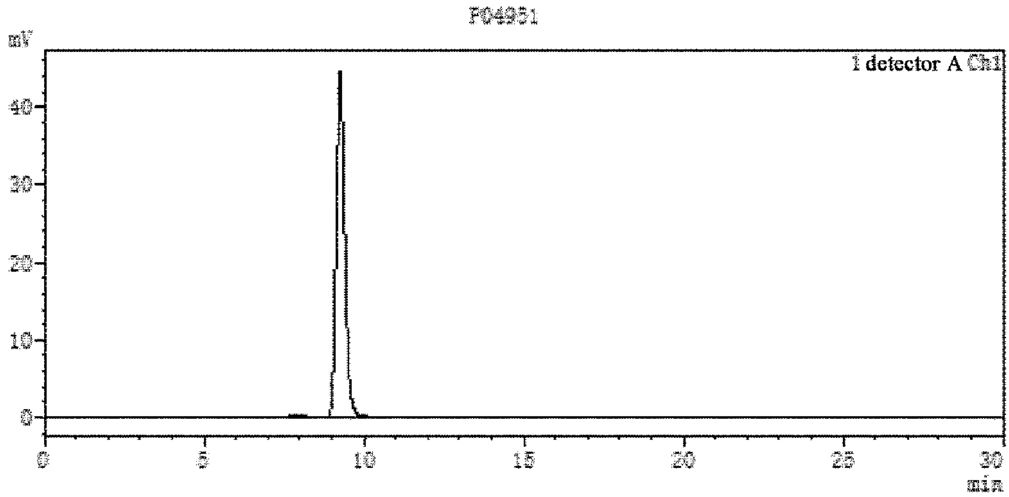


Figure 2D

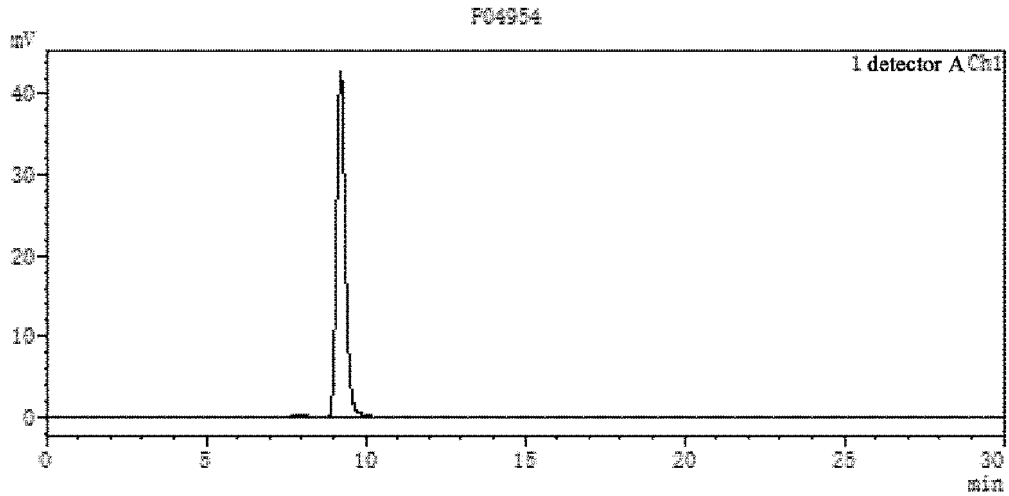


Figure 2E

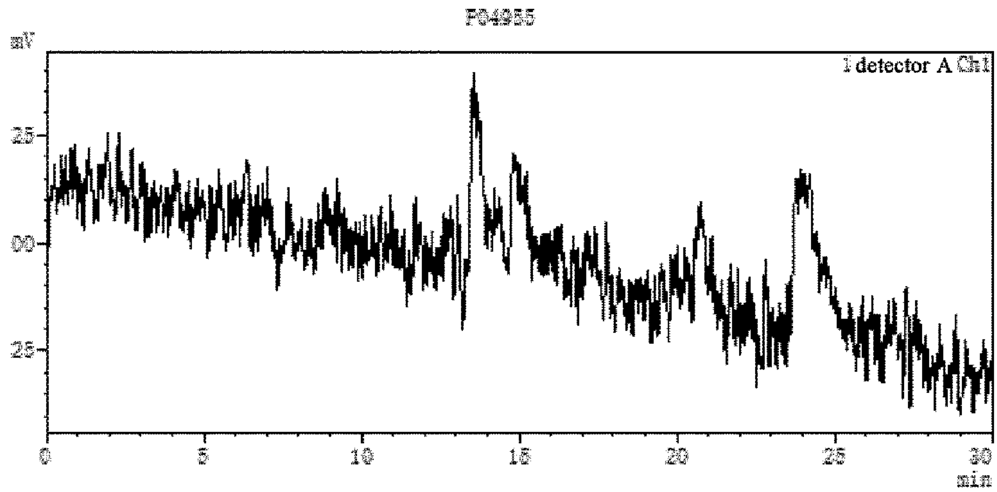


Figure 2F

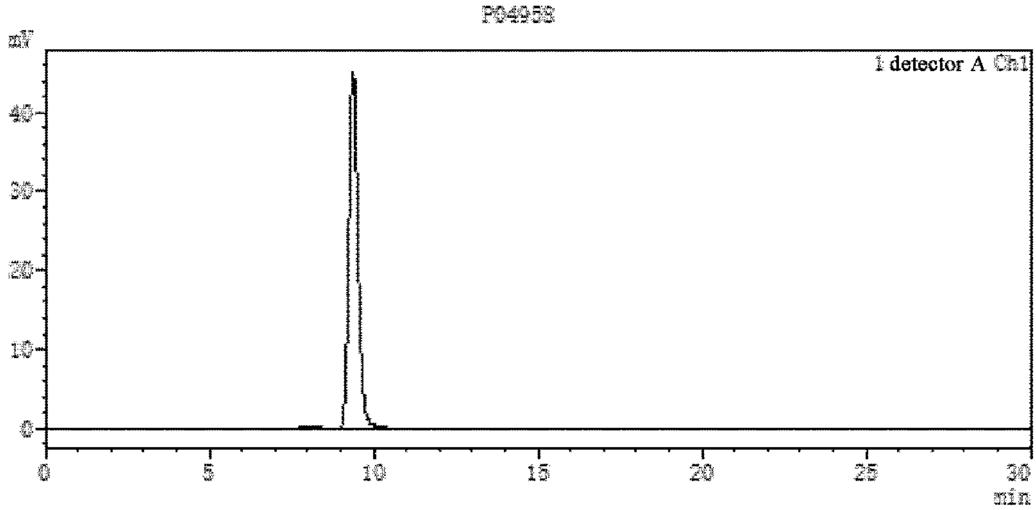


Figure 2G

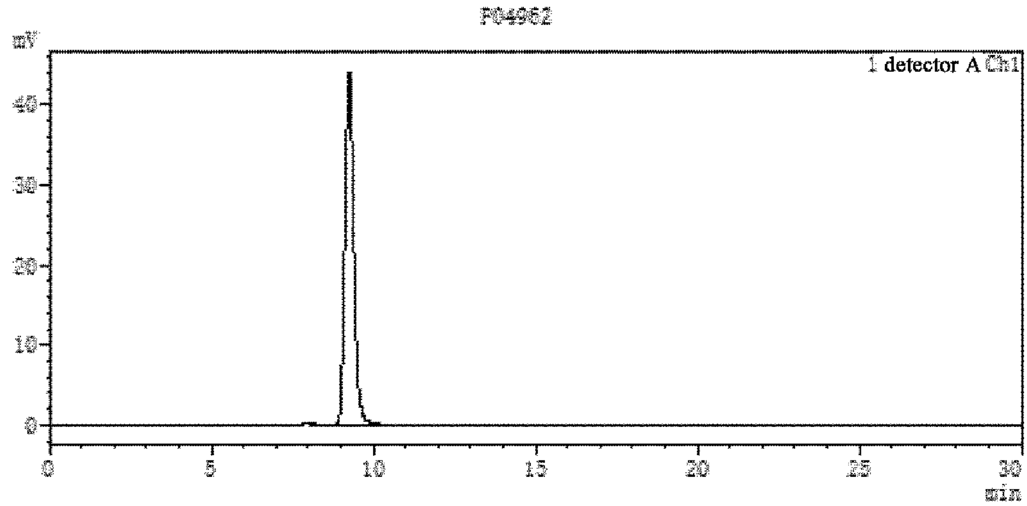


Figure 2H

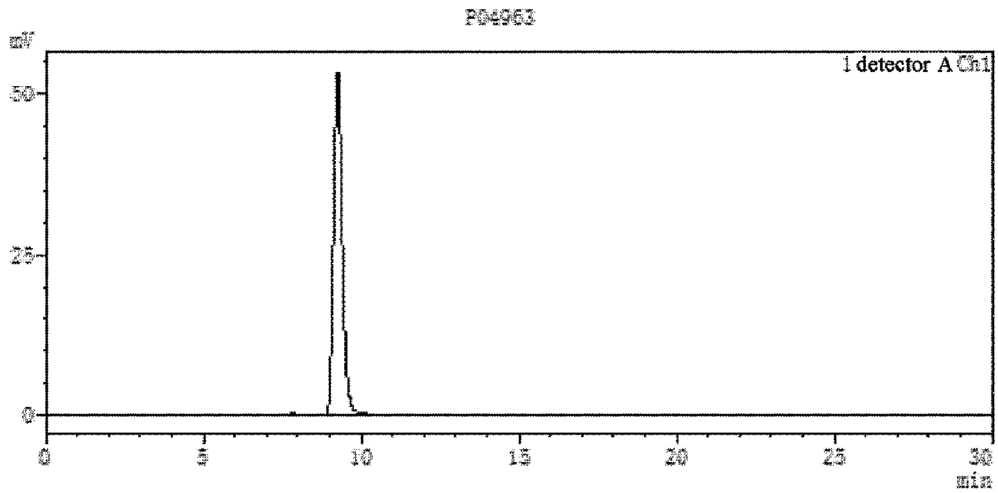


Figure 2I

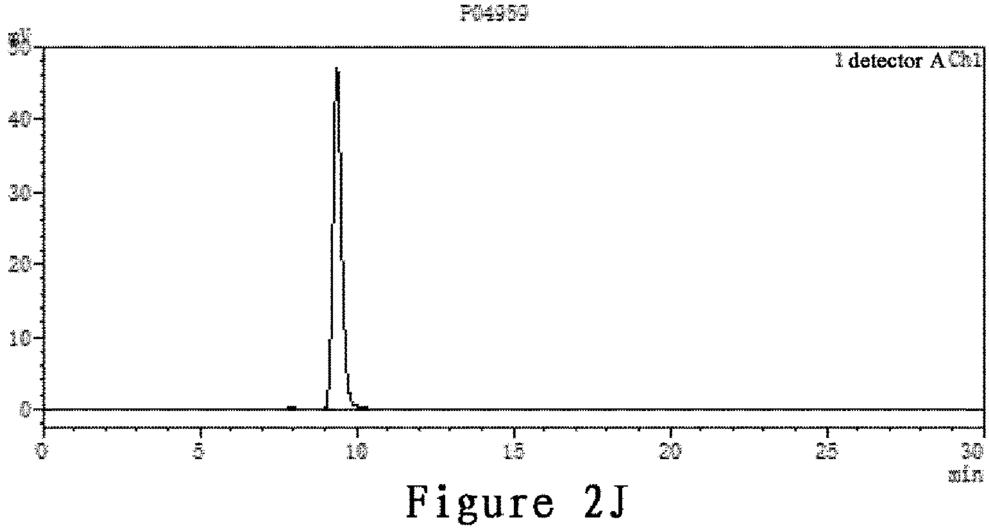


Figure 2J

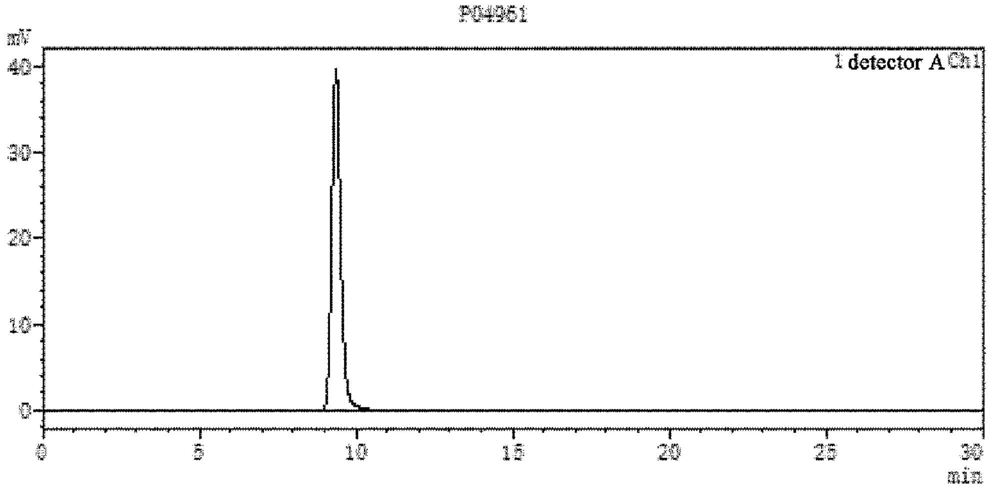


Figure 2K

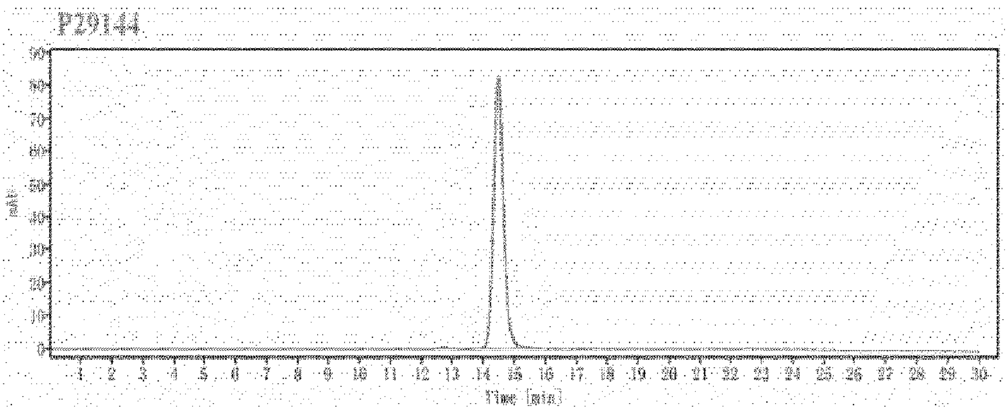


Figure 2L

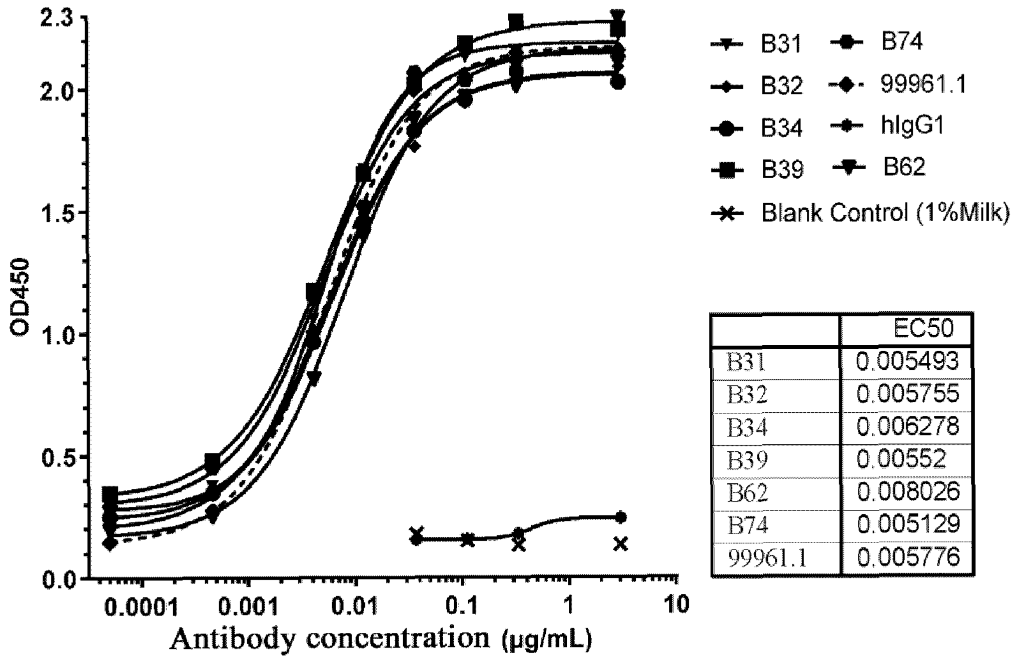


Figure 3A

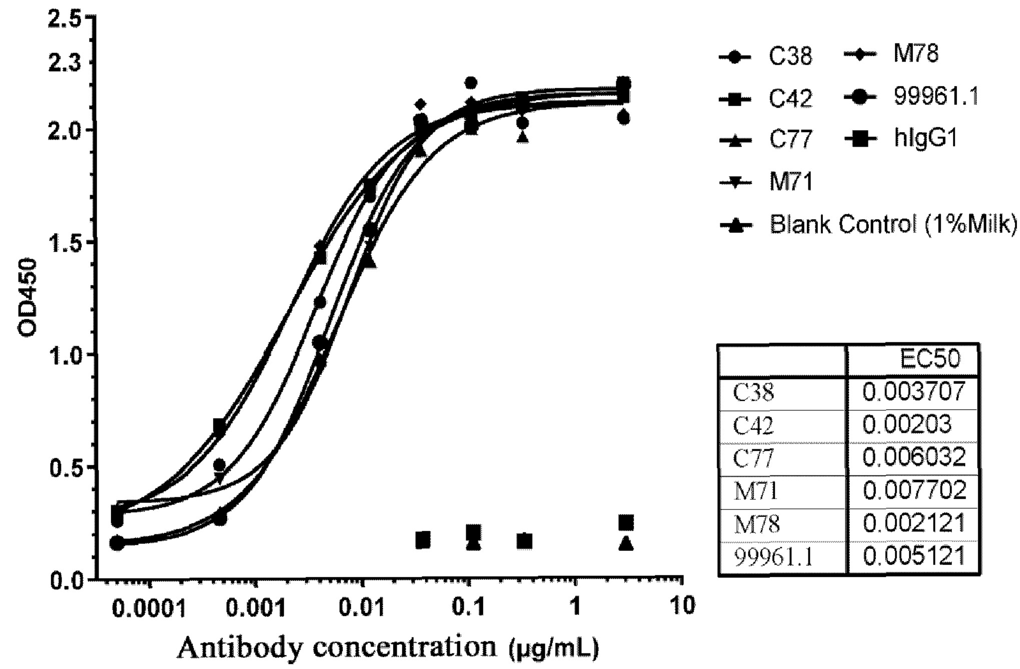


Figure 3B

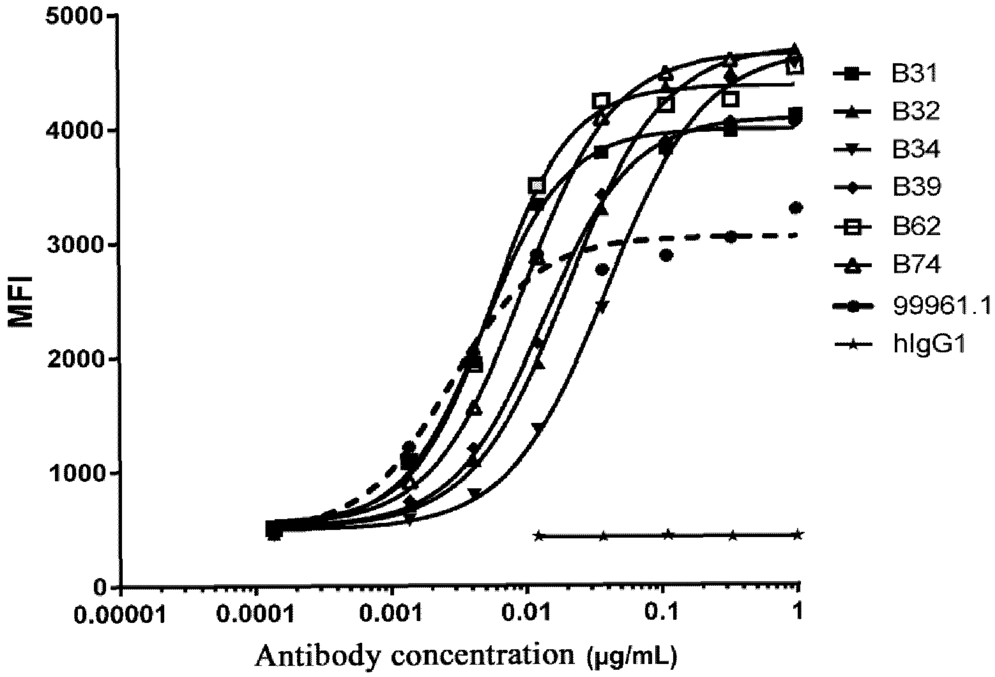


Figure 4A

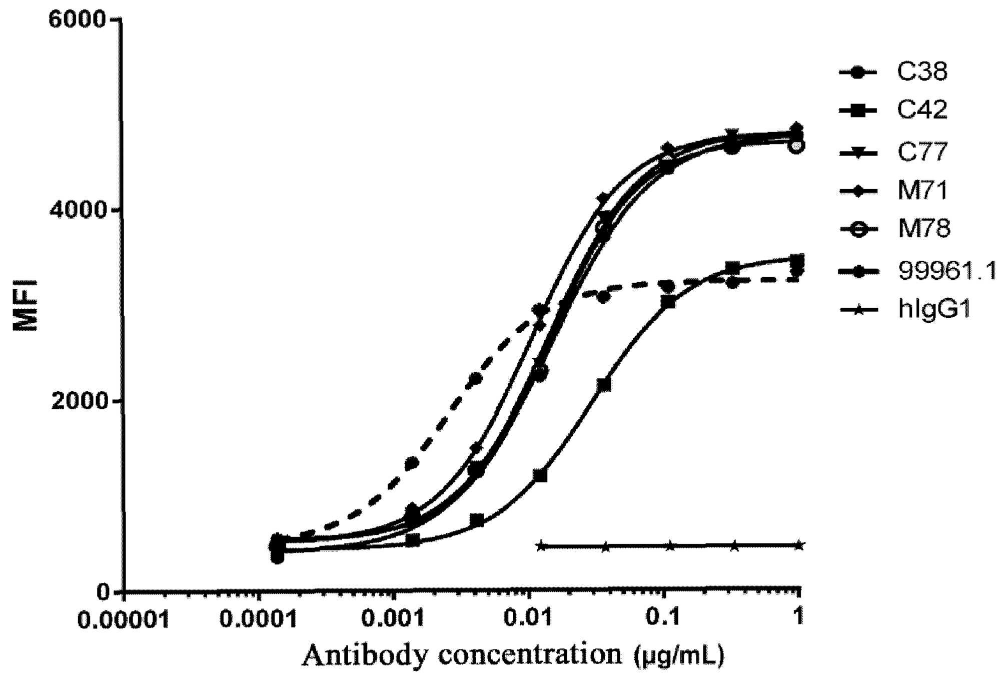


Figure 4B

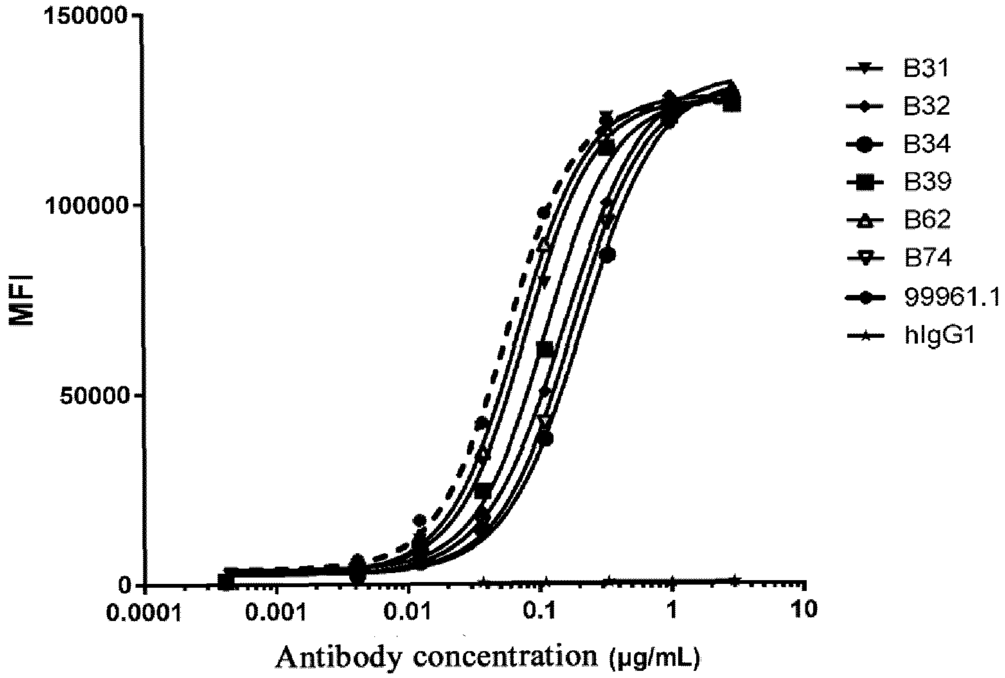


Figure 5A

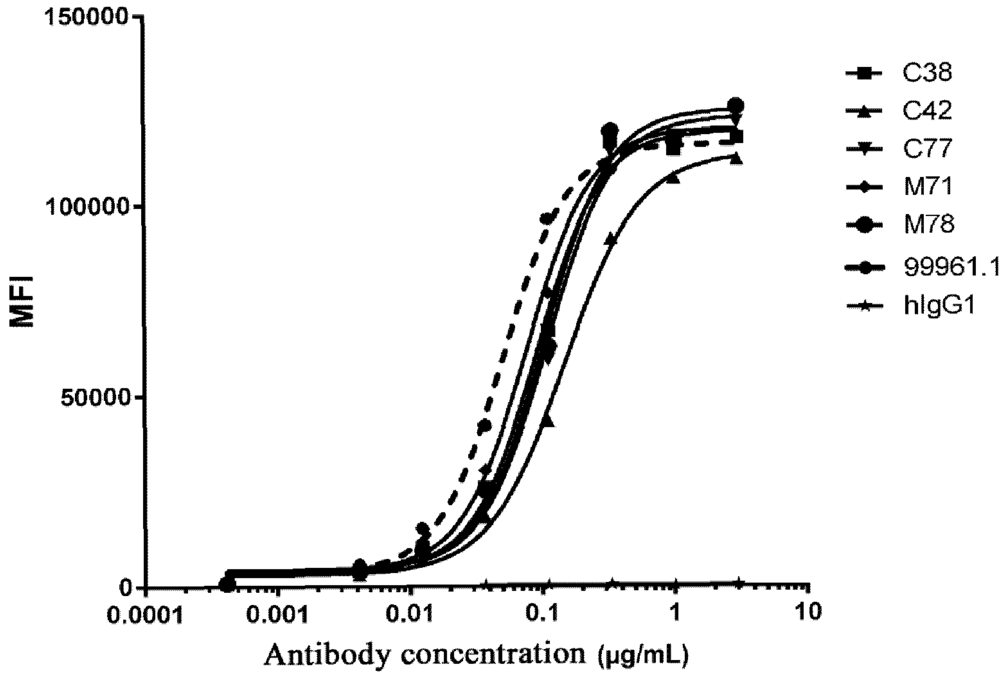


Figure 5B

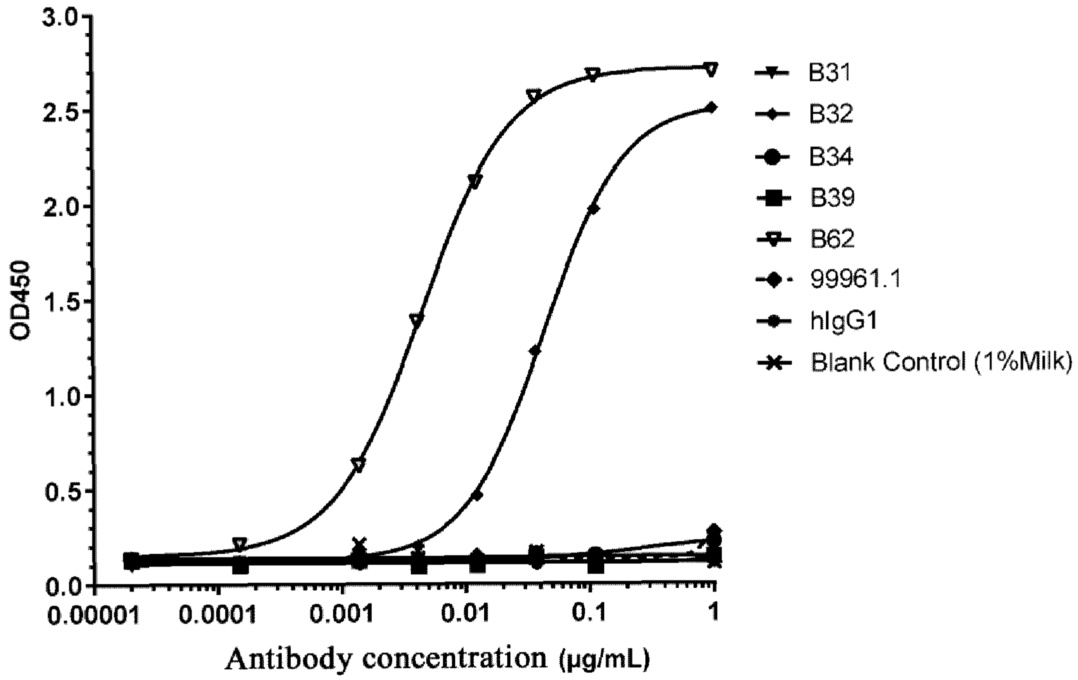


Figure 6A

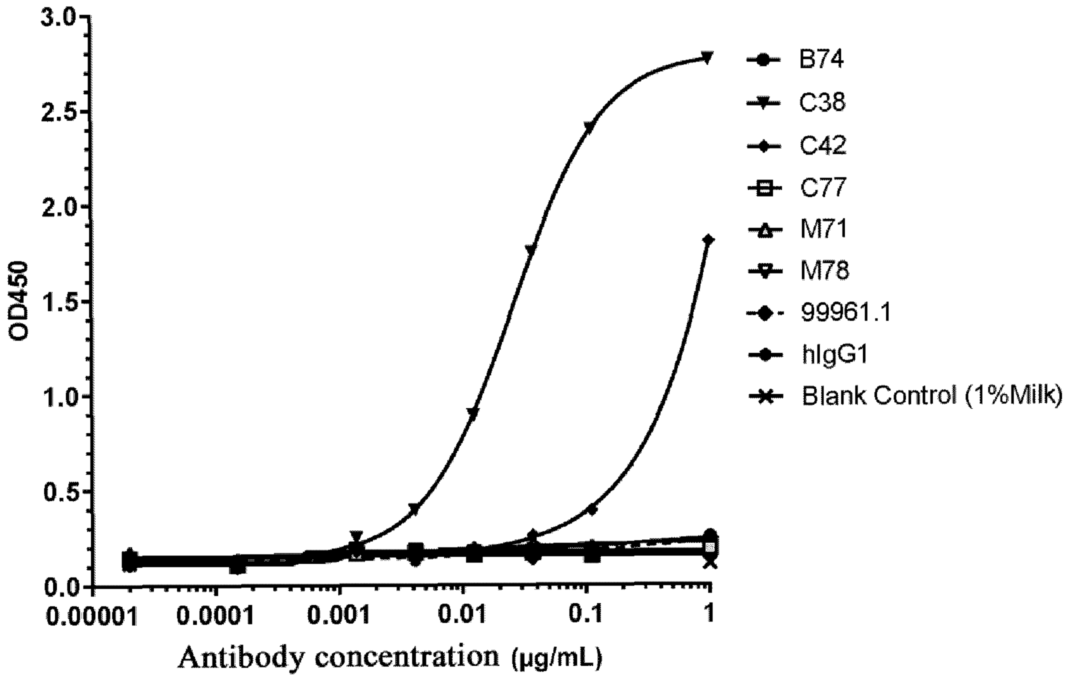


Figure 6B

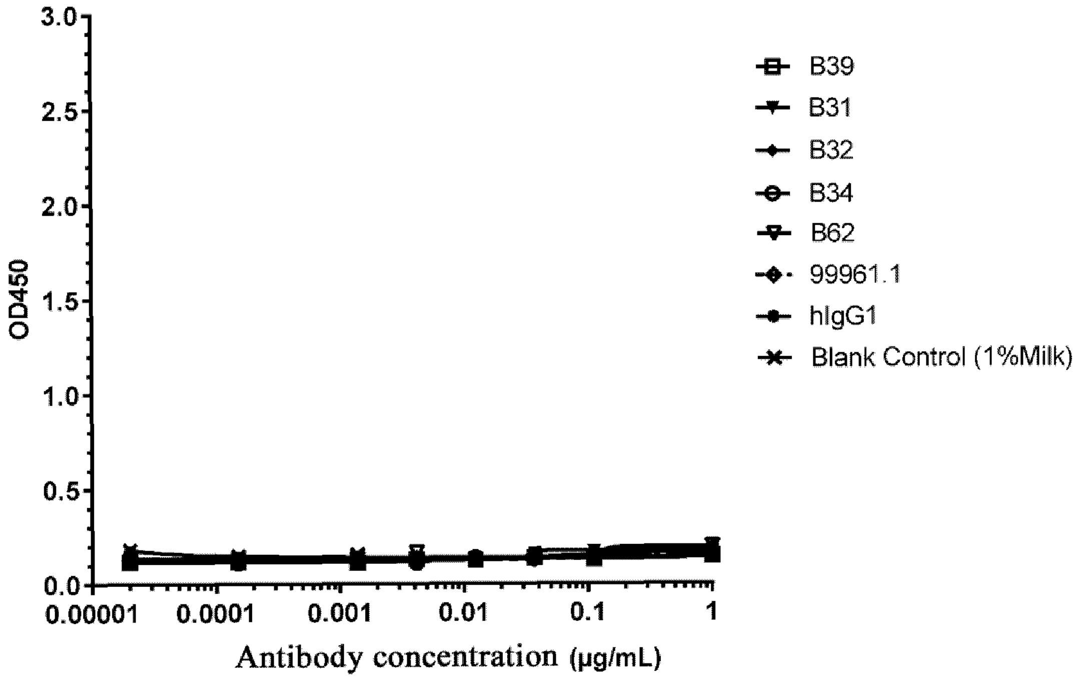


Figure 7A

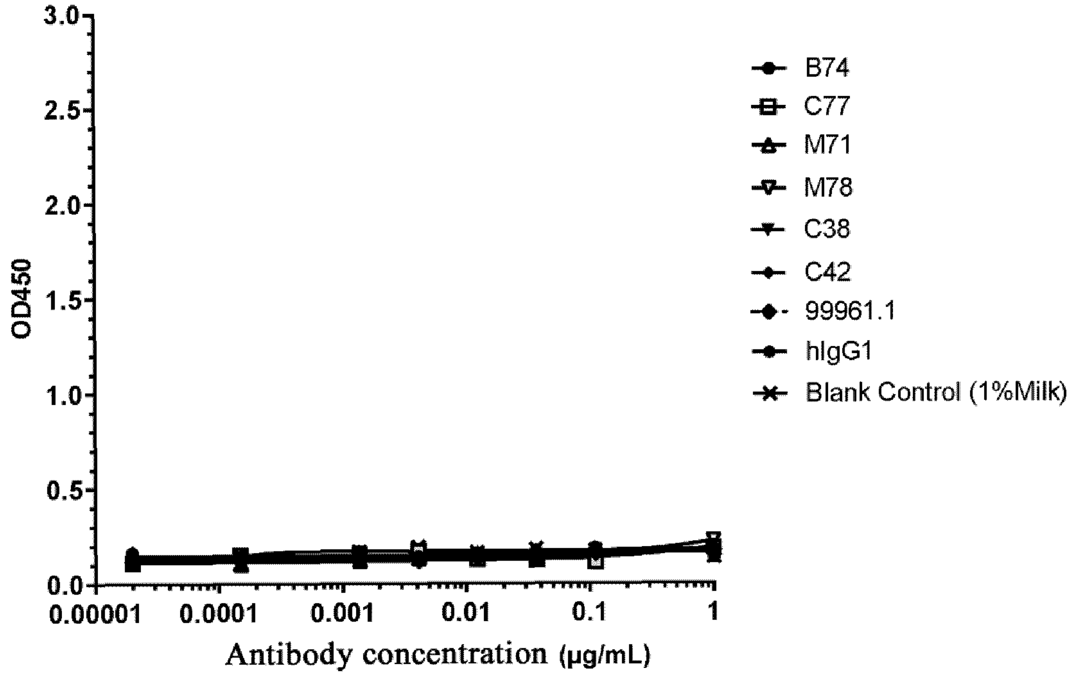


Figure 7B

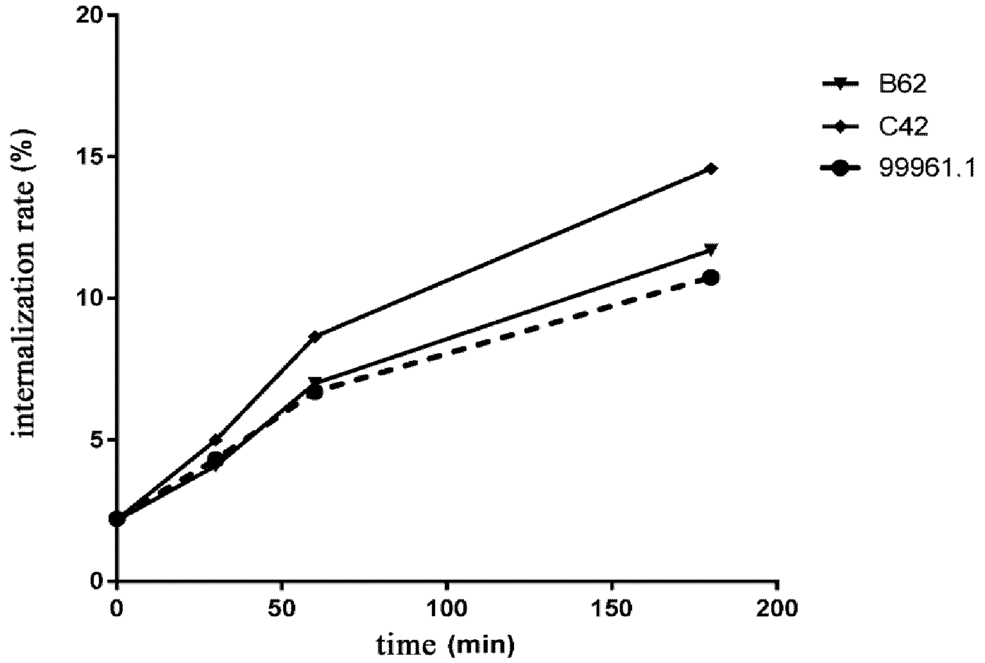


Figure 8A

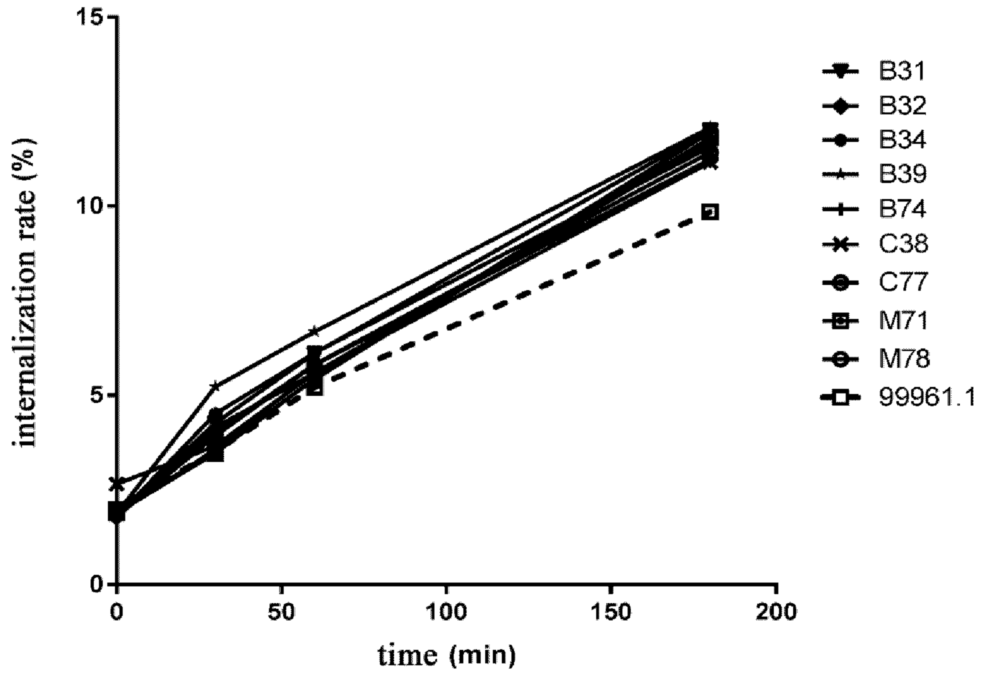


Figure 8B

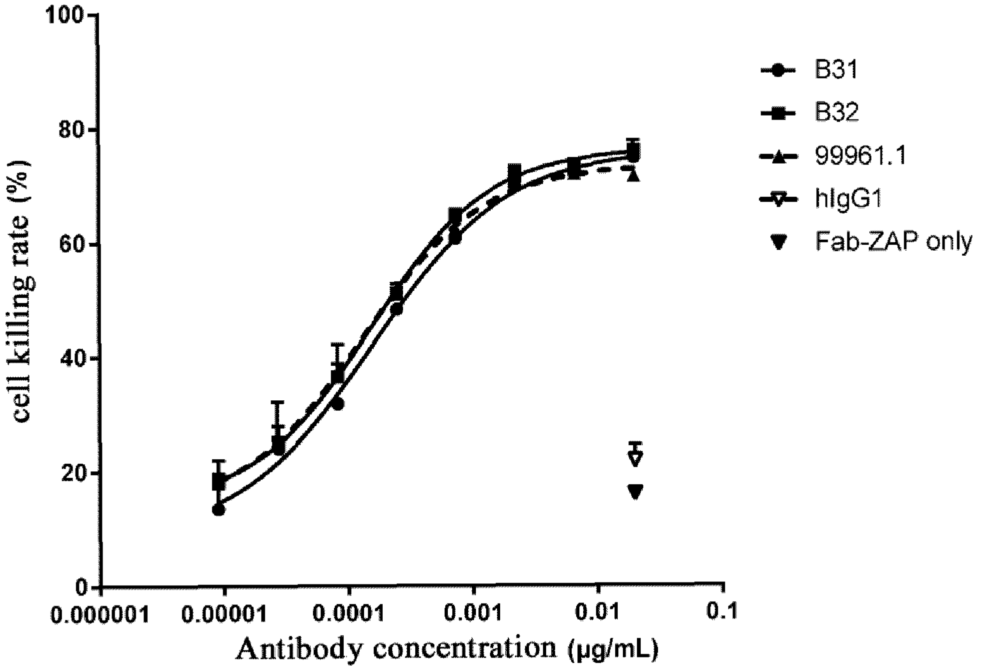


Figure 9A

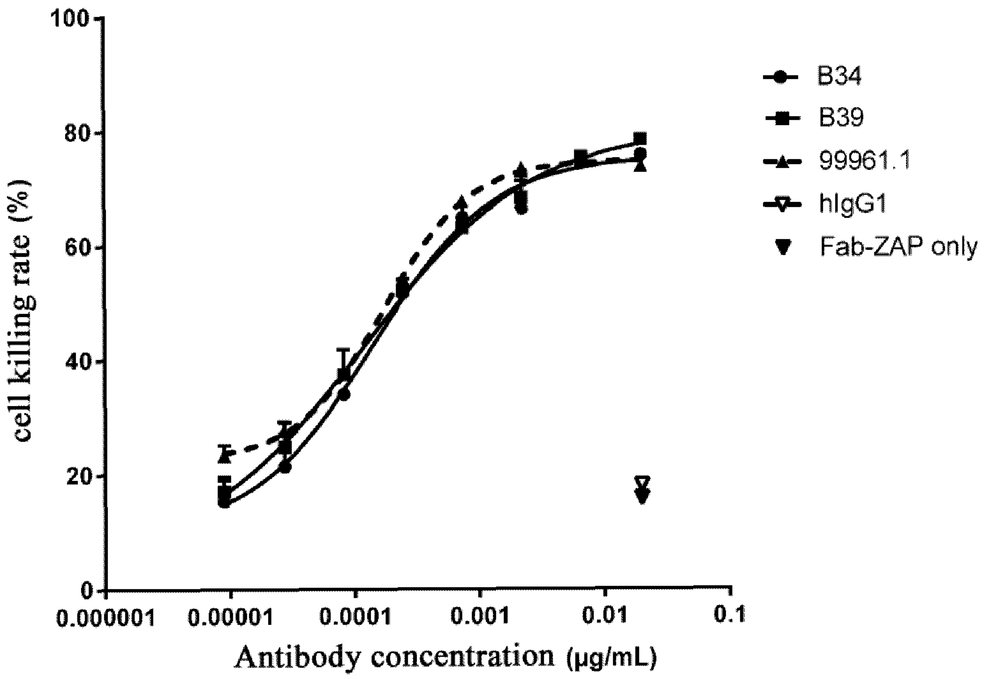


Figure 9B

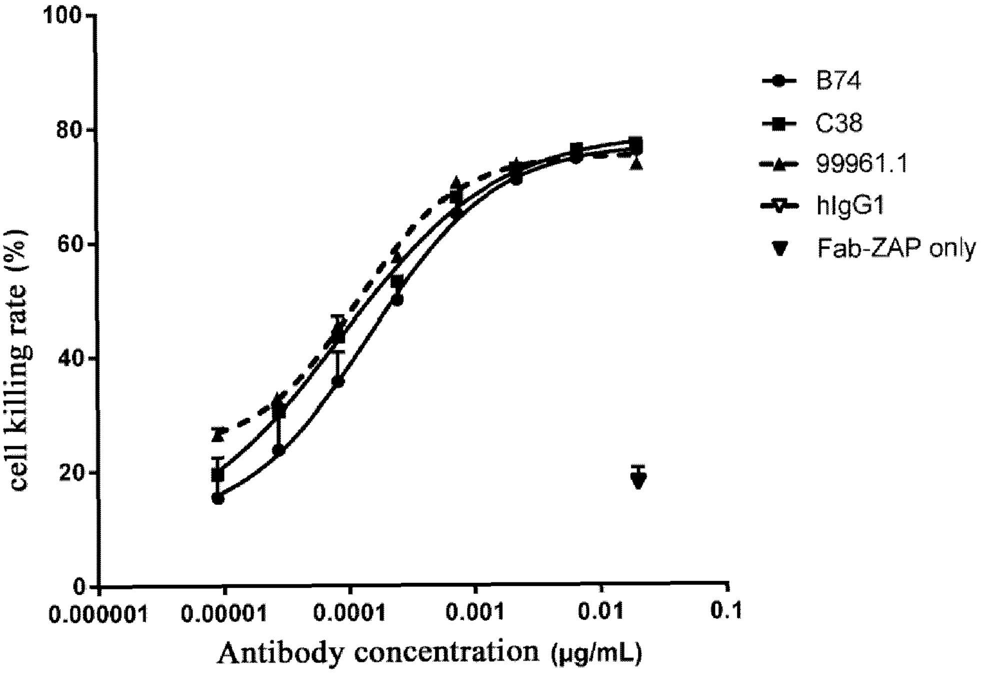


Figure 9C

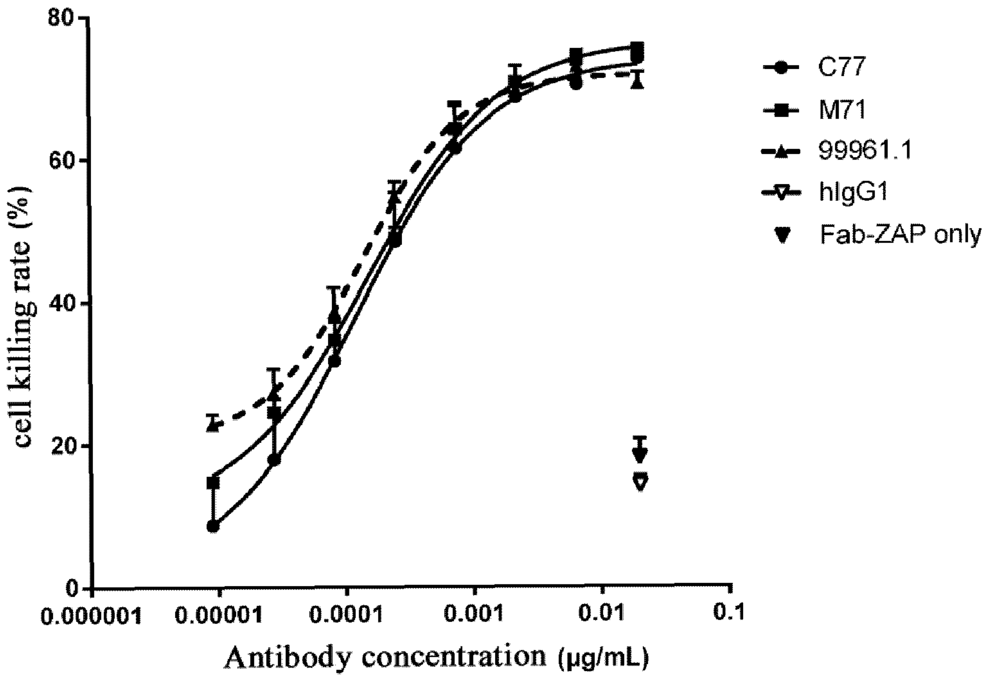


Figure 9D

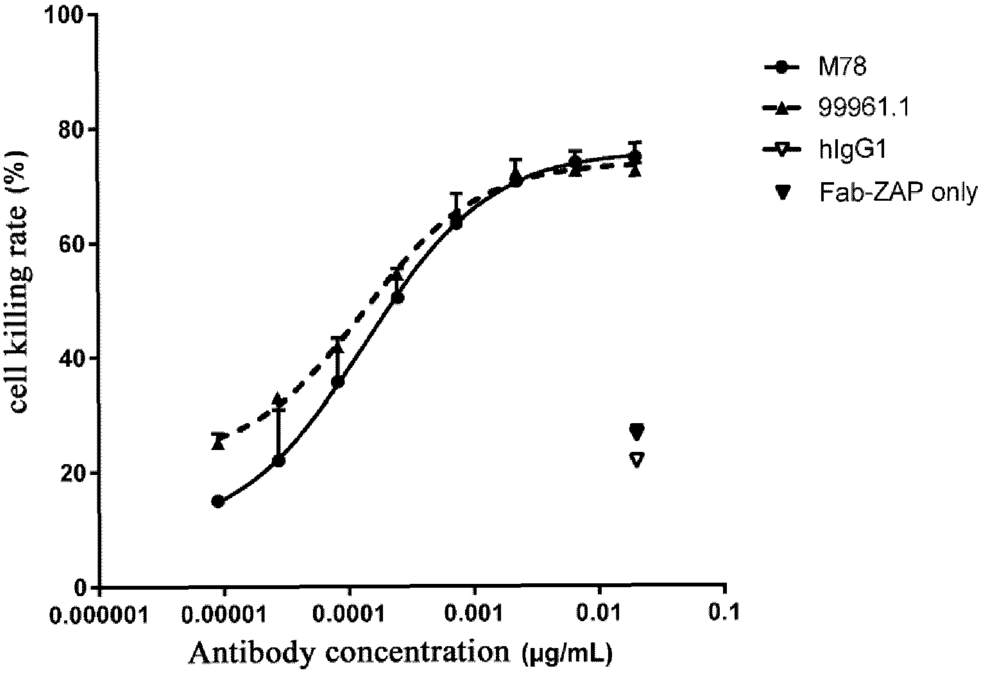


Figure 9E

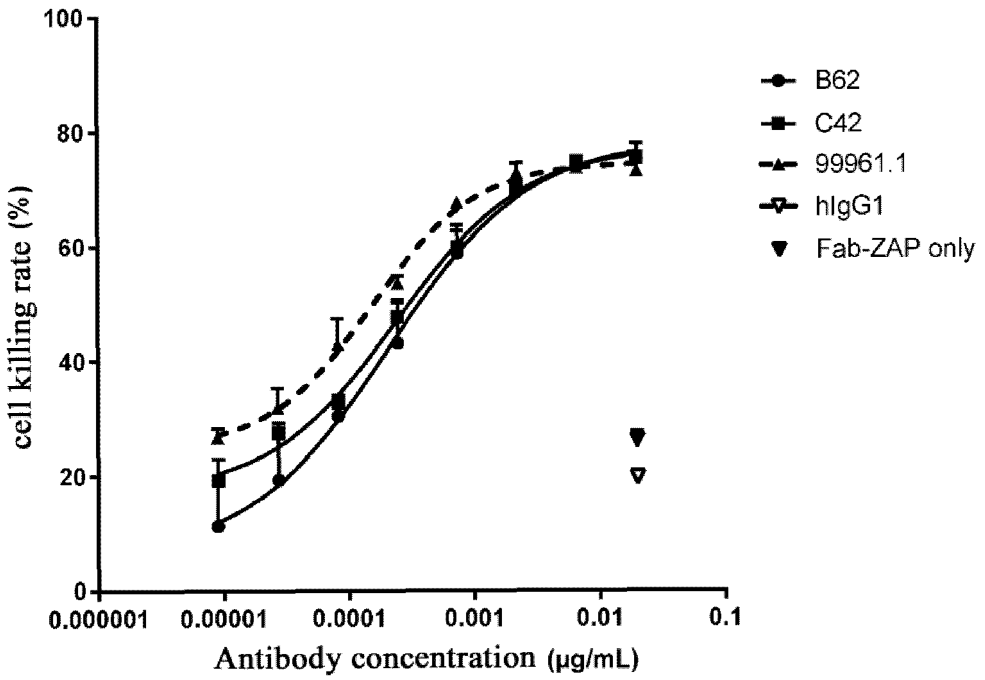


Figure 9F

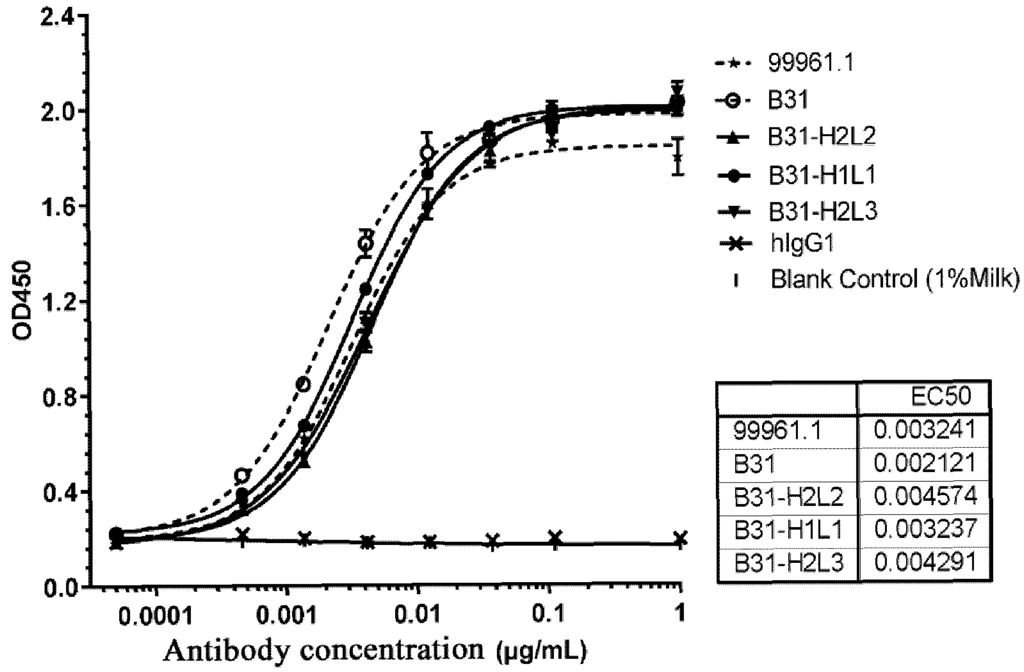


| | EC50 |
|---|---|
| 99961.1 | 0.003241 |
| B31 | 0.002121 |
| B31-H2L2 | 0.004574 |
| B31-H1L1 | 0.003237 |
| B31-H2L3 | 0.004291 |

Figure 10A

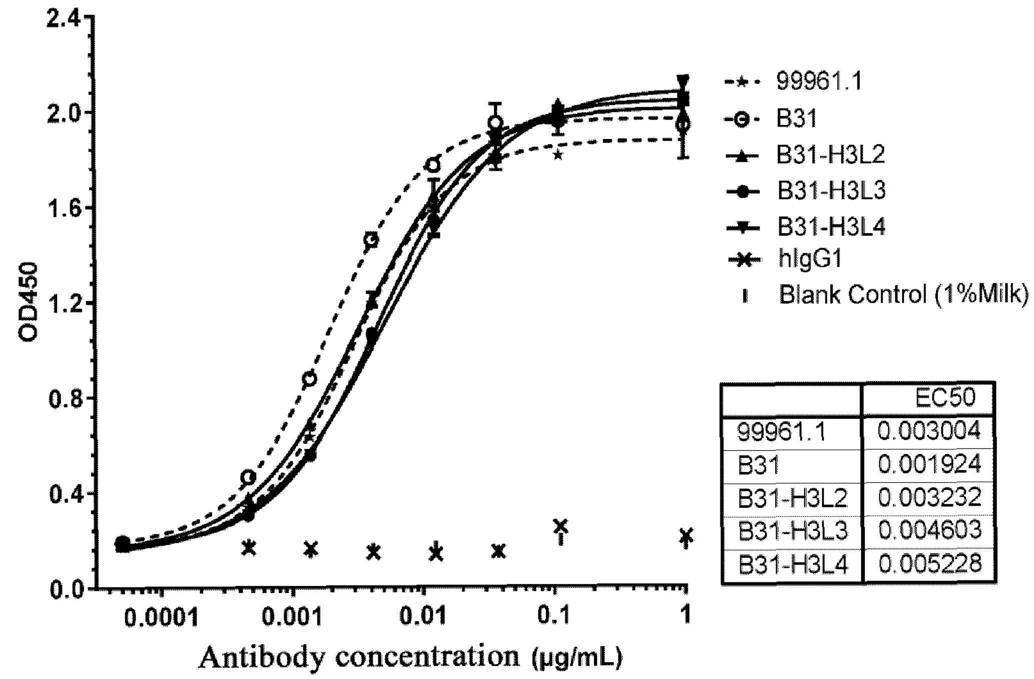


| | EC50 |
|---|---|
| 99961.1 | 0.003004 |
| B31 | 0.001924 |
| B31-H3L2 | 0.003232 |
| B31-H3L3 | 0.004603 |
| B31-H3L4 | 0.005228 |

Figure 10B

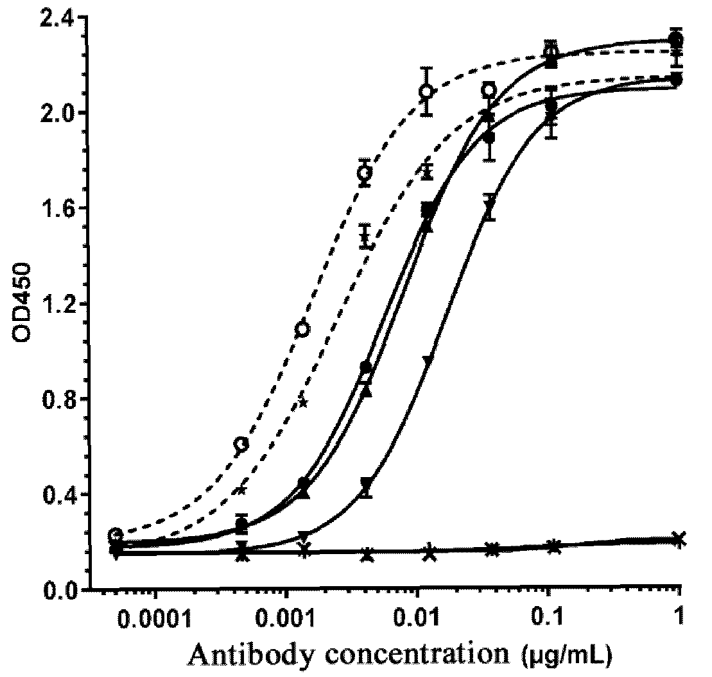


| | EC50 |
|---|---|
| 99961.1 | 0.002559 |
| B31 | 0.001629 |
| B31-H4L2 | 0.008115 |
| B31-H4L3 | 0.005743 |
| B31-H4L4 | 0.01712 |

Figure 10C

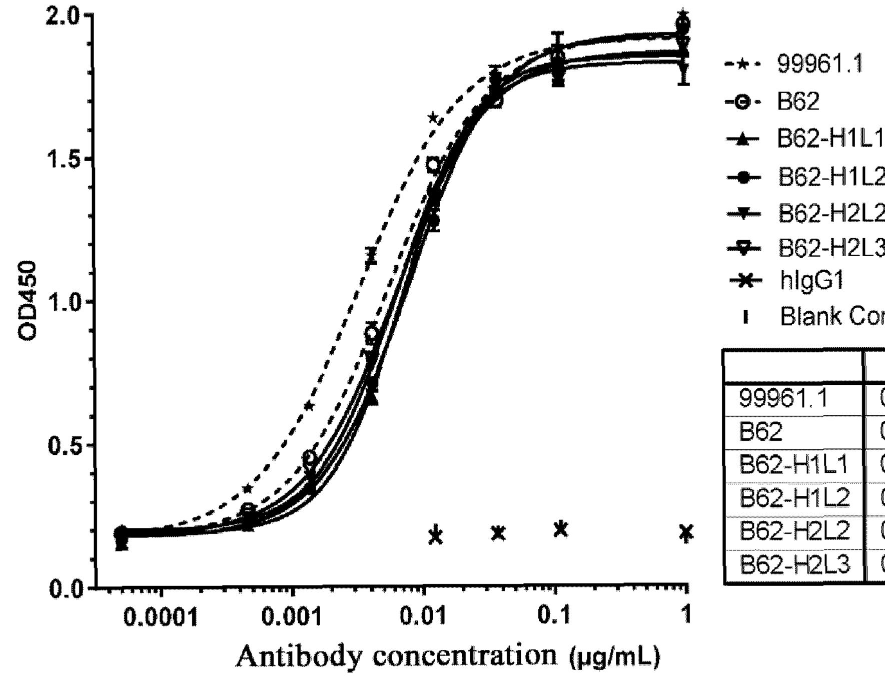


| | EC50 |
|---|---|
| 99961.1 | 0.003303 |
| B62 | 0.00558 |
| B62-H1L1 | 0.0071 |
| B62-H1L2 | 0.007875 |
| B62-H2L2 | 0.00627 |
| B62-H2L3 | 0.006299 |

Figure 10D

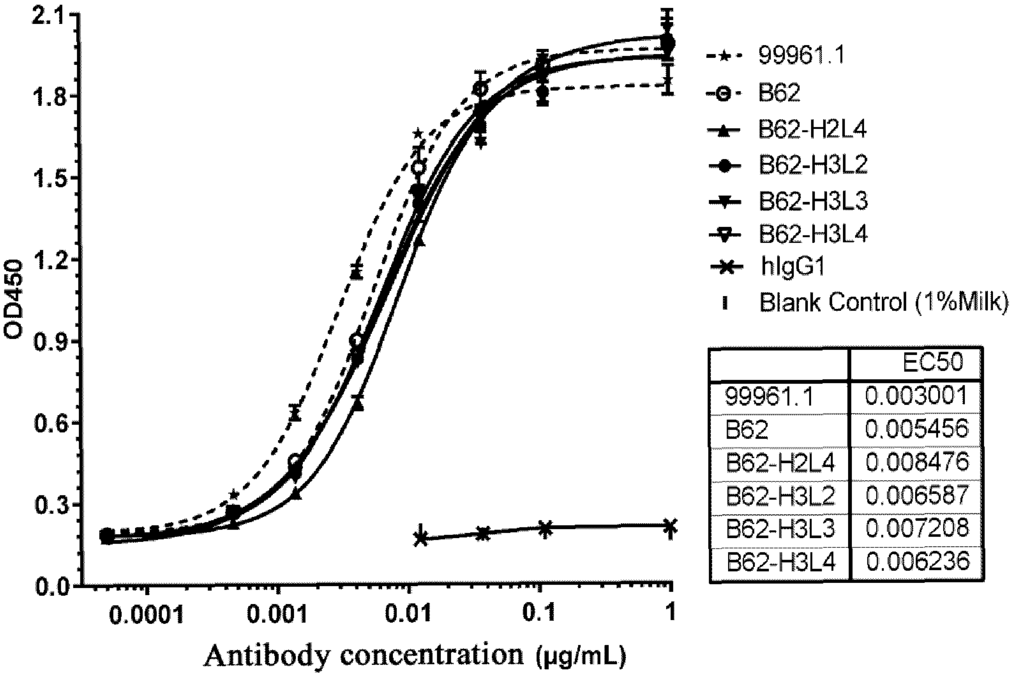


| | EC50 |
|---|---|
| 99961.1 | 0.003001 |
| B62 | 0.005456 |
| B62-H2L4 | 0.008476 |
| B62-H3L2 | 0.006587 |
| B62-H3L3 | 0.007208 |
| B62-H3L4 | 0.006236 |

Figure 10E

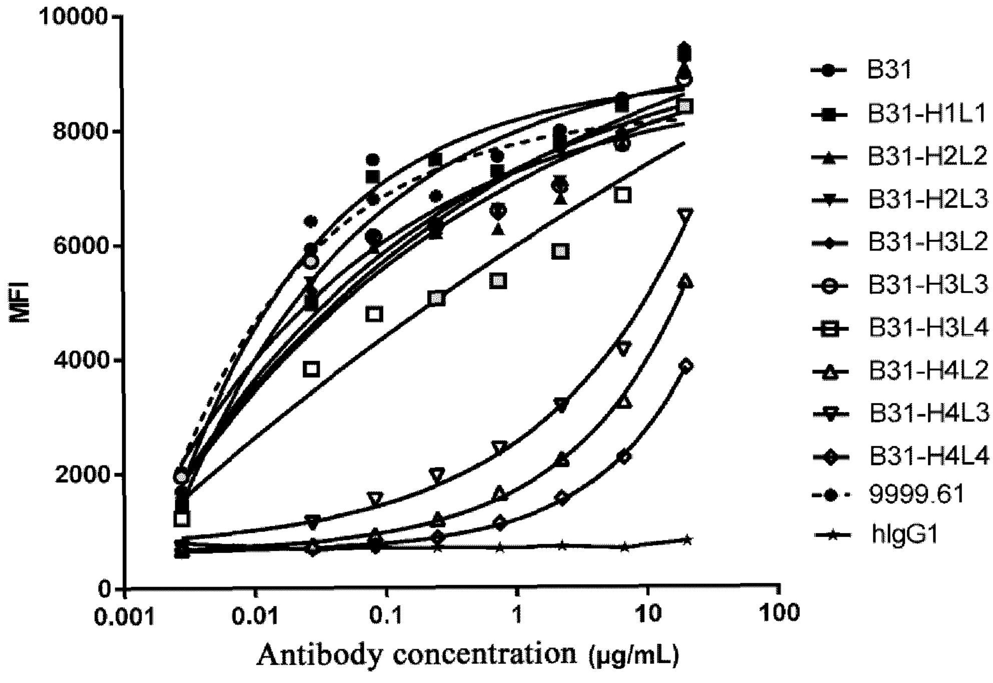


Figure 11A

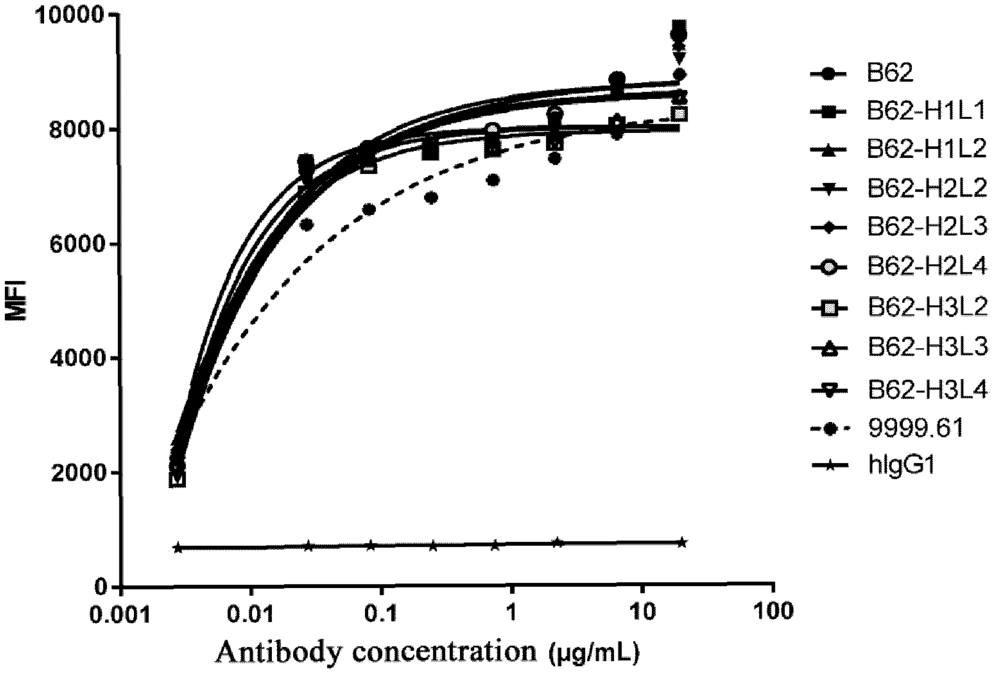


Figure 11B

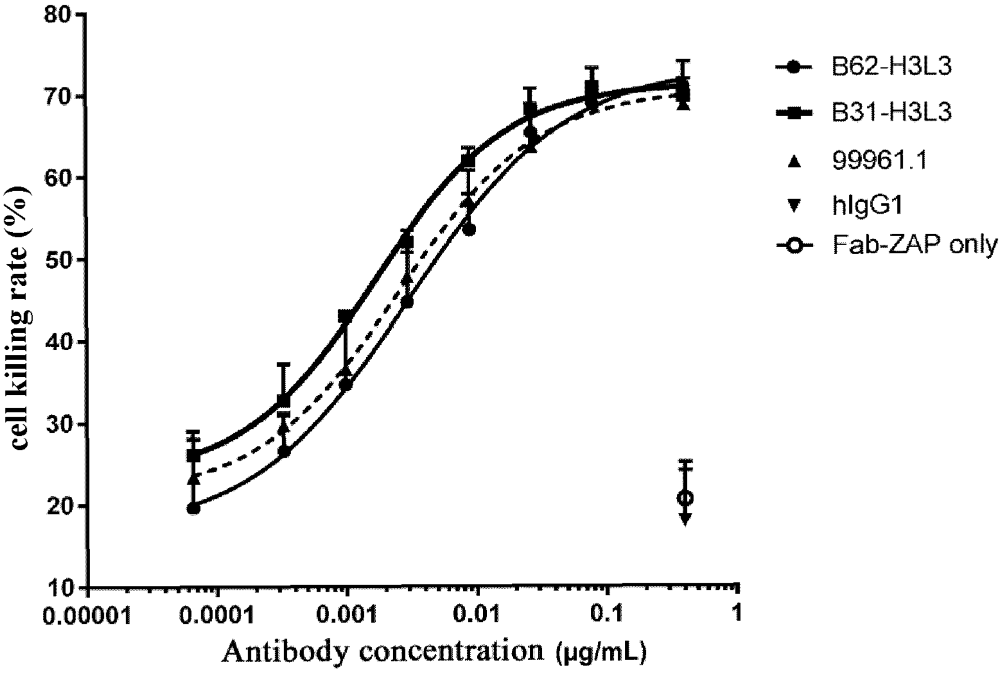


Figure 12

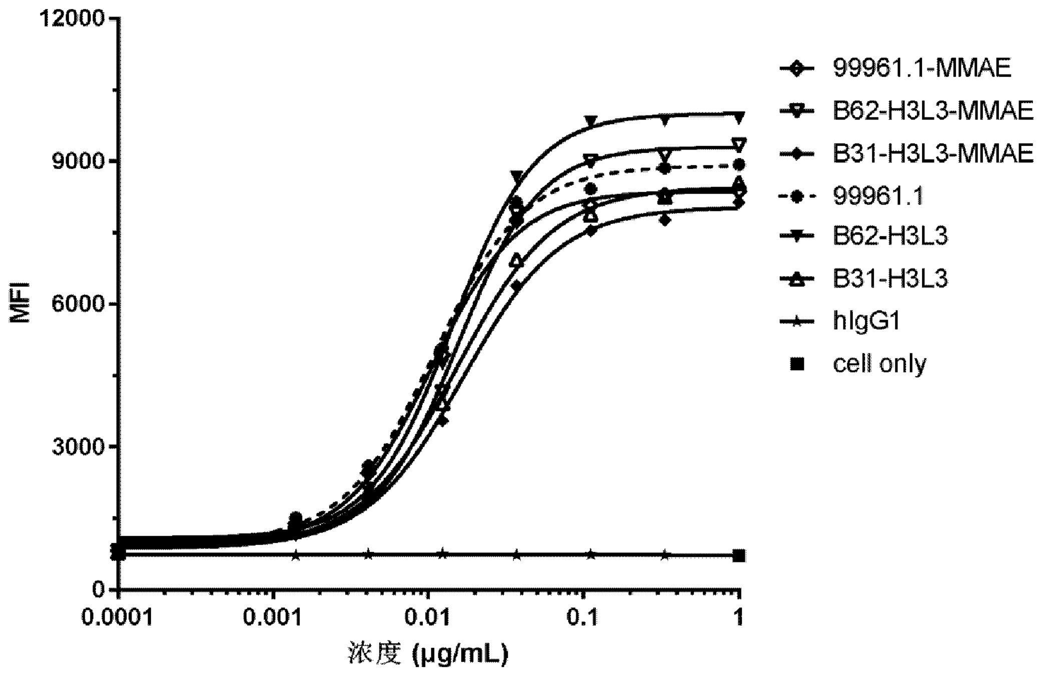


Figure 13A

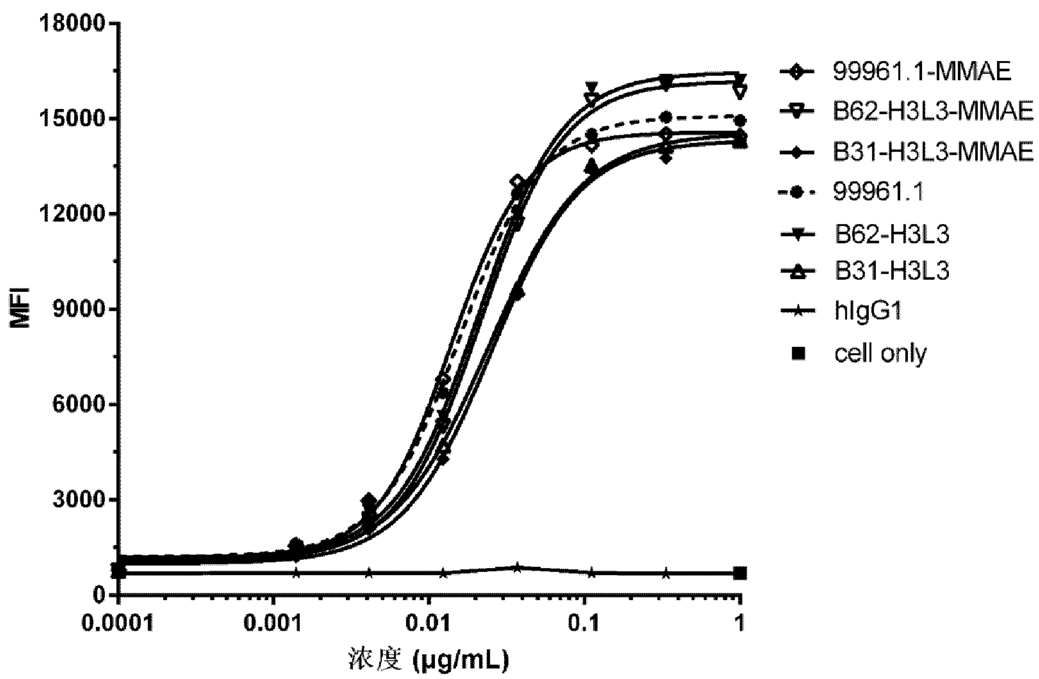


Figure 13B

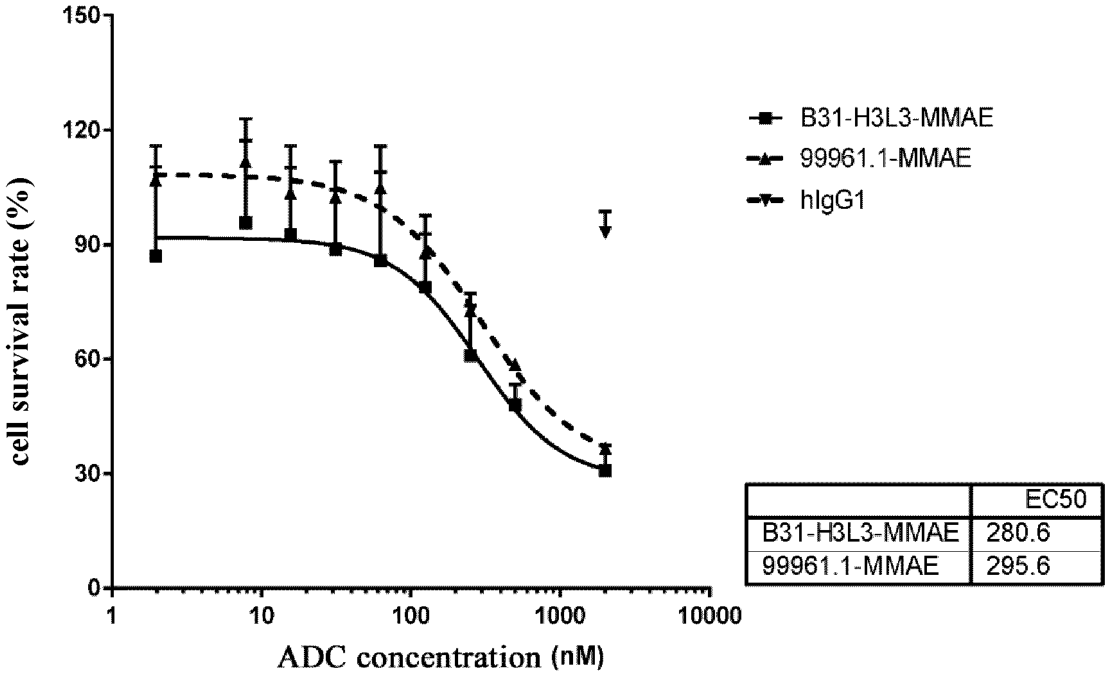


Figure 14A

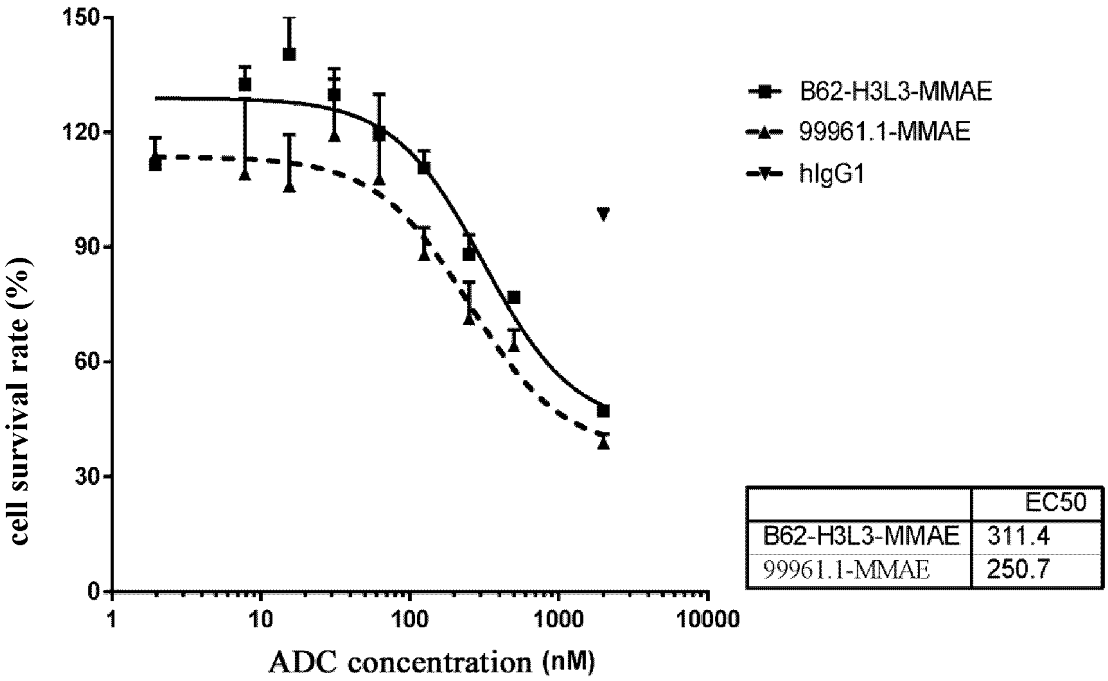


Figure 14B

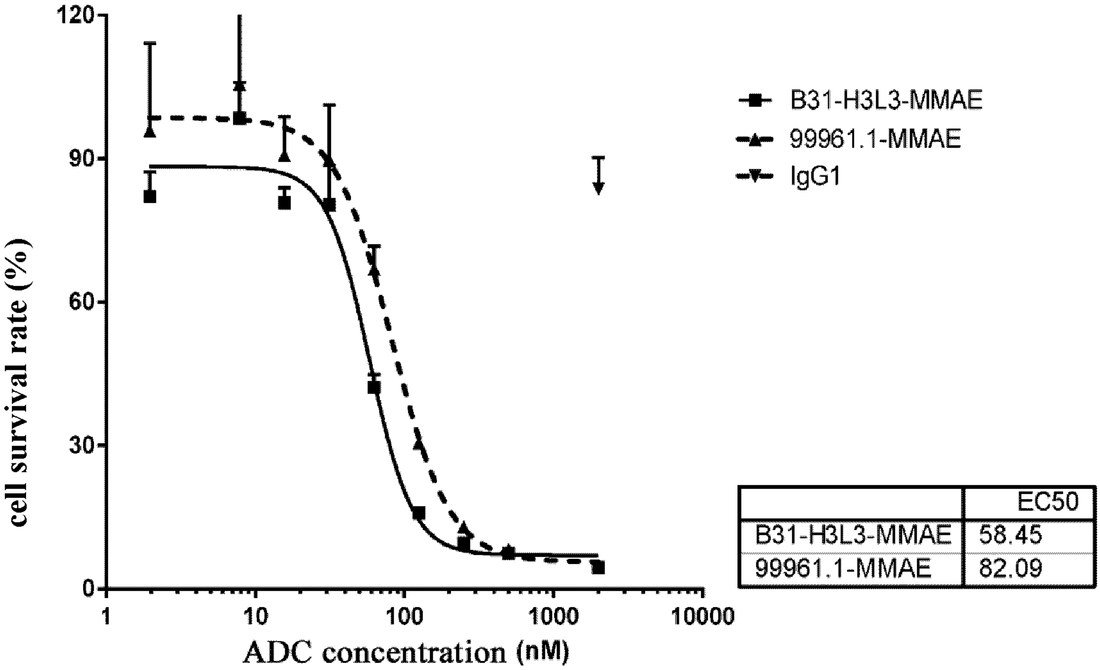


Figure 14C

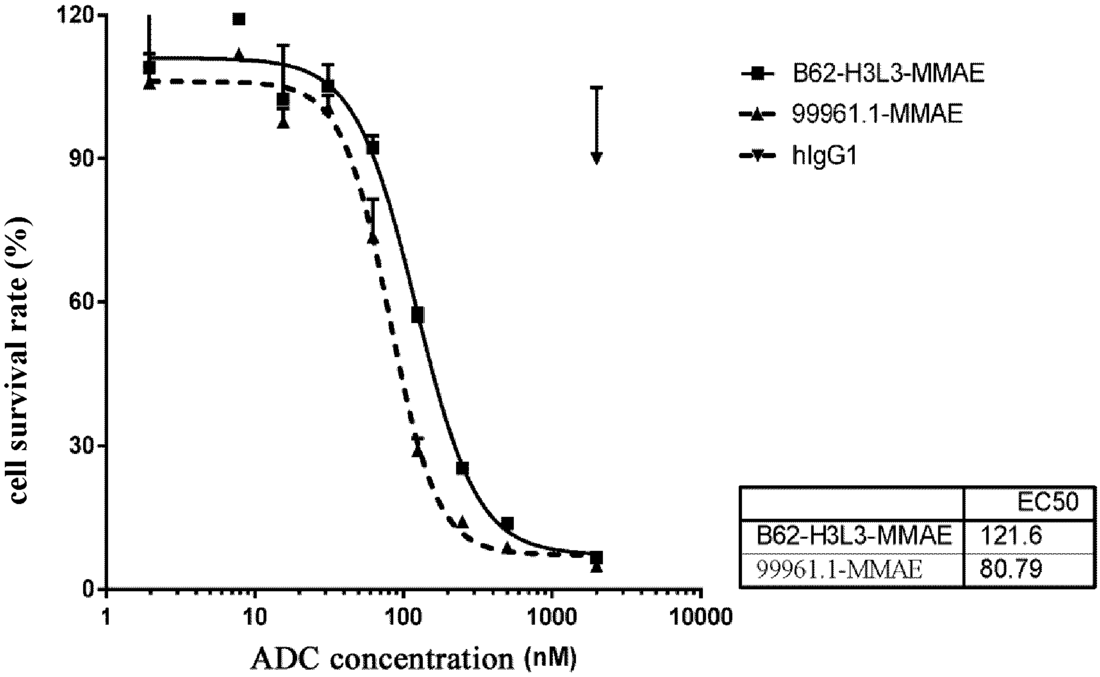


Figure 14D

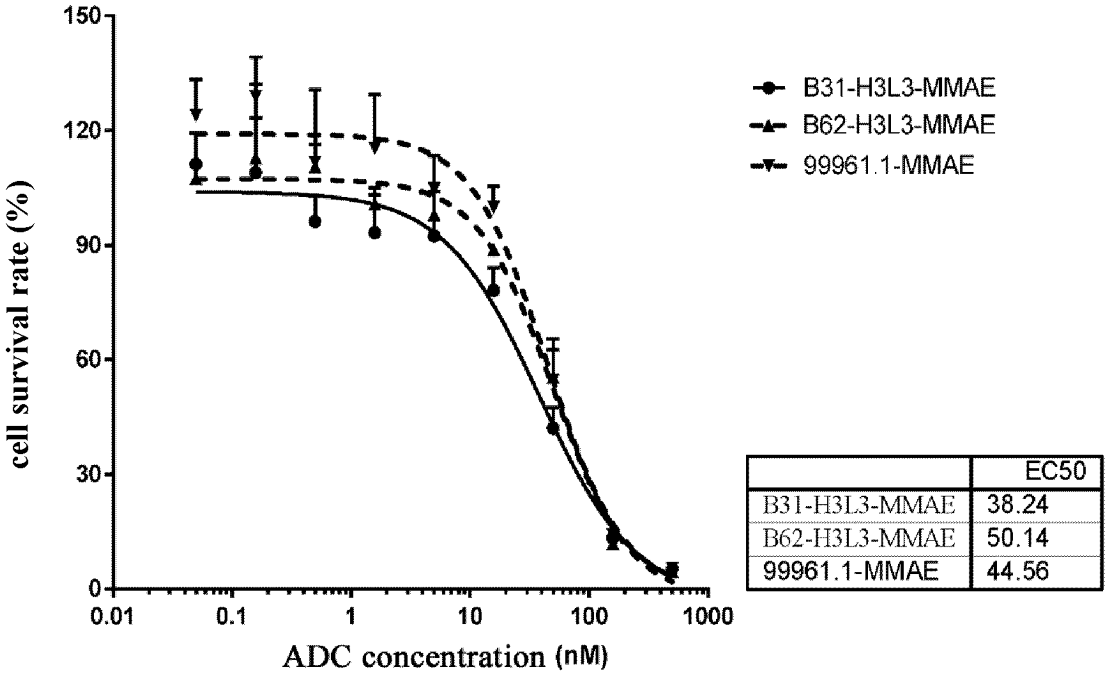


Figure 15A

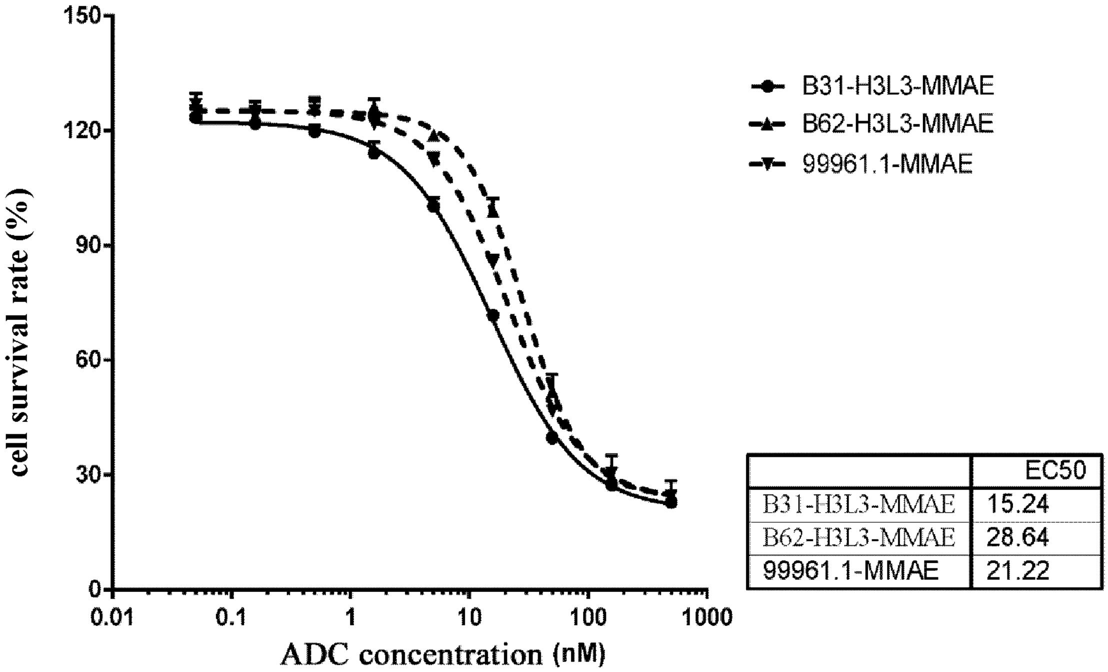


Figure 15B

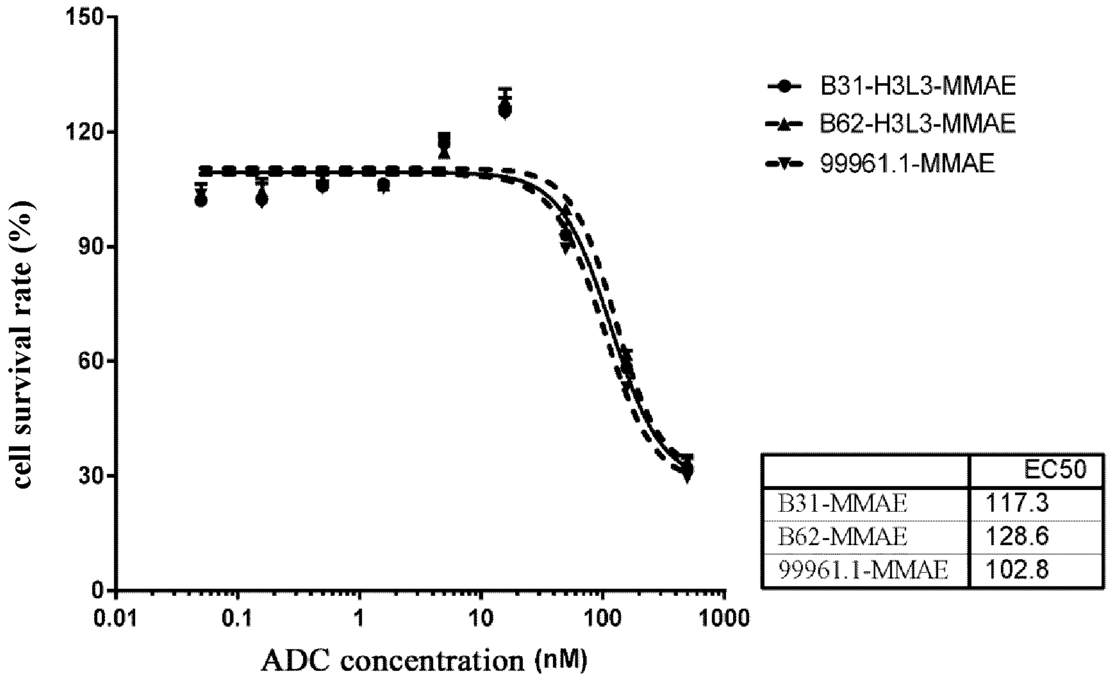


| | EC50 |
|---|---|
| B31-MMAE | 117.3 |
| B62-MMAE | 128.6 |
| 99961.1-MMAE | 102.8 |

Figure 15C

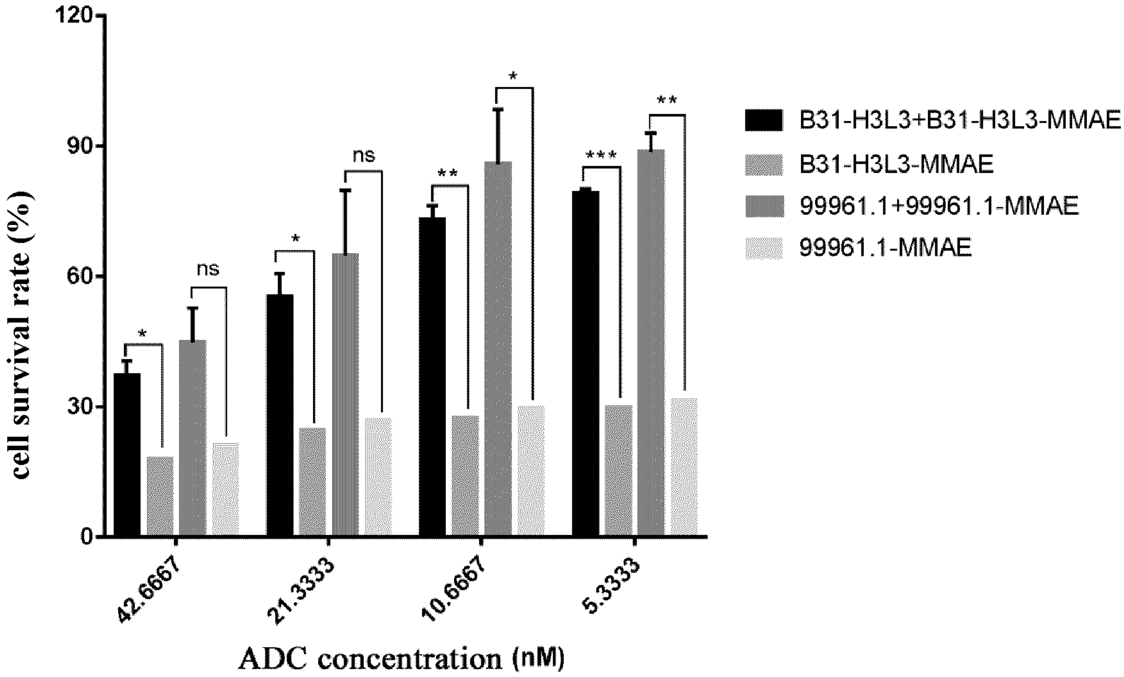


Figure 16

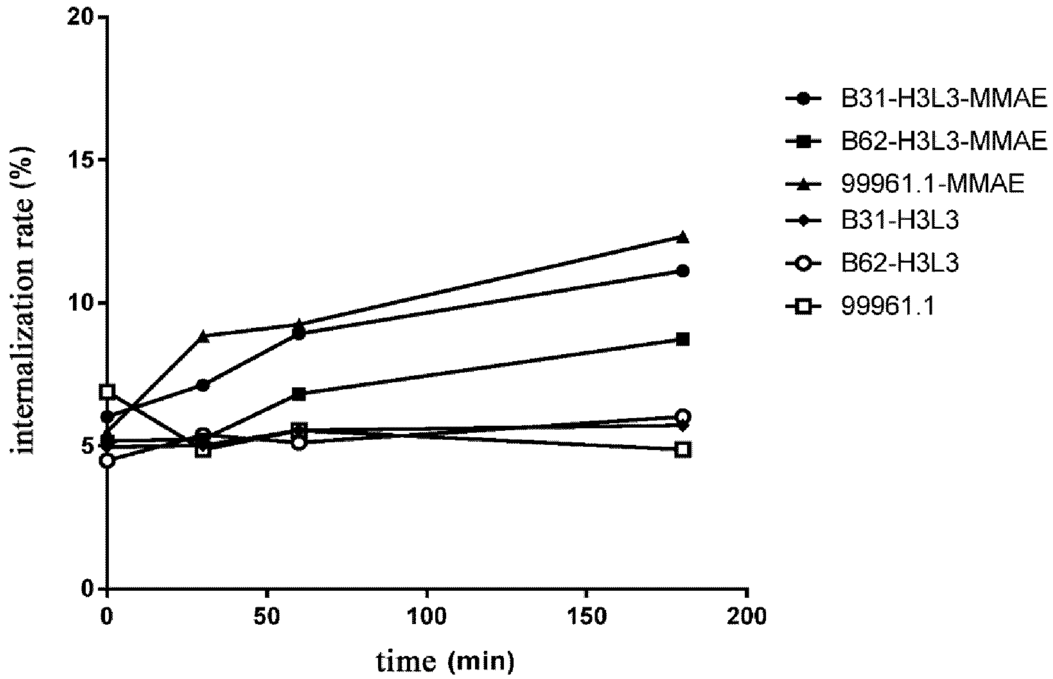


Figure 17A

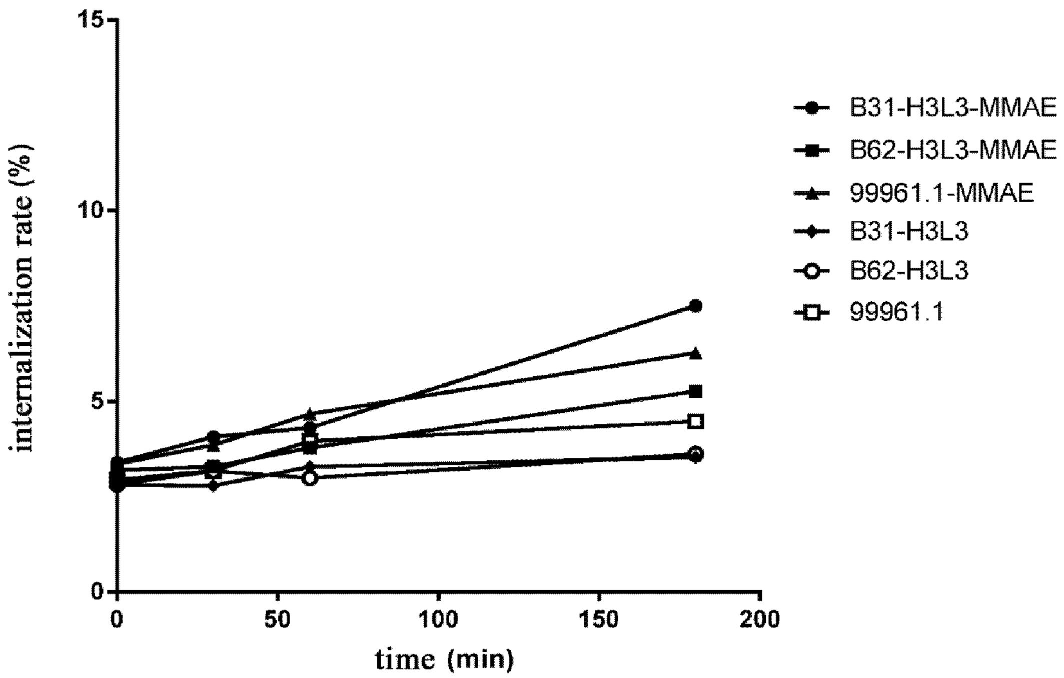


Figure 17B

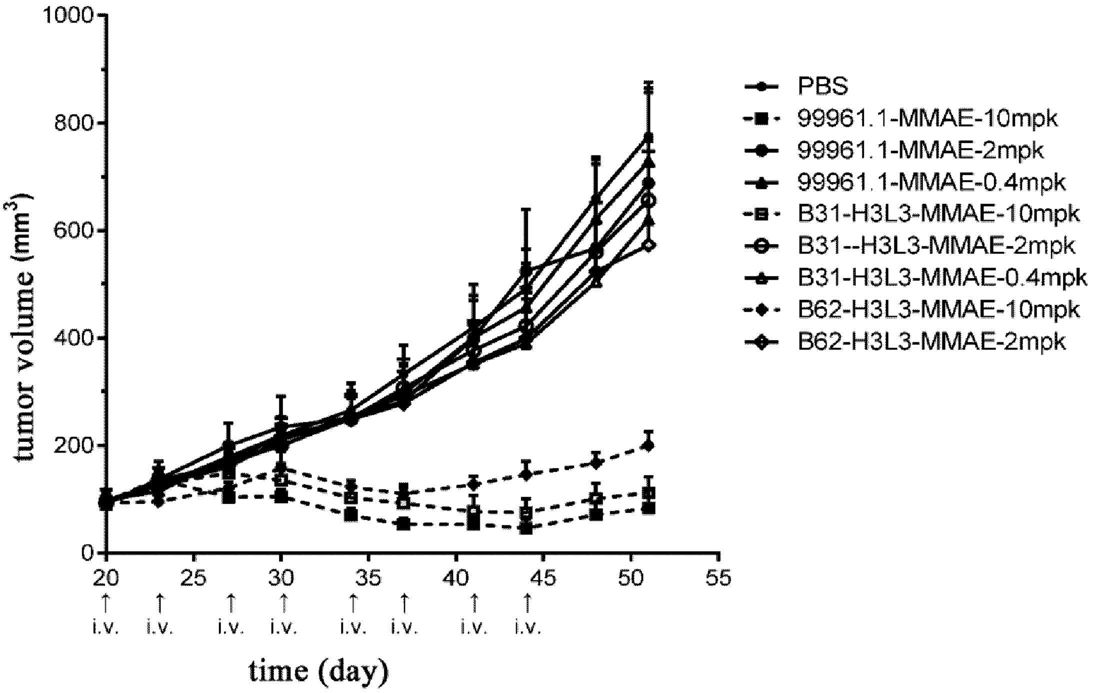


Figure 18A

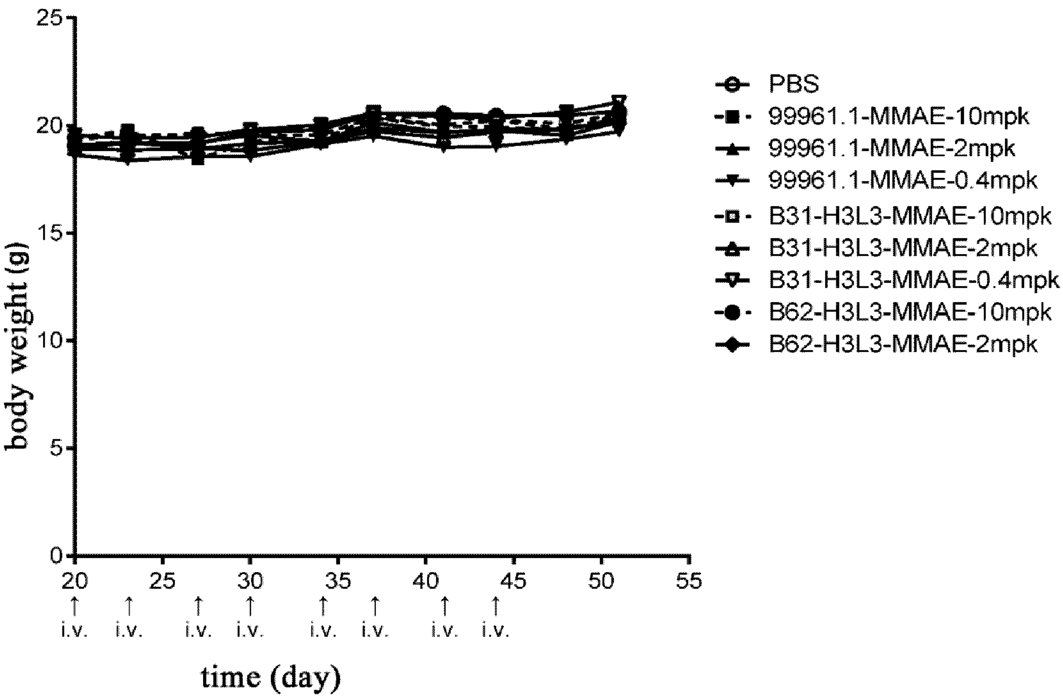


Figure 18B

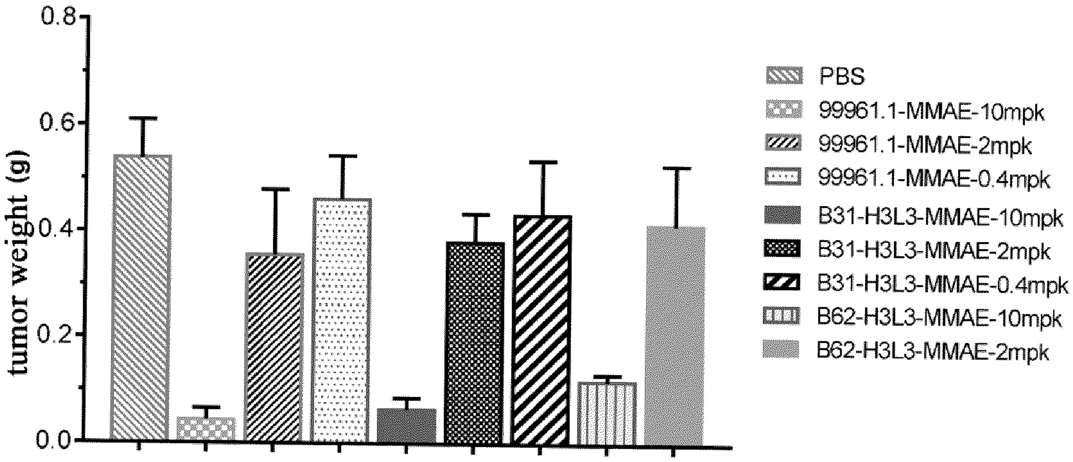


Figure 18C

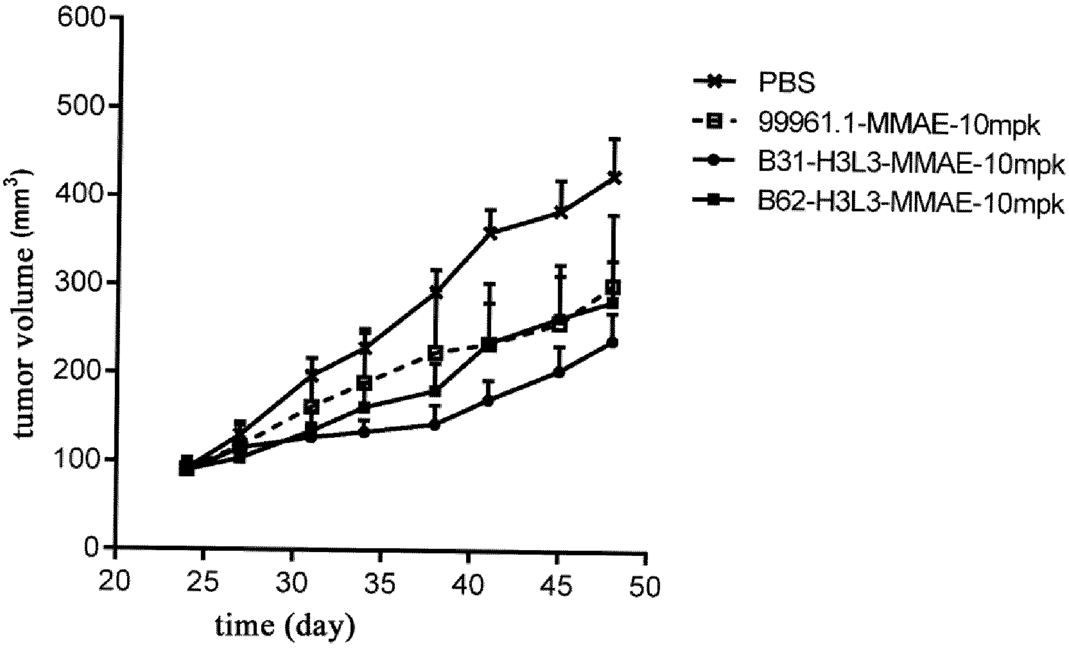


Figure 19A

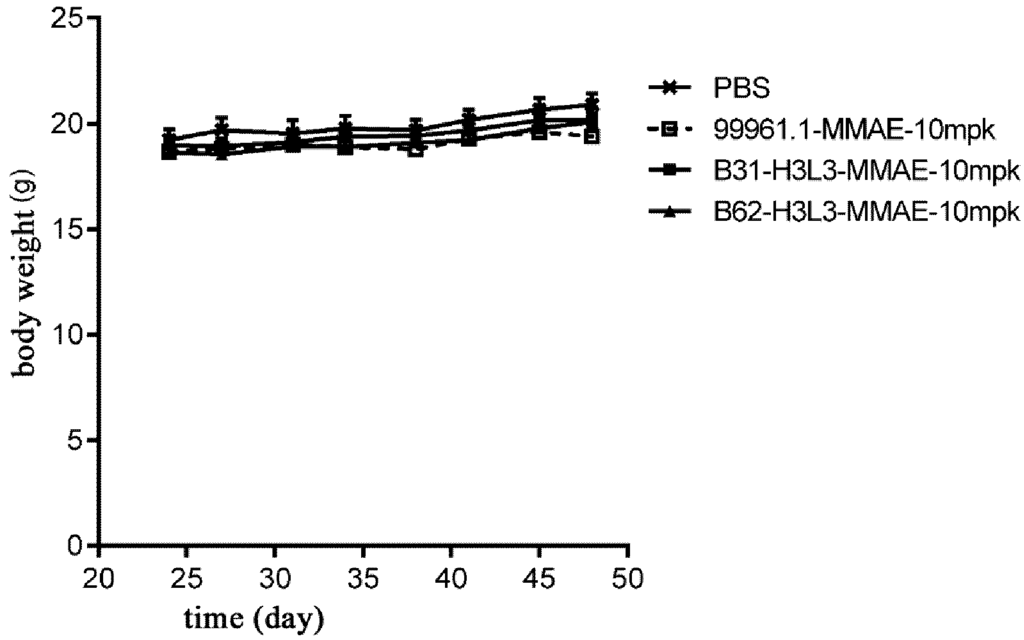


Figure 19B

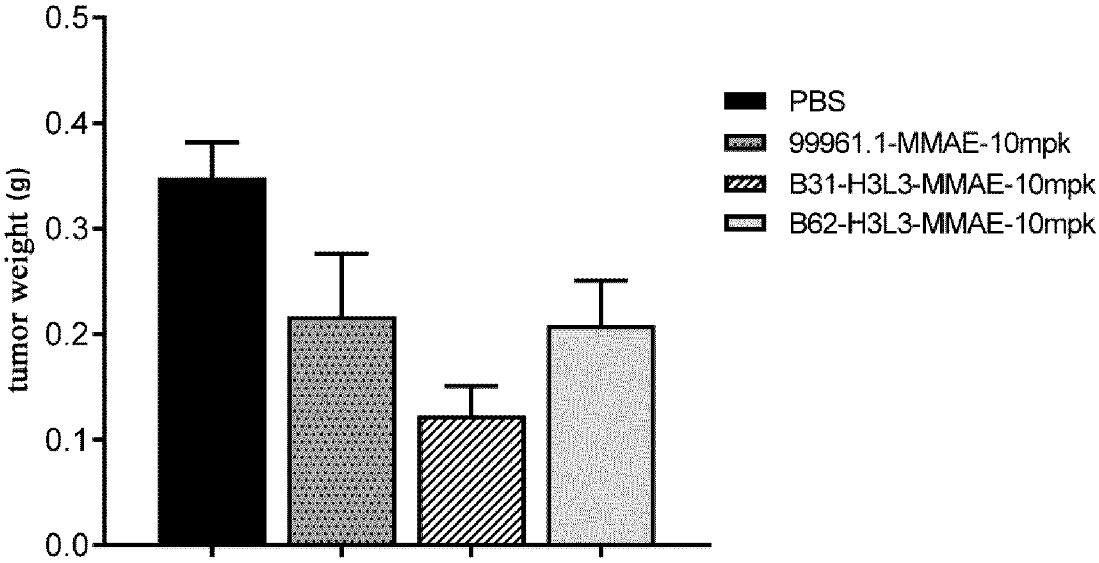


Figure 19C

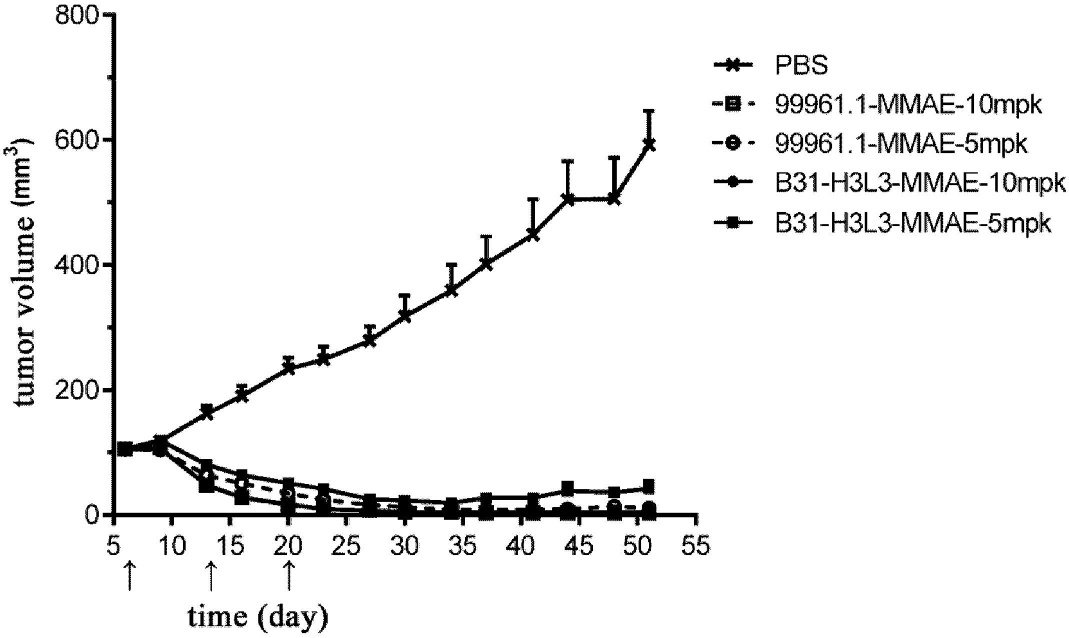


Figure 20A

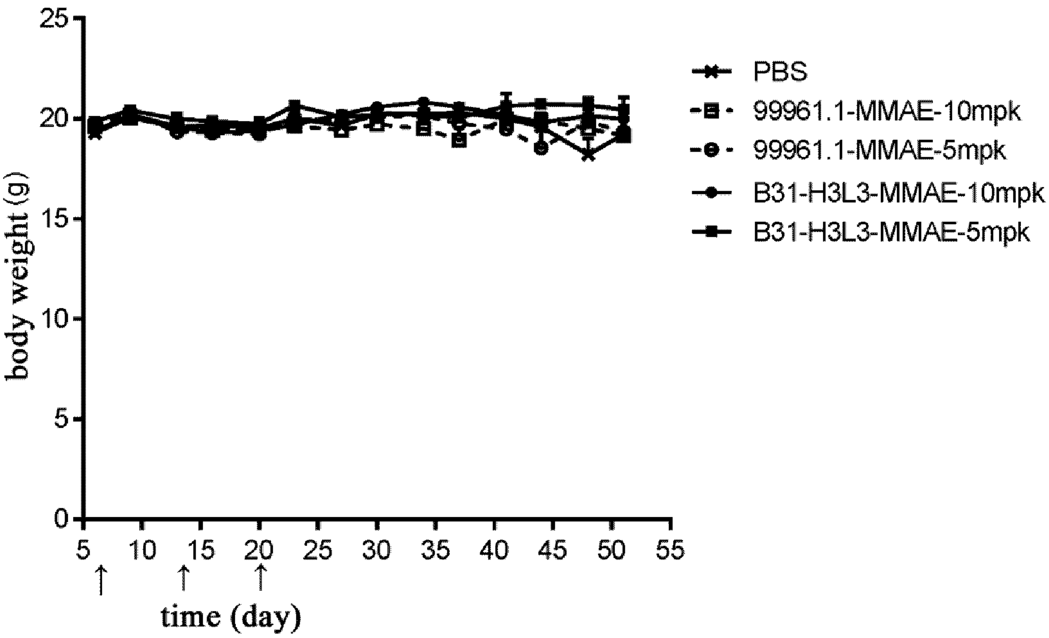


Figure 20B

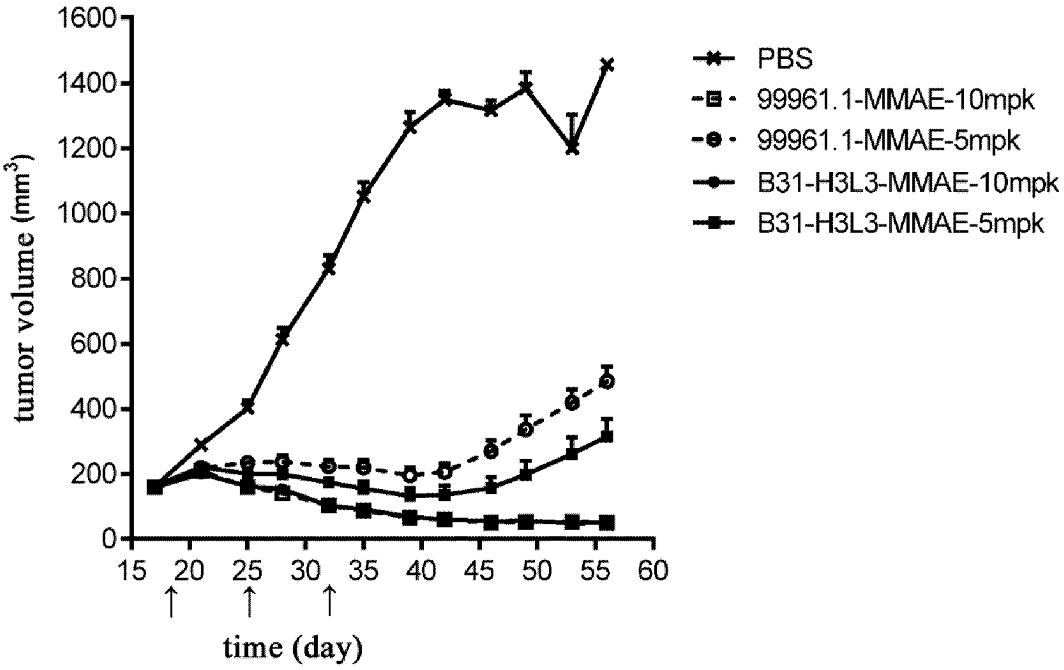


Figure 21A

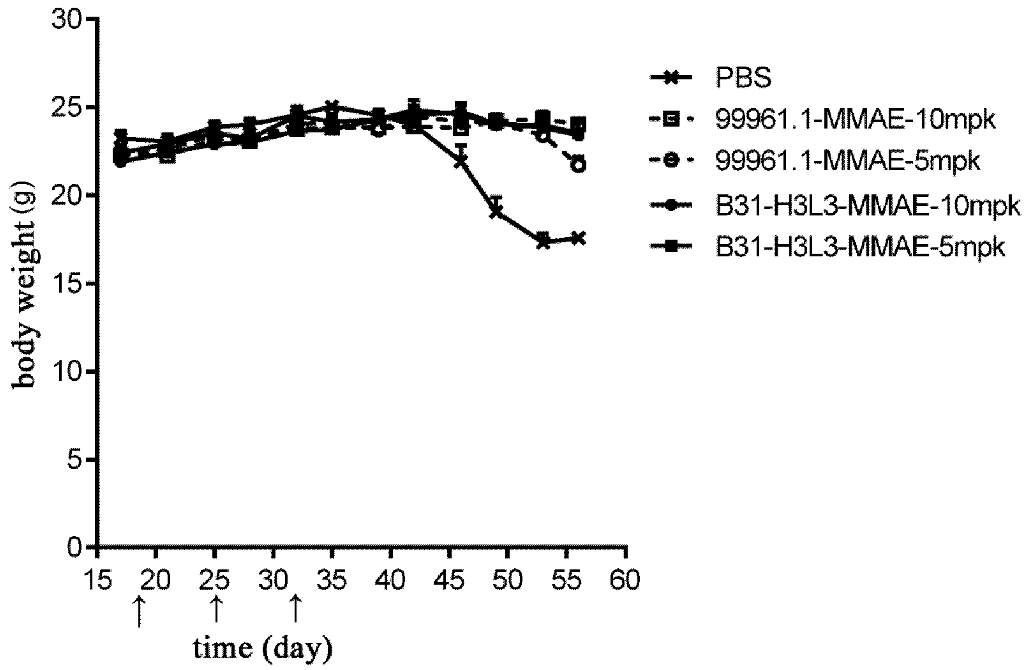


Figure 21B

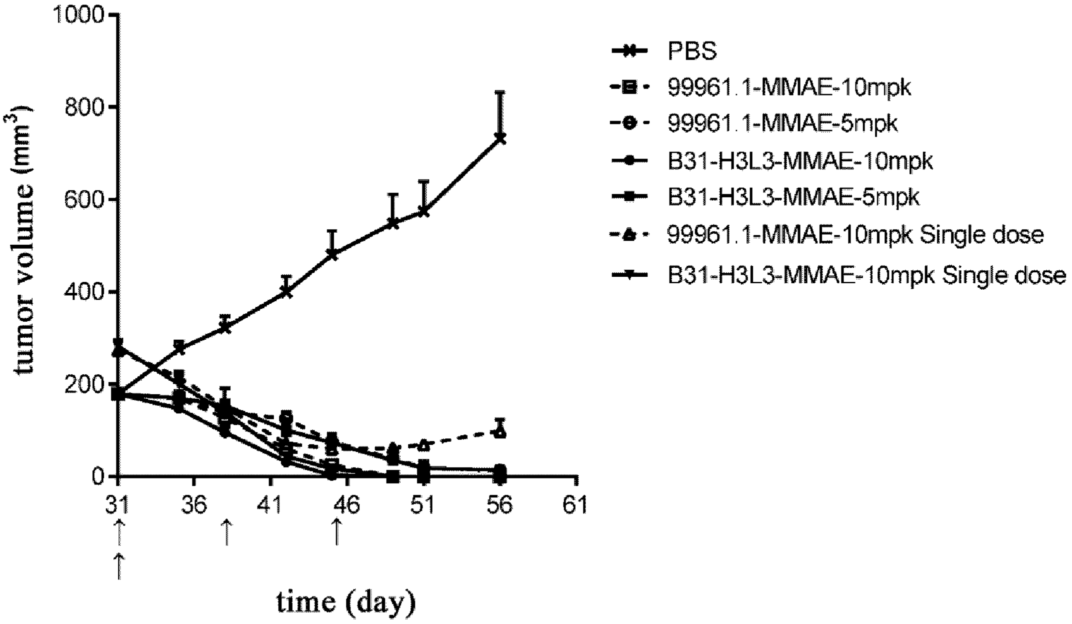


Figure 22A

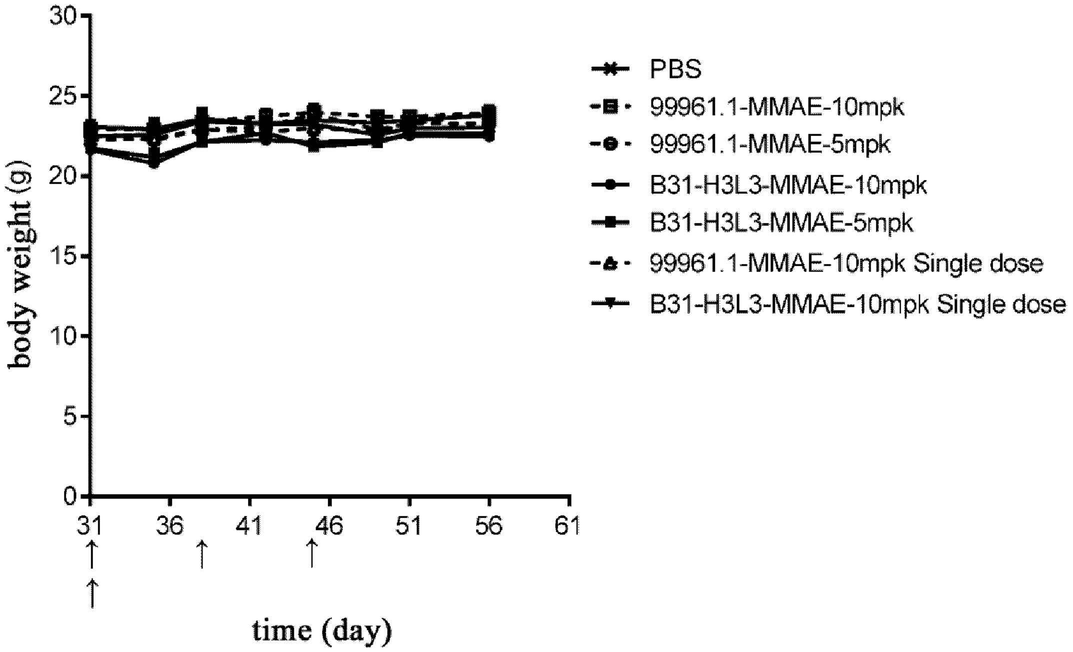


Figure 22B

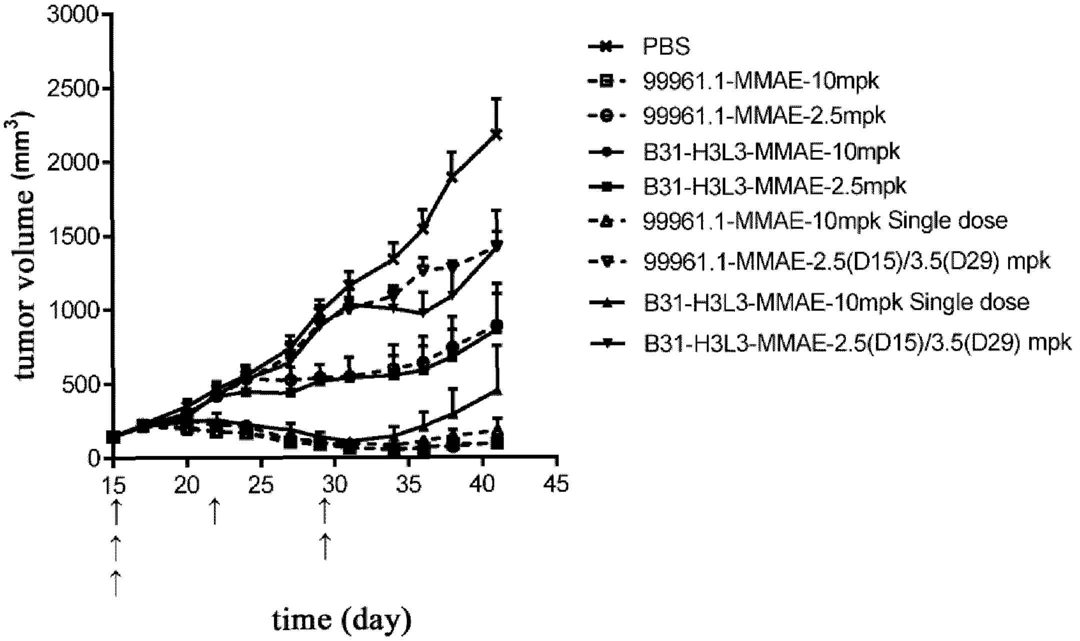


Figure 23A

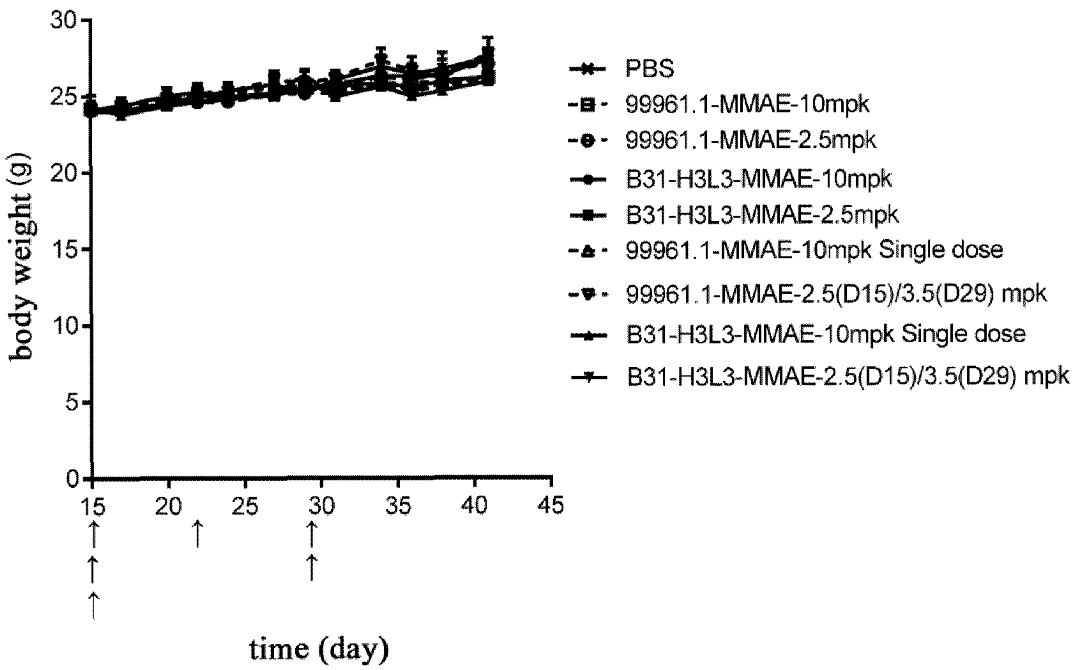


Figure 23B

**INTERNATIONAL SEARCH REPORT**

International application No.
**PCT/CN2023/079562**

**A. CLASSIFICATION OF SUBJECT MATTER**

C07K 16/28(2006.01)i

According to International Patent Classification (IPC) or to both national classification and IPC

**B. FIELDS SEARCHED**

Minimum documentation searched (classification system followed by classification symbols)

C07K

Documentation searched other than minimum documentation to the extent that such documents are included in the fields searched

Electronic data base consulted during the international search (name of data base and, where practicable, search terms used)

CNMED, CNABS, DWPI, WPABS, WPABSC, CJFD, AUABS, TWMED, ILABS, HKABS, MOABS, CNTXT, ENTXT, ENTXTC, WOTXT, USTXT, VCN, VEN, GBTXT, EPTXT, CATXT, CNKI, PubMed, EMBL, Web of Science, GoogleScholar: ROR1, 抗体, antibod+, ADC, receptor tyrosine kinase-like orphan receptor1 中国专利生物序列检索系统, China Patent Biological Sequence Search System, NCBI, EMBL, STN: 基于SEQ ID NO: 1-3, 28-30, 56, 57的序列检索, search based on SEQ ID NO: 1-3, 28-30, 56, 57

**C. DOCUMENTS CONSIDERED TO BE RELEVANT**

| Category* | Citation of document, with indication, where appropriate, of the relevant passages | Relevant to claim No. |
|---|---|---|
| A | AU 2015252014 A1 (US HEALTH) 19 November 2015 (2015-11-19)<br>description, paragraphs 6-11 | 1-24, 27, 28 (all in part) |
| A | CN 107827984 A (ZHANG, Huilin; ZHU, Jin) 23 March 2018 (2018-03-23)<br>abstract | 1-24, 27, 28 (all in part) |
| A | CN 108707619 A (SHANGHAI HRAIN BIOTECHNOLOGY CO., LTD.) 26 October 2018 (2018-10-26)<br>abstract | 1-24, 27, 28 (all in part) |
| A | CN 110590951 A (ANMENGDE MEDICAL TECHNOLOGY (SHANGHAI) CO., LTD.) 20 December 2019 (2019-12-20)<br>abstract | 1-24, 27, 28 (all in part) |
| A | EP 2649098 A1 (BIOINVENT INTERNATIONAL AB) 16 October 2013 (2013-10-16)<br>abstract | 1-24, 27, 28 (all in part) |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 May 2023** | **18 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/079562**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/079562**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **25-28 (in part)**
because they relate to subject matter not required to be searched by this Authority, namely:

Claims 25-28 (in part) relate to the subject matter of the antibody or an antigen-binding fragment thereof of any one of claims 1-7, or an effective amount of the antibody-drug conjugate of any one of claims 8-18, or an effective amount of the pharmaceutical composition of claim 19 being used for prevention or treatment, which falls within the cases set out in PCT Rule 39.1(iv) for which no international search is required, and therefore no international search is performed. An international search is still carried out on the technical solutions of claims 27 and 28 that refer to the pharmaceutical use of claim 24.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Group 1: claims 1-28 (in part) set forth the technical solution relating to an anti-ROR1 antibody including three heavy chain CDRs included in a heavy chain variable region as shown in SEQ ID NO: 57 and three light chain CDRs included in a light chain variable region as shown in SEQ ID NO: 56.

Group 2: claims 1-28 (in part) set forth the technical solution relating to an anti-ROR1 antibody including three heavy chain CDRs included in a heavy chain variable region as shown in SEQ ID NO: 55 and three light chain CDRs included in a light chain variable region as shown in SEQ ID NO: 54.

…

Group 18: claims 1-28 (in part) set forth the technical solution relating to an anti-ROR1 antibody including three heavy chain CDRs included in a heavy chain variable region as shown in SEQ ID NO: 84 and three light chain CDRs included in a light chain variable region as shown in SEQ ID NO: 89.

The common technical feature between groups 1-18 is that the antibody binds to ROR1. However, the prior art document CN 110590951 A (15 June 2019, see the claims) discloses an anti-ROR1 antibody. Therefore, the above-mentioned technical feature cannot constitute the specific technical feature distinguishing the inventions from the prior art, and the 18 groups of inventions do not have the specific technical features that contribute to the prior art, are not linked with each other, and thus do not comply with PCT Rules 13.1 and 13.2.

Form PCT/ISA/210 (continuation of first sheet) (July 2022)

**INTERNATIONAL SEARCH REPORT**

International application No.
**PCT/CN2023/079562**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-28 (in part) set forth the technical solution relating to an anti-ROR1 antibody including three heavy chain CDRs included in a heavy chain variable region as shown in SEQ ID NO: 57 and three light chain CDRs included in a light chain variable region as shown in SEQ ID NO: 56.**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2023/079562**

| Patent document cited in search report | Publication date (day/month/year) | Patent family member(s) | Publication date (day/month/year) |
|---|---|---|---|
| AU 2015252014 A1 | 19 November 2015 | AU 2015252014 B2 | 02 March 2017 |
| CN 107827984 A | 23 March 2018 | None | |
| CN 108707619 A | 26 October 2018 | None | |
| CN 110590951 A | 20 December 2019 | None | |
| EP 2649098 A1 | 16 October 2013 | EP 2649098 B1 | 22 November 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20130129753 A
- WO 2019173843 A
- CN 112250763 B


### Non-patent literature cited in the description


- **OISHI I** ; **SUZUKI H** ; **ONISHI N** ; **TAKADA R** ; **KANI S** ; **OHKAWARA B** ; **KOSHIDA I** ; **SUZUKI K** ; **YAMADA G** ; **SCHWABE GC et al.** *Genes Cells*, 2003, vol. 8, 645-654
- **FUKUDA T** ; **CHEN L** ; **ENDO T** ; **TANG L** ; **LU D** ; **CASTRO JE** ; **WIDHOPF GF II** ; **RASSENTI LZ** ; **CANTWELL MJ** ; **PRUSSAK CE et al.** *Proc Natl Acad Sci USA*, 2008, vol. 105, 3047-3052
- **PAGANONI S** ; **BERNSTEIN J** ; **FERREIRA A**. *Neuroscience*, 2010, vol. 165, 1261-1274
- **YAMAGUCHI T** ; **YANAGISAWA K** ; **SUGIYAMA R** ; **HOSONO Y** ; **SHIMADA Y** ; **ARIMAC** ; **KATOS** ; **TOMIDAS** ; **SUZUKI M** ; **OSADAH et al.** *Cancer Cell*, 2012, vol. 21, 348-361
- **CUI B** ; **ZHANG S** ; **CHEN L** ; **YU J** ; **WIDHOPF GF** ; **FECTEAU J-F** ; **RASSENTI LZ** ; **KIPPS TJ**. *Cancer Res*, 2013, vol. 73, 3649-3660
- **MCCOMBS J** ; **OWEN S.** Antibody drug conjugates: design and selection of linker, payload and conjugation chemistry.. *AAPS J.*, 2015, vol. 17, 339-51
- **KINDT et al.** Kuby Immunology. WH Freeman and Co., 2007, 91
- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948
- **KABAT et al.** Sequences of Proteins of Immunological Interest. U.S. Department of Health and Human Services, 1987
- **R.C. ROWE** ; **P.J. SESKEY** ; **S.C. OWEN**. Handbook of Pharmaceutical Excipients. Pharmaceutical Press
- **MCCOMBS J** ; **OWEN S.** Antibody drug conjugates: design and selection of linker, payload and conjugation chemistry. *AAPS J.*, 2015, vol. 17, 339-51